# EUROPEAN PATENT SPECIFICATION

(11) **EP 1 908 850 B1**
(45) Date of publication and mention of the grant of the patent: **04.01.2012**
(21) Application number: 07121248.4
(22) Date of filing: 24.11.2003
(51) Int. Cl.: C12Q 1/68, C07H 21/00, A01N 43/04

(54) **Target for therapy of cognitive impairment**
Target zur Therapie kognitiver Behinderungen
Cible pour la thérapie des déficiences cognitives

(30) Priority: 22.11.2002 US 428229 P
(43) Date of publication of application: 09.04.2008
(62) Divisional of application: 03808425.7
(73) Proprietor: The Johns Hopkins University, Baltimore, MD 21218 (US)
(72) Inventor: Gallagher, Michela, Baltimore, NC 21230 (US); Lund, Pauline, Kay, Carrboro, NC 27510 (US)
(74) Representative: Evenson, Jane Harriet

(56) References cited:
- EP-A1- 1 270 567
- WO-A-01/12184
- WO-A-2004/076675
- WO-A2-03/070965
- SHIMADA FUMIKI ET AL: "The neuroprotective agent MS-153 stimulates glutamate uptake" EUROPEAN JOURNAL OF PHARMACOLOGY, vol. 386, no. 2-3, 15 December 1999 (1999-12-15), pages 263-270, XP002446492 ISSN: 0014-2999
- UEDA YUTO ET AL: "Molecular regulation of glutamate and GABA transporter proteins by valproic acid in rat hippocampus during epileptogenesis" EXPERIMENTAL BRAIN RESEARCH, vol. 133, no. 3, August 2000 (2000-08), pages 334-339, XP002446493 ISSN: 0014-4819
- LIANG ZHE ET AL: "Effects of estrogen treatment on glutamate uptake in cultured human astrocytes derived from cortex of Alzheimer's disease patients" JOURNAL OF NEUROCHEMISTRY, vol. 80, no. 5, March 2002 (2002-03), pages 807-814, XP002446494 ISSN: 0022-3042
- DATABASE BIOSIS [Online] BIOSCIENCES INFORMATION SERVICE, PHILADELPHIA, PA, US 29 November 1997 GALLAGHER MICHELA: 'Animal models of memory impairment' Database accession no. PREV199800080665 & PHILOSOPHICAL TRANSACTIONS OF THE ROYAL SOCIETY OF LONDON B BIOLOGICAL SCIENCES, vol. 352, no. 1362, 29 November 1997 (1997-11-29), pages 1711-1717, ISSN: 0962-8436

## Description

This application claims the benefit of US Application Serial Number 60/413,152, filed November 22, 2002.

### 2. Government support

This invention was made with government support under grant No. PO1 AG09973 awarded by the National Institutes on Aging. The government may have certain rights in the invention.

### 3. Background of the Invention

As the understanding of cognitive impairment increases so does the necessity to develop sensitive methods to detect the impairment and treatments for the impairment. There are many conditions, such as dementias (e.g. Lewy body dementia, vascular dementia, Alzheimer's Disease, and HIV associated dementia), Huntington's Disease, Parkinson's Disease, schizophrenia, depression, amyotrophic lateral sclerosis, Mild Cognitive Impairment (MCI) and Age Related Cognitive Decline (ARCD), of which sensitive detection of cognitive impairment would benefit the sufferer of the condition.

A major risk factor for a variety of conditions with cognitive impairment (such as Lewy body dementia, vascular dementia, Alzheimer's Disease, HIV associated dementia, Huntington's Disease, Parkinson's Disease, amyotrophic lateral sclerosis, MCI and ARCD) is aging. Individuals with these conditions have cognitive symptoms that increase in severity over the course of the disease. The effect of aging itself on cognition, in the absence of such disease, is important for defining the boundary between illness and normal aging. At the same time, the effects of aging on cognition may interact with the disease process in neurodegenerative illnesses, in determining vulnerability, rate of progression or other features of illness.

An important resource for developing detection methods and treatments for cognitive impairments includes using laboratory animals. Features that characterize cognitive impairments in animal models likely extend to cognitive impairments in humans. In the context of age-related cognitive impairments, extensive behavioral characterization has identified a naturally occurring form of cognitive impairment in an outbred strain of aged Long-Evans rats (Charles River Laboratories; Gallagher M, et al., Behav. Neurosci. 107:618-626; 1993). This model of cognitive aging uses animals that are maintained pathogen-free throughout their lives. Tests of physiological function and necropsies performed on all aged rats are used to exclude animals with conditions that would confound the study of aging with illness or disease. An important feature of this model is that it mirrors the phenomenon of variability in cognitive decline among elderly humans. Furthermore, the individual differences in cognitive decline in aged rats in this model are seen in a behavioral assessment that is sensitive to the function of interconnected structures in the medial temporal lobe, a system that is essential for declarative memory in humans.

Another important feature of this model is that it is directed to understanding multiplicity of genes that contribute to age-associated cognitive impairment. The genetic contribution to age-associate cognitive impairment is not likely to be monogenic, meaning caused by a deletion or mutation in a single gene. Monogenic diseases are very rare and typically affect the young. Because of their severity, monogenic diseases are frequently inconsistent with attainment of average life expectancy. In humans, the vast majority of common but serious conditions affect the adult population, increase in frequency and severity with increasing chronological age and cannot be attributed to a single gene (see for example, Hegele RA. Trends Endocrinol Metab. 2003 8:371-377; Shih DQ, et al. Curr Diab Rep. 2002 2:125-134; Barlassina C, et al. J Am Soc Nephrol. 2002 Suppl 3:5155-5164). Accumulating evidence suggests that the genetic component of maturity onset or aging-associated conditions reflects more subtle changes in expression of multiple genes than the absolute deficiency or dramatic gain of function underlying monogenic disease. The challenge in defining the molecular basis of these age-associated conditions is to identify the multiplicity of genes and establish if the relatively small changes in expression of a defined group of genes indeed associate with or lead to condition in an outbred population such as the human population. Thus, using a mammalian outbred model of aging facilitates the analysis of the relationship between levels of expression of multiple genes within the hippocampus and learning ability, in out-bred young and aged subjects.

In a behavioral assessment with the Morris Water Maze (MWM), rats learn and remember the location of an escape platform guided by a configuration of spatial cues surrounding the maze. The cognitive basis of performance is tested in probe trials using measures of the animal's spatial bias in searching the location of the escape platform. Aged rats in the study population have no difficulty swimming to a visible platform, but an age-dependent impairment is detected when the platform is camouflaged, requiring the use of spatial information. As reported in many publications, performance for individual aged rats in the outbred Long-Evans strain varies greatly, with a proportion of those rats performing on a par with young adults but approximately 40-50% falling outside the range of young performance (Gallagher et al. Behav. Neurosci. 107: 618-626, 1993). This variability among aged rats reflects reliable individual differences. Thus, within the aged population some animals are cognitively impaired and designated aged impaired (AI). Other aged animals are cognitively unimpaired, or aged unimpaired (AU).

In a reassessment using the MWM in a new spatial environment several weeks after the original characterization, the AI animals are consistently impaired, whereas the AU animals again perform proficiently (Colombo et al. Proc. Natl. Acad. Sci. 94: 14195-14199, 1997). The difference in cognitive ability in the MWM assessment for AI and AU rats is reliable even over an interval of 3 months (Gallagher and Burwell, Neurobiol. Aging 10: 691-708, 1989). Further, AI and AU characterization in the MWM differentiates the performance of the same aged subjects in other behavioral tasks that require the same cognitive function, such as the Barnes circular maze (Gallagher and Burwell Neurobiol. Aging 10: 691-708, 1989), and the radial arm maze (RAM). This naturally occurring impairment in an aged population of rodents indicates that cognitive aging is not inevitable or strictly linked to chronological age, and, importantly, it affords the opportunity to compare the trajectory of changes in the brain that lead to decline or preserved memory. Additional background research using this model indicates that cognitive impairment occurs independent of neurodegeneration involving loss of neurons or widespread degradation of relevant circuits (Rapp and Gallagher Proc. Natl. Acad. Sci. 93: 9926-9930, 1996). Thus, this model is likely to be a more sensitive test of cognitive aging than preparations intended to measure the effects of neuron loss.

In addition to reliability, the cognitive assessment used in this model has proven sensitive to effects of aging on relevant brain systems. Significant biological differences have been shown to occur in AU and AI rats within neural circuits that are critical for the cognitive function assessed in the MWM. For example, the neurons in the hippocampus have a reduced response to certain chemical transmitters, such as acetylcholine and glutamate, in AI rats as compared to both AU and young rats (Nicolle et al. J. Neurosci. 19: 9604-9610, 1999). In a study of the anatomical distribution of glutamate receptor subtypes, the use of this model revealed a decrease in kainate binding in the CA3 region of the hippocampus that was confined to aged unimpaired rats and differed from both young and aged impaired (Nicolle et al. Neuroscience 74: 741-756, 1996). There is a need for a greater understanding of the biological and genetic basis of cognitive impairment.

WO 2001/012184 recites the use of β-lactam containing compounds (including cephalosporin compounds) in the treatment of cognitive and behavioral disorders and use of these compounds for the preparation of medicaments for the treatment of such disorders. Shimada et al., European Journal of Pharmacology (1999), vol. 386, pp. 263-370 recites the use of MS-153 as a potential therapeutic agent in ischemic stroke. Ueda et al., Experimental Brain Research (2000), vol. 133, pp. 334-339 recites the use of valproate for regulating the glutamate transporter in neural tissue during epileptogenisis. Liang et al., Journal of Neurochemistry (2002), vol. 80, pp. 807-814 recites the effect of estrogen on glutamate uptake in Alzheimer's disease patients. EP1270567-A1 (published 2 January 2003) recites the use of pyrazoline derivatives for the treatment of cerebral ischemia and ALS. WO 2003/070965 (published 28 August 2003) recites screening assays for identifying compounds which modulate the activity of the promoter, and methods for treating neurological disorders comprising administration of EAAT2 promoter modulators. WO 2004/076675 (published 10 September 2004) recites compounds useful for modulating EAAT protein expression and treating neurological disorders. The citeability of EP1270567-A1, WO 2003/070965, and WO 2004/076675 is dependent on the present application's entitlement to the claimed priority date. WO 2004/076675 is, in any case, not citeable for inventive step.

The invention provides a compound for use in treating cognitive impairment during aging in a subject in need thereof, wherein the compound is valproic acid or a pharmaceutically acceptable salt thereof. Preferably, the cognitive impairment is mild cognitive impairment.

The invention provides use of a compound for the manufacture of a medicament for treating cognitive impairment during aging in a subject in need thereof, wherein the compound is valproic acid or a pharmaceutically acceptable salt thereof. Preferably, the cognitive impairment is mild cognitive impairment.

### 4. Summary of the Invention

A method is disclosed of identifying a gene associated with a desired behavior of a subject, such as a mammal, comprising providing a test population of subjects having the desired behavior, providing a control population of subjects lacking the desired behavior, isolating and pooling expressed RNA from neural tissue, such as the of the test and control populations, respectively, determining the level of expression of a plurality of genes in each of the control and test RNA pools and selecting a gene from the plurality of genes, the expression of which differs between the test population and the control population of mammals. The selected gene is a candidate gene associated with a desired behavior. The level of expression of the plurality of genes may be detected by any appropriate means, such as microarray analysis, in situ hybridization histochemistry, quantitative PCR, SAGE analysis, Northern blot analysis or dot blot analysis, or by appropriate methods of measuring protein levels, including Western blot, protein slot blot or protein arrays. The plurality of genes may comprise genes involved in glutamate transport, such as EAAT1, EAAT2, BAAT3, BAAT4 and EAAT5, genes other than the glutamate transporters EAAT1, EAAT2, EAAT3, EAAT4 and EAAT5 or genes involved in the catabolism of glutamate in the synaptic cleft and/or extrasynaptic space between neurons, such as aspartate aminotransferase. Preferably, the gene selected from the plurality of genes exhibits increased levels of expression. Alternatively, the gene selected may exhibit decreased levels of expression.

A method is disclosed of identifying a gene associated with a cognitive function of a subject comprising providing a test population of mammals having the desired cognitive function, providing a control population of mammals lacking the desired cognitive function, isolating and pooling expressed RNA from neural tissue, such as the hippocampus, of the test and control populations, respectively, determining the level of expression of a plurality of genes in each of the control and test RNA pools and selecting a gene from the plurality of genes, the expression of which differs between the test population and the control population of mammals. The selected gene is a candidate gene associated with a desired cognitive function. The level of expression of the plurality of genes may be measured by any appropriate means, such as microarray analysis, in situ hybridization histochemistry, quantitative PCR, SAGE analysis, Northern blot analysis or dot blot analysis, or by appropriate methods of measuring protein levels, including Western blot, protein slot blot or protein arrays. The plurality of genes may comprise genes involved in glutamate transport, such as EAAT1, EAAT2, EAAT3, EAAT4 and EAAT5, genes other than the glutamate transporters EAAT1, EAAT2, EAAT3, EAAT4 and EAAT5 or genes involved in the catabolism of glutamate in the synaptic cleft and/or extrasynaptic space between neurons, such as aspartate aminotransferase. Preferably, the gene selected from the plurality of genes exhibits increased levels of expression. Alternatively, the gene selected may exhibit decreased levels of expression.

A method is disclosed of screening compounds for utility in promoting cognitive function comprising administering a test compound to a subject, such as a mammal, determining the level of expression of a gene in neural tissue, such as the hippocampus, of said subject following administration of said test compound, comparing said level of expression of said gene to a reference level of expression thereof in neural tissue of a subject to whom said test compound was not administered and determining whether the level of expression of said gene differs from the corresponding reference level of expression thereof, wherein said difference indicates that the test compound is a candidate therapeutic agent for promoting cognitive function. The test compound may be a small molecule, such as but not limited to those found in formula I, II or III. Further, the method may comprise comparing the level of expression of said gene to a reference level of expression thereof in neural tissue of a subject to whom ceftriaxone was administered. The level of expression of the gene may be detected by any appropriate means, such as microarray analysis, in situ hybridization histochemistry, quantitative PCR, SAGE analysis, Northern blot analysis or dot blot analysis, or by appropriate methods of measuring protein levels, including Western blot, protein slot blot or protein arrays. The gene may be involved in glutamate transport, such as EAAT1, EAAT2, EAAT3, EAAT4 and EAAT5 or maybe involved in the catabolism of glutamate in the synaptic cleft and/or extrasynaptic space between neurons, such as aspartate aminotransferase. Preferably, the gene selected from the plurality of genes exhibits increased levels of expression. Alternatively, the gene selected may exhibit decreased levels of expression.

A method is disclosed of screening compounds for utility in promoting cognitive function comprising administering a test compound to a subject, such as a mammal, determining the level of expression of a glutamate transporter gene in neural tissue, such as the hippocampus, of said subject following administration of said test compound, comparing said level of expression of said gene to a reference level of expression thereof in neural tissue of a subject to whom said test compound was not administered and determining whether the level of expression of said gene differs from the corresponding reference level of expression thereof, wherein said difference indicates that the test compound is a candidate therapeutic agent for promoting cognitive function. The test compound may be a small molecule, such as but not limited to those found in formula I, II or III. The level of expression of the gene may be detected by any appropriate means, such as microarray analysis, in situ hybridization histochemistry, quantitative PCR, SAGE analysis, Northern blot analysis or dot blot analysis, or by appropriate methods of measuring protein levels, including Western blot, protein slot blot or protein arrays. Preferably, the gene selected from the plurality of genes exhibits increased levels of expression. Alternatively, the gene selected may exhibit decreased levels of expression.

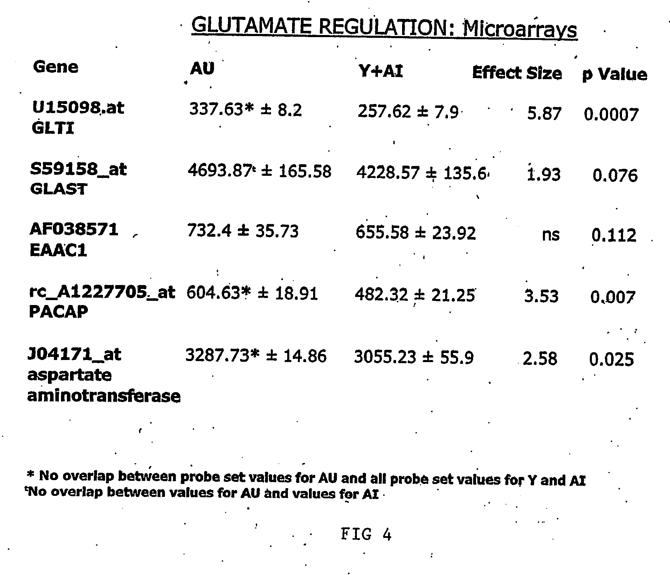

A method of screening compounds for utility in promoting cognitive function in a subject, such as a mammal, comprising the steps of contacting a test compound with a cell expressing a gene listed in Figure 4, e.g., a glutamate transporter gene BAAT1, 2, 3, 4 or 5, aspartate aminotransferase or pituitary adenyl cyclase activator polypeptide (PACAP), and determining whether the level of expression of said gene is changed by contact of said cell with said test compound, said change if present being indicative of the ability of said compound to promote cognitive function in a subject, such as a mammal, in need thereof. The compound may be a small molecule, such as those found in formula I, II or III. The cell may be derived from neural tissue, such as culturered neurons, cultured glia or primary neuronal culture; or may be an immortalized cell, a neuronal cell line, glial cell line or an astrocyte cell line. Preferably, the gene selected from the plurality of genes exhibits increased levels of expression. Alternatively, the gene selected may exhibit decreased levels of expression.

The test compound used in each of the above-mentioned disclosures may be a small molecule, such as any of the third-generation cephalosporins (cefsulodin, cefotaxime, ceftizoxime, ceftriaxone, cefoperazone, moxalactam, and ceftazidime), valproic acid or MS-153. Further the test compound may activate gene expression, including the glutamate transporters selected from the group consisting of EAAT1, EAAT2, EAAT3, EAAT4 and EAAT5, or the aspartate aminotransferase gene. Alternatively, the test compound may be an inhibitor of gene expression.

A library is disclosed comprising a plurality of cDNA sequences coding for genes that are differentially expressed in mammalian neural tissue upon preservation of cognitive function in a mammal. Preferably, a library comprises cDNA sequences coding for genes that are differentially expressed in neural tissue upon treatment of the mammal with ceftriaxone, valproic acid or MS-153. The library may contain cDNA sequences derived for a glutamate transporter gene, such as EAAT1, EAAT2, EAAT3, EAAT4 and EAAT5, or a sequence derived from aspartate aminotransferase. The library containing cDNA at least 20%, 50% or 80% of sequences derived from a glutamate transpoter gene.

A microarray chip is disclosed comprising a solid support having attached thereto, at individually addressed locations, cDNA sequences corresponding to members of the above-mentioned cDNA library, such as those cDNA sequences that are differentially expressed in neural tissue upon preservation of cognitive function in a subject or upon treating the subject with ceftriaxone or valproic acid. Members of the microarray chip include either a glutamate transporter sequence selected from the group consisting of EEAT1, EEAT2, EEAT3, EEAT4 and EEAT or an aspartate aminotransferase sequence.

A pharmaceutical composition is disclosed comprising a therapeutically effective amount of a compound that stimulates neural tissue expression of a gene listed in Figure 4, e.g., a glutamate transporter gene EAAT1, 2, 3, 4 or 5, aspartate aminotransferase or pituitary adenyl cyclase activator polypeptide (PACAP). The pharmaceutical composition may further comprise a small molecule.

A pharmaceutical composition is disclosed comprising a therapeutically effective amount of formula I, II or III. Alternative, the pharmaceutical composition may comprise a therapeutically effective amount of a compound other than ceftriaxone or valproic acid, that was identified by a method screening compounds for utility in promoting cognitive function by administering compounds to subjects, such as mammals, or cells and measuring differential gene expression between those subjects or cells with and without exposure to the compounds. These compounds are candidate compounds for promoting cognitive function.

A method is disclosed for preserving cognitive function in a mammal, such as a human, or treating impaired cognitive function in a mammal, such as a human, by stimulating the neural tissue expression of a gene involved in either glutamate transport or glutamate catabolism in neural tissue. Further, preserving cognitive function in a mammal, such as a human, in need thereof comprises administering a pharmaceutical composition that that stimulates neural tissue expression of a gene listed in Figure 4, e.g., a glutamate transporter gene EAAT1, 2, 3, 4 or 5, aspartate aminotransferase or pituitary adenyl cyclase activator polypeptide (PACAP).

A method is disclosed of preserving cognitive function in a mammal, such as a human, in need thereof comprises administering a pharmaceutical composition that is a small molecule of any one of the following formulas: I, II or III. For a method of preserving cognitive function in a mammal, such as a human, in need thereof comprises administering a compound of formula I, the mammal is free of symptoms of an infectious disease for which antibiotic treatment is indicated.

Promoting cognitive function in a mammal is disclosed, such as a human, in need thereof, comprising administering to said mammal an amount of a pharmaceutical composition that stimulates neural tissue expression of a gene listed in Figure 4 sufficient to promote the following cognitive functions: spatial memory acquisition, long term spatial memory or spatial memory retrieval. Preserving cognitive function or treating cognitive impairment in an aged mammal is disclosed; such as a human, and treating impaired cognitive function in a mammal, such as a human, by administering a therapeutically effective amount of ceftriaxone or analogs or derivatives thereof, valproic acid or analogs or derivatives thereof or MS-153 or analogs or derivatives thereof to the mammal in need thereof. In the cases in which a mammal manifests impaired cognitive function the impaired cognitive function may be associated with one of the following conditions: mild cognitive impairment, age related cognitive decline, memory loss, senility or dementia. Additionally, cases in which a mammal manifests impaired cognitive function the impaired cognitive function may be associated Alzheimer's Disease.

Other features and advantages of the invention will be apparent based on the following Detailed Description and Claims.

### 5. Brief Description of the Drawings

**Figure 1** is a graph depicting the behavioral characterization of young and aged rats in the MWM assessment.
**Figure 2** is a graph depicting the reliability between the initial MWM characterization for 10 aged rats and and their memory performance in the RAM
**Figure 3** is a table summarizing the distribution of mammalian glutamate transporters and their human homologues in various cell types found in brain tissue.
**Figure 4** is a table summarizing the expression of the EAAT2/GLT1, EAAT1/GLAST, and EAAT3/EEAC1 mRNAs in young (Y), aged-impaired (AI) and aged-unimpaired (AU) animals using the microarray.
**Figure 5** is a table summarizing the abundance of EAAT2/GLT1, EAAT1/GLAST, and EAAT3/EEAC 1 mRNAs in young (Y), aged-impaired (AI) and aged-unimpaired (AU) animals using in situ hybridization histochemistry.
**Figure 6** is a graph depicting the reduction of memory errors in AI rats treated with ceftriaxone (daily injection of 200 mg/kg im, for 1 week).

### 6. Detailed Description

For convenience, certain terms employed in the specification, examples, and appended claims are collected here. Unless defined otherwise, all technical and scientific terms used herein have the same meaning as commonly understood by one of ordinary skill in the art to which this invention belongs.

### 6.1 Definitions

The articles "a" and "an" are used herein to refer to one or to more than one (i.e., to at least one) of the grammatical object of the article. By way of example, an element means one element or more than one element.

"Aged" is used herein to refer to mammals at or near the end of their average life span. For example, an aged rat would be about 24-30 months of age. An aged human would be seventy or more years of age.

The term "aliphatic" is art-recognized and refers to a linear, branched, cyclic alkane, alkene, or alkyne. In certain embodiments, aliphatic groups in the present invention are linear or branched and have from 1 to about 20 carbon atoms.

The term "alkyl" is art-recognized, and includes saturated aliphatic groups, including straight-chain alkyl groups, branched-chain alkyl groups, cycloalkyl (alicyclic) groups, alkyl substituted cycloalkyl groups, and cycloalkyl substituted alkyl groups. In certain embodiments, a straight chain or branched chain alkyl has about 30 or fewer carbon atoms in its backbone (e.g., C₁-C₃₀ for straight chain, C₃-C₃₀ for branched chain), and alternatively, about 20 or fewer. Likewise, cycloalkyls have from about 3 to about 10 carbon atoms in their ring structure, and alternatively about 5, 6 or 7 carbons in the ring structure. The term "alkyl" is also defined to include halosubstituted alkyls.

The terms "amine" and "amino" are art-recognized and refer to both unsubstituted and substituted amines, e.g., a moiety that may be represented by the general formulas: 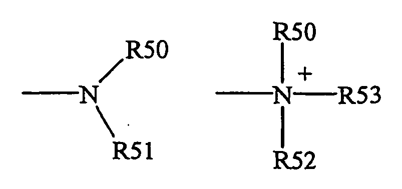 wherein R50, R51 and R52 each independently represent a hydrogen, an alkyl, an alkenyl, - (CH₂)ₘ-R61, or R50 and R51, taken together with the N atom to which they are attached complete a heterocycle having from 4 to 8 atoms in the ring structure; R61 represents an aryl, a cycloalkyl, a cycloalkenyl, a heterocycle or a polycycle; and m is zero or an integer in the range of 1 to 8. In certain embodiments, only one of R50 or R51 may be a carbonyl, e.g., R50, R51 and the nitrogen together do not form an imide. In other embodiments, R50 and R51 (and optionally R52) each independently represent a hydrogen, an alkyl, an alkenyl, or - (CH₂)ₘ-R61. Thus, the term "alkylamine" includes an amine group, as defined above, having a substituted or unsubstituted alkyl attached thereto, i.e., at least one of R50 and R51 is an alkyl group.

The term "acylamino" is art-recognized and refers to a moiety that may be represented by the general formula: 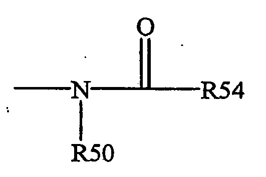 wherein R50 is as defined above, and R54 represents a hydrogen, an alkyl, an alkenyl or - (CH₂)ₘ-R61, where m and R61 are as defined above.

The term "amido" is art recognized as an amino-substituted carbonyl and includes a moiety that may be represented by the general formula: 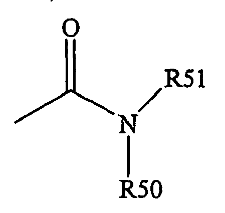 wherein R50 and R51 are as defined above. Certain embodiments of the amide will not include imides which may be unstable.

The term "alkylthio" refers to an alkyl group, as defined above, having a sulfur radical attached thereto. In certain embodiments, the "alkylthio" moiety is represented by one of -S-alkyl, -S-alkenyl, -S-alkynyl, and -S-(CH₂)ₘ-R61, wherein m and R61 are defined above. Representative alkylthio groups include methylthio, ethyl thio, and the like.

The term "aralkyl" is art-recognized and refers to an alkyl group substituted with an aryl group (e.g., an aromatic or heteroaromatic group).

The terms "alkenyl" and "alkynyl" are art-recognized and refer to unsaturated aliphatic groups analogous in length and possible substitution to the alkyls described above, but that contain at least one double or triple bond respectively.

Unless the number of carbons is otherwise specified, "lower alkyl" refers to an alkyl group, as defined above, but having from one to about ten carbons, alternatively from one to about six carbon atoms in its backbone structure. Likewise, "lower alkenyl" and "lower alkynyl" have similar chain lengths.

The terms "alkoxyl" or "alkoxy" are art-recognized and refer to an alkyl group, as defined above, having an oxygen radical attached thereto. Representative alkoxyl groups include methoxy, ethoxy, propyloxy, tert-butoxy and the like. An "ether" is two hydrocarbons covalently linked by an oxygen. Accordingly, the substituent of an alkyl that renders that alkyl an ether is or resembles an alkoxyl, such as may be represented by one of - O-alkyl, -O-alkenyl, -O-alkynyl, -O--(CH₂)ₘ-R61, where m and R61 are described above.

"Analog" is used herein to refer to a compound which functionally resembles another chemical entity, but does not share the identical chemical structure thereof. For example, a ceftriaxone analog is sufficiently similar to ceftriaxone that it can substitute for the ceftriaxone in therapeutic applications, despite minor structural differences from the structure of ceftriaxone.

The terms "array" and "matrix" are used herein to refer to an arrangement of addressable locations or "addresses" on a device. The locations can be arranged in two dimensional arrays, three dimensional arrays, or other matrix formats. The number of locations can range from several to at least hundreds of thousands. Most importantly, each location represents a totally independent reaction site. A "nucleic acid array" refers to an array containing nucleic acid probes, such as oligonucleotides or larger portions of genes. The nucleic acid on the array may be single stranded¹. Arrays wherein the probes are oligonucleotides are referred to as "oligonucleotide arrays" or "oligonucleotide chips." A "microarray," also referred to herein as a "biochip," "biological chip" or "gene array" is an array of regions having a density of discrete regions of at least about 100/cm², and preferably at least about 1000/cm². The regions in a microarray have typical dimensions, e.g., diameters, in the range of between about 10-250 µm, and are separated from other regions in the array by about the same distance.

"Aspartate aminotransferase" is used herein to refer to the enzyme (E.C. 2.6.1.1) that catalyzes the coversion of oxaloacetate and glutamate into aspartate and 2-oxoglutarate, and the nucleic acid and homologs (see for example, GenBank accession Nos.: BC000498 or XM_062678) encoding amino acids with aspartate aminotransferase activity. Aspartate aminotransferase is involved in the catabolism of glutamate in the synaptic cleft and
¹ CDNA arrays may be double stranded. extrasynaptic space. Homologs of the foregoing are believed to exist in other mammals, including primates, canines, felines and rodents.

"Beta-arrestin 2" is used herein to refer to the intracellular scaffold/adapter proteins that facilitate the transmission of additional signals from activated G protein-coupled receptors. Additionally, these proteins are involved in the endocytosis of transmembrane receptor endocytosis. Beta-arrestin 2 also refers to the nucleic acids that encode the β-arrestin protein. Homologs of the foregoing are believed to exist in other mammals, including primates, canines, felines and rodents.

The term "carbocycle" is art-recognized and refers to an aromatic or non-aromatic ring in which each atom of the ring is carbon.

The term "carbonyl" is art recognized and includes such moieties as may be represented by the general formulas: 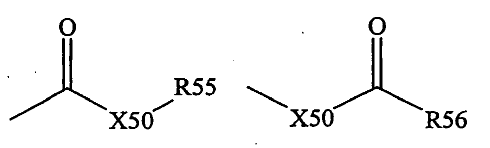 wherein X50 is a bond or represents an oxygen or a sulfur, and R55 and R56 represents a hydrogen, an alkyl, an alkenyl, -(CH₂)ₘ-R61 or a pharmaceutically acceptable salt, R56 represents a hydrogen, an alkyl, an alkenyl or -(CH₂)ₘ-R61, where m and R61 are defined above. Where X50 is an oxygen and R55 or R56 is not hydrogen, the formula represents an "ester". Where X50 is an oxygen, and R55 is as defined above, the moiety is referred to herein as a carboxyl group, and particularly when R55 is a hydrogen, the formula represents a "carboxylic acid". Where X50 is an oxygen, and R56 is hydrogen, the formula represents a "formate". In general, where the oxygen atom of the above formula is replaced by sulfur, the formula represents a "thiolcarbonyl" group. Where X50 is a sulfur and R55 or R56 is not hydrogen, the formula represents a "thiolester." Where X50 is a sulfur and R55 is hydrogen, the formula represents a "thiolcarboxylic acid." Where X50 is a sulfur and R56 is hydrogen, the formula represents a "thiolformate." On the other hand, where X50 is a bond, and R55 is not hydrogen, the above formula represents a "ketone" group. Where X50 is a bond, and R55 is hydrogen, the above formula represents an "aldehyde" group.

The term "chiral" is art-recognized and refers to molecules which have the property of non-superimposability of the mirror image partner, while the term "achiral" refers to molecules which are superimposable on their mirror image partner. A "prochiral molecule" is a molecule which has the potential to be converted to a chiral molecule in a particular process.

The term "cis" is art-recognized and refers to the arrangement of two atoms or groups around a double bond such that the atoms or groups are on the same side of the double bond. Cis configurations are often labeled as (Z) configurations.

"Cognitive function" is used herein to refer to higher order intellectual, brain processes involved in learning and memory, including, but not limited to, attention, acquisition, short-term memory, long-term memory and memory retrieval, and expressing an interest in one's surroundings and self-care. In animal model systems, cognitive function may be measured any number of ways known in the art, including using the following apparati: Morris water maze, Barnes circle maze, elevated radial arm maze, T maze or any other mazes in which subjects use spatial information. Other tests known in the art may be used to assess cognitive function, such as fear conditioning, active avoidance, illuminated open-field, dark activity meter, elevated plus-maze, two-compartment exploratory test or forced swimming test. In humans, cognitive function may be measured, without limitation, by the Alzheimer's Disease Assessment Scale-cognitive subscale (ADAS-cog); the clinical global impression of change scale (CIBIC-plus scale); the Alzheimer's Disease Cooperative Study Activities of Daily Living Scale (ADCS-ADL); the Mini Mental State Exam (MMSE); the Neuropsychiatric Inventory (NPI); the Clinical Dementia Rating Scale (CDR); the Cambridge Neuropsychological Test Automated Battery (CANTAB) or the Sandoz Clinical Assessment-Geriatric (SCAG). In addition, cognitive function may be measured using imaging techniques such as Positron Emission Tomography (PET), functional magnetic resonance imaging (fMRI), Single Photon Emission Computed Tomography (SPECT), or any other imaging technique that allows one to measure brain function.

"Promoting" cognitive function refers to affecting impaired cognitive function so that it more closely resembles the function of an aged-matched normal, unimpaired subject, and includes affecting states in which cognitive function is reduced, e.g., by about 10%, 30%, 50%, 75%, 90% or 95% as compared to a normal subject. Cognitive function may be promoted to any detectable degree, but preferably is promoted sufficiently to allow an impaired subject to carry out daily activities of normal life.

"Preserving" cognitive function refers to affecting normal or impaired cognitive function such that it does not decline or does not fall below that observed in the subject upon first presentation or diagnosis.

"Impaired cognitive function" refers to cognitive function that is not as robust as that observed in an age-matched normal subject and includes states in which cognitive function is reduced, e.g., by about 10%, 30%, 50%, 75%, 90% or 95% as compared to cognitive function measured in an age-matched normal subject. Impaired cognitive function may be associated with many diseases or disorders, involving dementias (e.g. Lewy body dementia, vascular dementia, Alzheimer's Disease, and HIV associated dementia), Huntington's Disease, Parkinson's Disease, schizophrenia, amyotrophic lateral sclerosis, Mild Cognitive Impairment (MCI) and Age Related Cognitive Decline (ARCD). Alternatively, impaired cognitive function may manifest in a subject that does not present with a diagnosable disease or disorder. For instance, impaired cognitive function may result from subtle metabolic, toxic, neurotoxic, iatrogenic, thermal or chemical changes in the subject. These subtle changes include without limitation, ischemia, hypoxia, cerebrovascular accident, trauma, surgery, pressure, mass effect, hemmorrhage, radiation, vasospasm, neurodegenerative disease or infection.

"Control population" is used herein to refer to mammals lacking a desired behavior associated with cognitive function, and usually includes mammals that are not young.

The term "covalent bond" is art-recognized and refers to a bond between two atoms where electrons are attracted electrostatically to both nuclei of the two atoms, and the net effect of increased electron density between the nuclei counterbalances the internuclear repulsion. The term covalent bond includes coordinate bonds when the bond is with a metal ion.

The terms "combinatorial library" or "library" are art-recognized and refer to a plurality of compounds, which may be termed "members," synthesized or otherwise prepared from one or more starting materials by employing either the same or different reactants or reaction conditions at each reaction in the library. There are a number of other terms of relevance to combinatorial libraries (as well as other technologies). The term "identifier tag" is art-recognized and refers to a means for recording a step in a series of reactions used in the synthesis of a chemical library. The term "immobilized" is art-recognized and, when used with respect to a species, refers to a condition in which the species is attached to a surface with an attractive force stronger than attractive forces that are present in the intended environment of use of the surface, and that act on the species. The term "solid support" is art-recognized and refers to a material which is an insoluble matrix, and may (optionally) have a rigid or semi-rigid surface. The term "linker" is art-recognized and refers to a molecule or group of molecules connecting a support, including a solid support or polymeric support, and a combinatorial library member. The term "polymeric support" is art-recognized and refers to a soluble or insoluble polymer to which a chemical moiety can be covalently bonded by reaction with a functional group of the polymeric support. The term "functional group of a polymeric support" is art-recognized and refers to a chemical moiety of a polymeric support that can react with an chemical moiety to form a polymer-supported amino ester.

"Derivative" is used herein to refer to the chemical modification of a compound, e.g., a cephalosporin or valproic acid. Chemical modifications of a compound can include, for example, replacement of hydrogen by an alkyl, acyl, or amino group. Many other modifications are also possible. A derivative of a compound retains at least one functional property of the original compound.

"Desired behavior" is used herein to refer to a behavioral manifestion of cognitive function as observed in a normal unimpaired subject. For example, in animals the desired behavior reflect the animals' cognitive function as measured on any one of a number of apparati, such as the Morris water maze, Barnes circle maze, elevated radial arm maze, T maze; or by any one of a number of tests, such as fear conditioning, active avoidance, illuminated open-field, dark activity meter, elevated plus-maze, two-compartment exploratory test or forced swimming test. In humans, the desired behavior reflect the subjects' cognitive function as measured by the ability of the subject to carry out daily activities of norman life or may be measured by performance on any number of tests for cognitive function including but not limited to ADAS-cog, CIBIC-plus scale, ADCS-ADL, MMSE, NPI, CDR, CANTAB or SCAG.

The term "heteroatom" is art-recognized and refers to an atom of any element other than carbon or hydrogen. Illustrative heteroatoms include boron, nitrogen, oxygen, phosphorus, sulfur and selenium.

The term "aryl" is art-recognized and refers to 5-, 6- and 7-membered single-ring aromatic groups that may include from zero to four heteroatoms, for example, benzene, pyrrole, furan, thiophene, imidazole, oxazole, thiazole, triazole, pyrazole, pyridine, pyrazine, pyridazine and pyrimidine, and the like. Those aryl groups having heteroatoms in the ring structure may also be referred to as "heteroaryl." The aromatic ring may be substituted at one or more ring positions with such substituents as described above, for example, halogen, azide, alkyl, aralkyl, alkenyl, alkynyl, cycloalkyl, hydroxyl, alkoxyl, amino, nitro, sulfhydryl, imino, amido, phosphonate, phosphinate, carbonyl, carboxyl, silyl, ether, alkylthio, sulfonyl, sulfonamido, ketone, aldehyde, ester, heterocyclyl, aromatic or heteroaromatic moieties, - CF₃, -CN, or the like. The term "aryl" also includes polycyclic ring systems having two or more cyclic rings in which two or more carbons are common to two adjoining rings (the rings are "fused rings") wherein at least one of the rings is aromatic, e.g., the other cyclic rings may be cycloalkyls, cycloalkenyls, cycloalkynyls, aryls and/or heterocyclyls.

The terms ortho, meta and para are art-recognized and refer to 1,2-, 1,3- and 1,4-disubstituted benzenes, respectively. For example, the names 1,2-dimethylbenzene and ortho-dimethylbenzene are synonymous.

The terms "heterocyclyl" or "heterocyclic group" are art-recognized and refer to 3- to about 10-membered ring structures, alternatively 3- to about 7-membered rings, whose ring structures include one to four heteroatoms. Heterocycles may also be polycycles. Heterocyclyl groups include, for example, thiophene, thianthrene, furan, pyran, isobenzofuran, chromene, xanthene, phenoxanthene, pyrrole, imidazole, pyrazole, isothiazole, isoxazole, pyridine, pyrazine, pyrimidine, pyridazine, indolizine, isoindole, indole, indazole, purine, quinolizine, isoquinoline, quinoline, phthalazine, naphthyridine, quinoxaline, quinazoline, cinnoline, pteridine, carbazole, carboline, phenanthridine, acridine, pyrimidine, phenanthroline, phenazine, phenarsazine, phenothiazine, furazan, phenoxazine, pyrrolidine, oxolane, thiolane, oxazole, piperidine, piperazine, morpholine, lactones, lactams such as azetidinones and pyrrolidinones, sultams, sultones, and the like. The heterocyclic ring may be substituted at one or more positions with such substituents as described above, as for example, halogen, alkyl, aralkyl, alkenyl, alkynyl, cycloalkyl, hydroxyl, amino, nitro, sulfhydryl, imino, amido, phosphonate, phosphinate, carbonyl, carboxyl, silyl, ether, alkylthio, sulfonyl, ketone, aldehyde, ester, a heterocyclyl, an aromatic or heteroaromatic moiety, -CF₃, -CN, or the like.

"Differentially expressed" is used herein to refer to the differing levels of expression, including both quantitative and qualitative measurements, of a gene of interest in tissues that have been treated differently or have been exposed to different environmental factors or changes in the physiological milieu.

"Gene" or "gene sequence" is used herein to refer to the partial or complete coding sequence of a gene, its compliment, and its 5' or 3' untranslated regions. The "coding sequence" of the gene is that set of nucleotides that are present in mRNA transcript of the gene. "Gene expression" refers to the process of making, or transcribing, an RNA based upon the DNA sequence of the gene. An "activator" of gene expression refers to a compound that stimulates the transcription of a gene's DNA sequence into a RNA transcript. "Endogenous" genes are genes naturally found within the species and not artificially incorporated, such as by random insertion or transfection, into the genome of an organism or cell.

"Glutamate transporter" is used herein to refer to transmembrane proteins that remove L-glutamate, the primary excitatory neurotransmitter in the mammalian central nervous system (CNS), from the extracellular space, including the synaptic cleft and extrasynaptic space. Glutamate transporters may be found in the membranes of both neurons and glial cells. Several glutamate transporters have been identified in humans and include, for example, Solute Carrier family 1, member 1 (SLC1A1 or EAAC1 or EAAT3; for example GenBank Accession No.:NM_004170), Solute Carrier family 1, member 2 (SLC1A2 or EAAT2 or GLT1; for example GenBank Accession No.:NM_004171), Solute Carrier family 1, member 3 (SLC1A3 or EAAT1, GLAST or GLAST1; for example GenBank Accession No.:NM_004172), Solute Carrier family 1, member 6 (SLC1A6 or EAAT4; for example GenBank Accession No.:NM_005071) and Solute Carrier family 1, member 7 (SLC1A7 or EAAT5; for example GenBank Accession No.:NM_006671). Further, glutamate transporters have been identified in *Rattus norvegicus* and *Mus musculus* (Slc1a1/Eaacl/REAAC1, Slc1a2/GluT/GLT-1/GluT-R, Slc1a3/Eaat1/GLAST/GluT-1 and Slcla6/Eaat4). Homologs of the foregoing are believed to exist in other mammals, including primates, canines, felines and rodents. The activity of a glutamate transporter protein is increased by administration of an agent that increases glutamate transporting activity of a glutamate transporter protein. Examples of agents reported to increase glutamate transport protein activativity include, for example, ((R)-(-)-5-methyl-1-nicotinoyl-2-pyrazoline (MS-153; Shimada et al., Eur J Pharmacol. 386:263-70, 1999); lidocaine (Do et al., Anesth Analg. 95:1263-8, 2002) and kinase inhibitors (e.g., Conradt, J Neurochem. 68:1244-51, 1997).

"Level of expression" of a gene is used herein to refer to the level of gene expression as measured by any method used to detect the presence of, a threshold amount of, a quantitative or qualitative measure of the expression of a gene- e.g. by measuring mRNA levels (e.g. by "Northern blot" or "microarray analysis") or protein (e.g. by detecting the amount of full-length or a truncated polypeptide gene product (e.g. immunologically with an antibody)).

The term "meso compound" is art-recognized and refers to a chemical compound which has at least two chiral centers but is achiral due to a plane or point of symmetry.

"Metabotropic glutamate receptor" (mGluR) is used herein to refer to the G protein-coupled receptors that respond to the neurotransmitter glutamate. Based upon their primary sequence similarity, signal transduction linkages and pharmacological profile, there are three groups of mGluR's. Group I consists of mGluRl (mGluR1a, mGluRlb, mGluR1c, mGluRld; e.g., GenBank Accession number NM_000838 for human splice variant mGluR1a) and mGluR5 (mGluR5a, mGluR5b; e.g., GenBank Accession number NM_000842 for human splice variant mGluR5a) that are positively coupled to phospholipase C. Group II consists of mGluR2 (e.g., GenBank Accession number NM_000839) and mGluR3 (e.g., GenBank Accession number NM_000840) that are negatively linked to adenyl cyclase. Group II consists of mGluR4 (mGluR4a, mGluR4b; e.g., GenBank Accession number NM_000841), mGluR6 (e.g., GenBank Accession number NM_000843), mGluR7 (mGluR7a, mGluR7b; e.g., GenBank Accession number NM_000844 for the human splice variant of mGluR7a) and mGluR8 (e.g., GenBank Accession number NM_000845) that are negatively linked to adenyl cyclase. There are a number of commercially available agonists and antagonists for the various mGluR groups. For example, Group I agonists include but are not limited to L-quisqualic acid ((L)-(+)-α-amino-3,5-dioxo-1,2,4-oxadiazolidine-2-propanoic acid), (S)-3,5-dihydroxyphenylglycine ((S)-3,5-DHPG), trans-azetidine-2,4-dicarboxylic acid (tADA), (1S,3R)-1-aminocyclopentane-1,3-dicarboxylic acid ((1S,3R)-ACPD) and (RS)-2-Chloro-5-hydroxyphenylglycine (CHPG); and antagonists include but are not limited to (S)-4-carboxy-3-hydroxyphenylglycine ((S)-4C3HPG), 7-(hydroxyimino)cyclopropa[b]chromen-1a-carboxylate ethyl ester (CPCCOEt), (RS)-1 aminoindan-1,5-dicarboxylic acid (AIDA; UPF 523), 2-methyl-6-(phenylethynyl)pyridine (MPEP hydrochloride), 2-methyl-6-(2-phenylethenyl) pyridine (SIB-1893), 6-methyl-2-(phenylazo)-3-pyridinol (SIB-1757), and (S)-(+)-α-amino-4-carboxy-2-methylbenzeneacetic acid (LY 367385). Group II agonists include (25,2'R,3'R)-2-(2',3'-dicarboxycyclopropyl)glycine (DCG IV), (2S,1'S,2'S)-2-(carboxycyclopropyl)glycine (L-CCG-I; (2S,3S,4S)-CCG), (S)-3 carboxy-4-hydroxyphenylglycine ((S)-3C4HPG) and (2R,4R)-4-aminopyrrolidine-2,4-dicarboxylate ((2R,4R)-APDC); and antagonists include (2S)-α-Ethylglutamic acid (EGLU) and (2S)-2-amino-2-[(1S,2S)-2-carboxycycloprop-1-yl]-3-(xanth-9-yl) propanoic acid (LY 341495). Group III agonist include (1S,3R,4S)-1-aminocyclopentane-1,2,4-tricarboxylic acid (ACPT-I), L(+)-2-amino-4-phosphonobutyric acid (L-AP4), (R,S)-4-phosphonophenylglycine ((R,S)-PPG) and O-phospho-L-serine (L-SOP); and antagonists include (RS)-α-Cyclopropyl-4-phosphonophenylglycine (CPPG), (S)-2-amino-2-methyl-4-phosphonobutanoic acid (MAP4) and (RS)-α-Methylserine-O-phosphate (MSOP). Recent evidence has shown that metabotropic glutamate receptors associated with glia can alter the expression of glutamate transporters (Aronica et al., Eur. J. Neurosci. 2003; 17: 2106-18, 2003).

"Middle-age" is used herein to refer to a mammal that is past the age of sexual maturity, i.e., not young, but not yet approaching the average life span of the species, i.e., not aged. For example, a middle-aged rat would be of about 12-18 months of age. A middle-aged human would be of between twenty and seventy years of age.

"Neural tissue" is used herein to refer to tissues of the nervous system, ie., tissues comprising both neurons and glia. Where specified, neural tissue may refer to particular structures found in the brain, including "hippocampal tissue." Hippocampal tissue refers to the seahorse shaped structure found in the temporal cortex that includes the following: entorhinal cortex, presubiculum, subiculum, prosubiculum, dentate gyrus, and areas known as CA1, CA2, CA3 and CA4. The hippocampus is involved in processes such as short-term memory, the formation of long-term memory, memory retrieval, declarative memory and spatial navigation.

"Neuroprotective" is used herein to refer to compositions and treatments that have the effect of reducing, arresting or ameliorating impaired cognitive function, and protecting, resuscitating, or reviving nervous tissue that has suffered impaired cognitive function.

The term "nitro" is art-recognized and refers to -NO₂; the term "halogen" is art-recognized and refers to -F, -Cl, -Br or -I; the term "sulfhydryl" is art-recognized and refers to -SH; the term "hydroxyl" means -OH; and the term "sulfonyl" is art-recognized and refers to -SO₂⁻. "Halide" designates the corresponding anion of the halogens, and "pseudohalide" has the definition set forth on 560 of "Advanced Inorganic Chemistry" by Cotton and Wilkinson.

The term "phosphoryl" is art-recognized and may in general be represented by the formula: 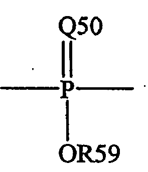 wherein Q50 represents S or O, and R59 represents hydrogen, a lower alkyl or an aryl. When used to substitute, e.g., an alkyl, the phosphoryl group of the phosphorylalkyl may be represented by the general formulas: 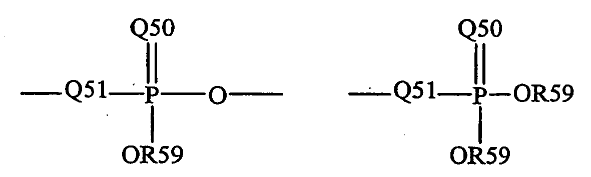 wherein Q50 and R59, each independently, are defined above, and Q51 represents O, S or N. When Q50 is S, the phosphoryl moiety is a "phosphorothioate".

The term "phosphoramidite" is art-recognized and may be represented in the general formulas: 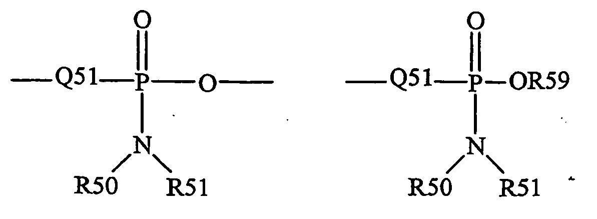 wherein Q51, R50, R51 and R59 are as defined above.

The term "phosphonamidite" is art-recognized and may be represented in the general formulas: 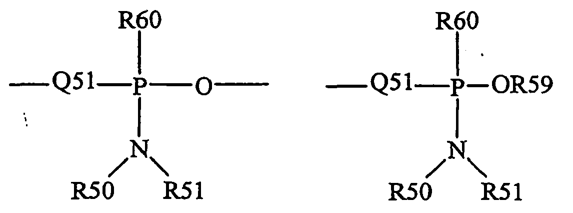 wherein Q51, R50, R51 and R59 are as defined above, and R60 represents a lower alkyl or an aryl.

Analogous substitutions may be made to alkenyl and alkynyl groups to produce, for example, aminoalkenyls, aminoalkynyls, amidoalkenyls, amidoalkynyls, iminoalkenyls, iminoalkynyls, thioalkenyls, thioalkynyls, carbonyl-substituted alkenyls or alkynyls.

The definition of each expression, e.g. alkyl, m, n, and the like, when it occurs more than once in any structure, is intended to be independent of its definition elsewhere in the same structure.

The term "selenoalkyl" is art-recognized and refers to an alkyl group having a substituted seleno group attached thereto. Exemplary "selenoethers" which may be substituted on the alkyl are selected from one of -Se-alkyl, -Se-alkenyl, -Se-alkynyl, and -Se-(CH₂)ₘ-R61, m and R61 being defined above.

The terms triflyl, tosyl, mesyl, and nonaflyl are art-recognized and refer to trifluoromethanesulfonyl, *p*-toluenesulfonyl, methanesulfonyl, and nonafluorobutanesulfonyl groups, respectively. The terms triflate, tosylate, mesylate, and nonaflate are art-recognized and refer to trifluoromethanesulfonate ester, *p*-toluenesulfonate ester, methanesulfonate ester, and nonafluorobutanesulfonate ester functional groups and molecules that contain said groups, respectively.

The abbreviations Me, Et, Ph, Tf, Nf, Ts, and Ms represent methyl, ethyl, phenyl, trifluoromethanesulfonyl, nonafluorobutanesulfonyl, *p*-toluenesulfonyl and methanesulfonyl, respectively. A more comprehensive list of the abbreviations utilized by organic chemists of ordinary skill in the art appears in the first issue of each volume of the Journal of Organic Chemistry; this list is typically presented in a table entitled Standard List of Abbreviations.

"Pituitary adenyl cyclase activator polypeptide" (PACAP) is used herein to refer to the neuropolypeptide that is a potent activator of cAMP-dependent signaling pathways. PACAP acts as a multifunctional peptide and is involved in such diverse processes as the regulation of hormonal secretion, energy metabolism, neuronal survival and is a regulator of glial glutamate transporters EAAT1 and EAAT2 (Figiel and Engele, J. Neurosci. 15: 3596-3605, 2000). PACAP belongs to the secretin/glucagon/vasoactive intestinal peptide (VIP) superfamily, and exists in two amidated forms as PACAP38 (38-amino acid residues) and PACAP27 (27-amino acid residues) derived from the same precursor. The primary structure of PACAP has been remarkably conserved throughout evolution among tunicata, ichthyopsida, amphibia and mammalia, and a PACAP-like neuropeptide has also been determined in Drosophila. Besides PACAP-38 and PACAP-27, a third agonist of the PACAP receptor is Maxadilian. Maxadilan is a potent vasodilator peptide isolated from salivary glands extracts of the hematophagous sand fly. Recently, it was demonstrated that maxadilan binds to PACAP receptor type 1 in mammals, although maxadilan has no significant amino acid sequence homology with PACAP (Moro and Lerner: Maxadilan, J. Biol. Chem. 272(2):966-70, 1997). Both PACAP and its receptors are mainly distributed in the nervous and endocrine systems showing pleiotropic functions with high potency. Thus, PACAP peptides, Maxadilan or peptide derivatives and analogs, peptide-like compounds and small-molecule agonists triggering the PACAP receptor can be used to increase glutamate transporter activity.

The terms "polycyclyl" or "polycyclic group" are art-recognized and refer to two or more rings (e.g., cycloalkyls, cycloalkenyls, cycloalkynyls, aryls and/or heterocyclyls) in which two or more carbons are common to two adjoining rings, e.g., the rings are "fused rings". Rings that are joined through non-adjacent atoms are termed "bridged" rings. Each of the rings of the polycycle may be substituted with such substituents as described above, as for example, halogen, alkyl, aralkyl, alkenyl, alkynyl, cycloalkyl, hydroxyl, amino, nitro, sulfhydryl, imino, amido, phosphonate, phosphinate, carbonyl, carboxyl, silyl, ether, alkylthio, sulfonyl, ketone, aldehyde, ester, a heterocyclyl, an aromatic or heteroaromatic moiety, -CF₃, -CN, or the like.

"Plurality" is used herein to refer to two or more.

The term "prodrug" is art-recognized and is intended to encompass compounds which, under physiological conditions, are converted into the antibacterial agents . A common method for making a prodrug is to select moieties which are hydrolyzed under physiological conditions to provide the desired compound. In other embodiments, the prodrug is converted by an enzymatic activity of the host animal.

The term "protecting group" is art-recognized and refers to temporary substituents that protect a potentially reactive functional group from undesired chemical transformations. Examples of such protecting groups include esters of carboxylic acids, silyl ethers of alcohols, and acetals and ketals of aldehydes and ketones, respectively. The field of protecting group chemistry has been reviewed by Greene and Wuts in Protective Groups in Organic Synthesis (2nd ed., Wiley: New York, 1991).

The term "hydroxyl-protecting group" is art-recognized and refers to those groups intended to protect a hydrozyl group against undesirable reactions during synthetic procedures and includes, for example, benzyl or other suitable esters or ethers groups known in the art.

The term "carboxyl-protecting group" is art-recognized and refers to those groups intended to protect a carboxylic acid group, such as the C-terminus of an amino acid or peptide or an acidic or hydroxyl azepine ring substituent, against undesirable reactions during synthetic procedures and includes. Examples for protecting groups for carboxyl groups involve, for example, benzyl ester, cyclohexyl ester, 4-nitrobenzyl ester, t-butyl ester, 4-pyridylmethyl ester, and the like.

The term "amino-blocking group" is art-recognized and refers to a group which will prevent an amino group from participating in a reaction carried out on some other functional group, but which can be removed from the amine when desired. Such groups are discussed by in Ch. 7 of Greene and Wuts, cited above, and by Barton, Protective Groups in Organic Chemistry ch. 2 (McOmie, ed., Plenum Press, New York, 1973). Examples of suitable groups include acyl protecting groups such as, to illustrate, formyl, dansyl, acetyl, benzoyl, trifluoroacetyl, succinyl, methoxysuccinyl, benzyl and substituted benzyl such as 3,4-dimethoxybenzyl, o-nitrobenzyl, and triphenylmethyl; those of the formula -COOR where R includes such groups as methyl, ethyl, propyl, isopropyl, 2,2,2-trichloroethyl, 1-methyl-1-phenylethyl, isobutyl, t-butyl, t-amyl, vinyl, allyl, phenyl, benzyl, p-nitrobenzyl, o-nitrobenzyl, and 2,4-dichlorobenzyl; acyl groups and substituted acyl such as formyl, acetyl, chloroacetyl, dichloroacetyl, trichloroacetyl, trifluoroacetyl, benzoyl, and p-methoxybenzoyl; and other groups such as methanesulfonyl, p-toluenesulfonyl, p-bromobenzenesulfonyl, p-nitrophenylethyl, and p-toluenesulfonyl-aminocarbonyl. Preferred amino-blocking groups are benzyl (-CH₂C₆H₅), acyl [C(O)R1] or SiR1₃ where R1 is C₁-C₄ alkyl, halomethyl, or 2-halo-substituted-(C₂-C₄ alkoxy), aromatic urethane protecting groups as, for example, carbonylbenzyloxy (Cbz); and aliphatic urethane protecting groups such as t-butyloxycarbonyl (Boc) or 9-fluorenylmethoxycarbonyl (FMOC).

The definition of each expression, e.g. lower alkyl, m, n, p and the like, when it occurs more than once in any structure, is intended to be independent of its definition elsewhere in the same structure.

The term "electron-withdrawing group" is art-recognized, and refers to the tendency of a substituent to attract valence electrons from neighboring atoms, i.e., the substituent is electronegative with respect to neighboring atoms. A quantification of the level of electron-withdrawing capability is given by the Hammett sigma (σ) constant. This well known constant is described in many references, for instance, March, Advanced Organic Chemistry 251-59 (McGraw Hill Book Company: New York, 1977). The Hammett constant values are generally negative for electron donating groups (σ(P) = - 0.66 for NH₂) and positive for electron withdrawing groups (σ(P) = 0.78 for a nitro group), σ(P) indicating para substitution. Exemplary electron-withdrawing groups include nitro, acyl, formyl, sulfonyl, trifluoromethyl, cyano, chloride, and the like. Exemplary electron-donating groups include amino, methoxy, and the like.

"RNA" is used herein to refer to the various species of ribonucleic acids, such as messenger RNA, mature RNA, polyadenylated RNA, unpolyadenylated RNA and RNA that contains introns and/or 5' or 3' untranslated regions. "Expressed RNA" is used herein to refer to RNA that is transcribed from genomic or mitochondrial DNA by a polymerase.

The term "regioisomers" is art-recognized and refers to compounds which have the same molecular formula but differ in the connectivity of the atoms. Accordingly, a "regioselective process" is one which favors the production of a particular regioisomer over others, e.g., the reaction produces a statistically significant increase in the yield of a certain regioisomer.

The term "epimers" is art-recognized and refers to molecules with identical chemical constitution and containing more than one stereocenter, but which differ in configuration at only one of these stereocenters.

"Small molecule" is used herein to refer to a composition, which has a molecular weight of less than about 5 kD and most preferably less than about 4 kD. Small molecules can be nucleic acids, peptides, polypeptides, peptidomimetics, carbohydrates, lipids or other organic (carbon-containing) or inorganic molecules. Many pharmaceutical companies and suppliers have extensive libraries of chemical and/or biological mixtures, often fungal, bacterial, or algal extracts, which can be screened with any of the assays disclosed to identify compounds that modulate a bioactivity, such as a desired behavior or cognitive function.

The term "stereoisomers" is art-recognized and refers to compounds which have identical chemical constitution, but differ with regard to the arrangement of the atoms or groups in space. In particular, "enantiomers" refer to two stereoisomers of a compound which are non-superimposable mirror images of one another. "Diastereomers", on the other hand, refers to stereoisomers with two or more centers of dissymmetry and whose molecules are not mirror images of one another.

Furthermore, a "stereoselective process" is one which produces a particular stereoisomer of a reaction product in preference to other possible stereoisomers of that product. An "enantioselective process" is one which favors production of one of the two possible enantiomers of a reaction product.

The term "structure-activity relationship" or "(SAR)" is art-recognized and refers to the way in which altering the molecular structure of a drug or other compound alters its interaction with a receptor, enzyme, nucleic acid or other target and the like.

"Subject" is used herein to refer to a mammal, e.g., a human, non-human primate, ovine, bovine, porcine, equine, feline, murine or canine. Preferably, the subject is a human. A subject or mammal "in need of" treatment has impaired cognitive function that can be ameliorated by the methods and compositions described herein. It will be understood that "substitution" or "substituted with" includes the implicit proviso that such substitution is in accordance with permitted valence of the substituted atom and the substituent, and that the substitution results in a stable compound, e.g., which does not spontaneously undergo transformation such as by rearrangement, cyclization, elimination, or other reaction.

The term "substituted" is also contemplated to include all permissible substituents of organic compounds. In a broad aspect, the permissible substituents include acyclic and cyclic, branched and unbranched, carbocyclic and heterocyclic, aromatic and nonaromatic substituents of organic compounds. Illustrative substituents include, for example, those described herein above. The permissible substituents may be one or more and the same or different for appropriate organic compounds. For purposes disclosed, the heteroatoms such as nitrogen may have hydrogen substituents and/or any permissible substituents of organic compounds described herein which satisfy the valences of the heteroatoms. This disclosure is not intended to be limited in any manner by the permissible substituents of organic compounds.

The term "sulfonate" is art recognized and refers to a moiety that may be represented by the general formula: 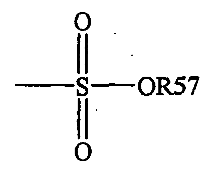 in which R57 is an electron pair, hydrogen, alkyl, cycloalkyl, or aryl.

The term "sulfate" is art recognized and includes a moiety that may be represented by the general formula: 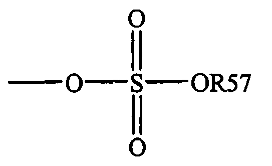 in which R57 is as defined above.

The term "sulfonamido" is art recognized and includes a moiety that may be represented by the general formula: 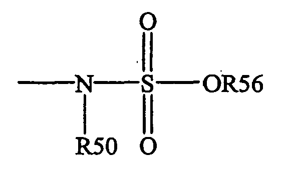 in which R50 and R56 are as defined above.

The term "sulfamoyl" is art-recognized and refers to a moiety that may be represented by the general formula: 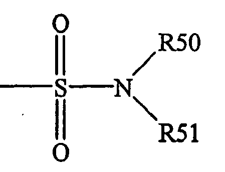 in which R50 and R51 are as defined above.

The term "sulfonyl" is art-recognized and refers to a moiety that may be represented by the general formula: 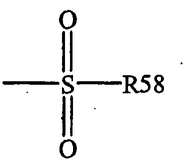 in which R58 is one of the following: hydrogen, alkyl, alkenyl, alkynyl, cycloalkyl, heterocyclyl, aryl or heteroaryl.

The term "sulfoxido" is art-recognized and refers to a moiety that may be represented by the general formula: 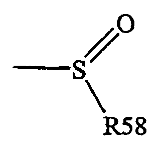 in which R58 is defined above.

The term "synthetic" is art-recognized and refers to production by in vitro chemical or enzymatic synthesis.

"Test population" is used herein to refer to subjects having a desired behavior or cognitive function. Members of the test population may include young, middle-aged and aged subjects.

"Therapeutic agent" is used herein to refer to a chemical compound or composition capable of inducing a desired therapeutic or prophylactic effect when properly administered to a subject in need thereof. The "therapeutic agent" may be any chemical moiety or biologic that is a biologically, physiologically, or pharmacologically active substance that acts locally or systemically in a subject in need thereof. Examples of chemical therapeutic agents, also referred to as "drugs", are described in well-known literature references such as the Merck Index, the Physicians Desk Reference, and The Pharmacological Basis of Therapeutics, and they include, without limitation, medicaments; vitamins; mineral supplements; substances used for the treatment, prevention, diagnosis, cure or mitigation of a disease or illness; substances which affect the structure or function of the body; or pro-drugs, which become biologically active or more active after they have been placed in a physiological environment. Antibiotic agents and Fab I/Fab K inhibitors are examples of therapeutic agents. Examples of biologic therapeutic agents include viral vectors that contain genes and deliver the gene to the subj ect.

Therapeutic agents induce a local or systemic effect in animals, particularly mammals, and more particularly humans caused by a pharmacologically active substance. Thus, a therapeutic agent may be used for the diagnosis, cure, mitigation, treatment or prevention of deleterious condition or in the enhancement of desirable physical or mental development and/or conditions in an animal or human.

To be effective, a therapeutic agent are delivered in an amount or concentration that produces some desired local or systemic effect at a reasonable benefit/risk ratio applicable to any treatment. The effective amount of such therapeutic agent will vary depending upon the subject and condition being treated, the weight and age of the subject, the severity of the disease condition, the manner of administration and the like, which can readily be determined by one of ordinary skill in the art. For example, certain compositions disclosed may be administered in a sufficient amount to produce an effect at a reasonable benefit/risk ratio applicable to such treatment. In the context of impaired cognitive function the presence of degree of therapeutic effect can be assessed using standard behavioral or other tests known in the art for assessing cognitive function.

The term "trans" is art-recognized and refers to the arrangement of two atoms or groups around a double bond such that the atoms or groups are on the opposite sides of a double bond. Trans configurations are often labeled as (E) configurations.

"Treating" impaired cognitive function in a subject or "treating" a subject having impaired cognitive function are used herein to refer to providing the subject with a therapeutic agent by any appropriate means, e.g., the administration of a drug, such that at least one symptom of the impaired cognitive function is stabilized or decreased. Treating impaired cognitive function can be preventing the impairment, delaying progression of the impairment or improving the impairment (lessening disease severity) or curing the impairment.

"Vector" is used herein to refer to compositions that may be used to introduce DNA or RNA into tissue. Methods which are well known to those skilled in the art can be used to construct expression vectors containing a nucleic acid encoding the protein of interest linked to appropriate transcriptional/translational control signals. See, for example, the techniques described in Sambrook & Russell, Molecular Cloning, A Laboratory Manual (3rd Edition), Cold Spring Harbor Laboratory, N.Y. (2001) and Ausebel et al. Current Protocols in Molecular Biology, Greene Publishing Associates & Wiley Interscience, N.Y (1989).

Suitable methods for transferring vectors or plasmids into cells include lipid/DNA complexes, such as those described in U.S. Pat. Nos. 5,578,475; 5,627,175; 5,705,308; 5,744,335; 5,976,567; 6,020,202; and 6,051,429. Suitable reagents include lipofectamine, a 3:1 (w/w) liposome formulation of the poly-cationic lipid 2,3-dioleyloxy-N-[2(sperminecarbox-amido)ethyl]-N,N-dimethyl-1-propanaminium trifluoroacetate (DOSPA) (Chemical Abstracts Registry name: N-[2-(2,5-bis[(3-aminopropyl)amino]-1-oxpentyl)amino)ethyl]-N,N-dimethyl-2,3-bis(9-octadecenyloxy)-1-propanamin-trifluoroacetate), and the neutral lipid dioleoyl phosphatidylethanolamine (DOPE) in membrane filtered water. Exemplary is the formulation Lipofectamine 2000^{™} (available from Invitrogen (formerly Gibco/Life Technologies) # 11668019). Other reagents include: FuGENE™ 6 Transfection Reagent (a blend of lipids in non-liposomal form and other compounds in 80% ethanol, obtainable from Roche Diagnostics Corp. # 1814443); and LipoTAXI™ transfection reagent (a lipid formulation from Invitrogen Corp., produce the desired biologically active protein. #204110). Transfection of cells can be performed by electroporation, e.g., as described in Roach and McNeish (Methods in Mol. Biol. 185:1 (2002)). Suitable viral vector systems for producing cells with stable genetic alterations may be based on adenoviruses, lentiviruses, retroviruses, adeno-associated viruses (AAV) and other viruses, and may be prepared using commercially available virus components. Vectors can be introduced into neural cells and tissues by art-known methods, including injection (e.g., in to a specified region of the brain), by use of a shunt to the ventricular space or cerebrospinal fluid and other mechanical means.

"Young" refers to adolescents and normal adult mammals at about the age of sexual maturity and when the hippocampus has just fully mature. In the case of rats, a "young" rat would be 6-9 months of age. In the case of humans, a "young" human would be 10-20 years of age.

### 6.2 Introduction: Combination Studies of Behavioral and Genetic Assessments of Cognitive Function

Behavioral assessments of cognitive function with the Morris Water Maze and Radial Arm Maze have been useful in identifying age-related changes in cognitive function. Upon using these behavioral assessments as a method for phenotyping animals on the basis of their cognitive function, one may combine behavioral assessments with genetic and physiological measurements of cognitive function to detect differences in the effects of aging on the brain.

The use of gene expression arrays offers the potential to simultaneously analyze up to thousands of expressed genes in order to gain a genetic template of age- and behavior-associated changes in the brain. Such approaches also offer some challenges. First, our rat model, like the aging human population comprises a genetically outbred population which can add individual variability as a confounding factor in gene expression profiling. Second, using traditional quantitative methods to assess levels of specific mRNAs in hippocampus, we have found that age and behavior related changes in gene expression are often relatively small, smaller than the two fold differences in levels of gene expression which have been reported as the limits of discriminating power in existing Genechip® or microarray approaches. For example, work by Landfield and colleagues was limited by not being able to detect less than two-fold changes in gene expression (WO 03/025122 A2).

Herein we describe strategies which overcome such challenges, demonstrating reliable detection of small changes in expression of genes which we have shown by traditional methods to differ between aged and young rats. Analyses of a wider range of genes indicate that this method reproducibly reveals a substantial number of genes which show changes in expression within the hippocampus that are associated with behavioral status in aged rats.

The identification of genes associated with cognitive impairment allows one for the first time to determine whether a candidate compound can modulate expression of genes associated with normal cognitive function. Compounds that modulate expression of such genes so as to more closely approximate the level of expression thereof in a mammal, e.g., a human, having a desired cognitive function are expected to restore or improve cognitive function when used as therapeutic agents. Using this approach, we report the discovery of genes assoicated with the preservation of cognitive function in aged mammals. Without being limited by speculation, we believe that such preservation represents an active biological process that can be triggered or induced by treatment with appropriate therapeutic agents. Indeed, we report herein that one such agent is ceftriaxone, a third generation cephalosporin. Other such agents are valproic acid and MS-153. Additional such therapeutic agents can be identified and optimized using the screening methods described below.

### 6.2.1 Isolating RNA

When isolating RNA from tissue samples or cells from individuals, it may be important to prevent any further changes in gene expression after the tissue or cells has been removed from the subject. Changes in expression levels are known to occur rapidly following perturbations, e.g., heat shock or activation with lipopolysaccharide (LPS) or other reagents. In addition, the RNA in the tissue and cells may quickly become degraded. Accordingly, in a preferred embodiment, the tissue or cells obtained from a subject is snap frozen as soon as possible.

RNA can be extracted from the tissue sample by a variety of methods, e.g., those described in the Examples or guanidium thiocyanate lysis followed by CsCl centrifugation (Chirgwin et al., 1979, Biochemistry 18:5294-5299). RNA from frozen tissue can be isolated by homogenizing the tissue in a phenol/guanidinium thiocyanate mixture (available from Invitrogen) and extracted with chloroform followed by precipitation with isopropanol. The RNA pellet can then be resuspended and further purified over RNeasy columns (Qiagen). All RNA may be stored at -80° in the absence of RNase inhibitors and the integrity assessed by agarose gel electrophoreisis. RNA from single cells can be obtained as described in methods for preparing cDNA libraries from single cells, such as those described in Dulac, C. (1998) Curr. Top. Dev. Biol. 36,245 and Jena et al. (1996) J. Immunol. Methods 190:199. Care to avoid RNA degradation must be taken, e.g., by inclusion of RNAse inhibitor.

The RNA sample can then be enriched in particular species. In one embodiment, poly(A)+ RNA is isolated from the RNA sample. In general, such purification takes advantage of the poly-A tails on mRNA. In particular and as noted above, poly-T oligonucleotides may be immobilized within on a solid support to serve as affinity ligands for mRNA. Kits for this purpose are commercially available, e.g., the MessageMaker kit (Invitrogen #10298016).

In a preferred embodiment, the RNA population is enriched in sequences of interest, such as those of genes involved in cognitive function. Enrichment can be undertaken, e.g., by primer-specific cDNA synthesis, or multiple rounds of linear amplification based on cDNA synthesis and template-directed in vitro transcription (see, e.g., Wang et al. (1989) PNAS 86, 9717; Dulac et al., supra, and Jena et al., supra).

The population of RNA, enriched or not in particular species or sequences, can further be amplified. Such amplification is particularly important when using RNA from a single or a few cells. A variety of amplification methods are suitable for use in the methods disclosed, including, e.g., PCR; ligase chain reaction (LCR) (see, e.g., Wu and Wallace, Genomics 4, 560 (1989), Landegren et al., Science 241, 1077 (1988)); self-sustained sequence replication (SSR) (see, e.g., Guatelli et al., Proc. Nat. Acad. Sci. USA, 87, 1874 (1990)); nucleic acid based sequence amplification (NASBA) and transcription amplification (see, e.g., Kwoh et al., Proc. Natl. Acad. Sci. USA 86, 1173 (1989)). For PCR technology, see, e.g., PCR Technology: Principles and Applications for DNA Amplification (ed. H. A. Erlich, Freeman Press, N.Y., N.Y., 1992); PCR Protocols: A Guide to Methods and applications (eds. Innis, et al., Academic Press, San Diego, Calif., 1990); Mattila et al., Nucleic Acids Res. 19,4967 (1991); Eckert et al., PCR Methods and Applications 1, 17 (1991); PCR (eds. McPherson et al., IRL Press, Oxford); and U.S. Pat. No. 4,683,202. Methods of amplification are described, e.g., in Ohyama et al. (2000) BioTechniques 29:530; Luo et al. (1999) Nat. Med. 5,117; Hegde et al. (2000) BioTechniques 29:548; Kacharmina et al. (1999) Meth. Enzymol. 303:3; Livesey et al. (2000) Curr. Biol. 10:301; Spirin et al. (1999) Invest. Ophtalmol. Vis. Sci. 40:3108; and Sakai et al. (2000) Anal. Biochem. 287:32. RNA amplification and cDNA synthesis can also be conducted in cells in situ (see, e.g., Eberwine et al. (1992) PNAS 89:3010). "Quantitative PCR" refers to using a PCR protocol that allows one to determine the amount of reaction product or number of reaction products in a sample.

One of skill in the art will appreciate that whatever amplification method is used, if a quantitative result is desired, care must be taken to use a method that maintains or controls for the relative frequencies of the amplified nucleic acids to achieve quantitative amplification. Methods of "quantitative" amplification are well known to those of skill in the art. For example, quantitative PCR involves simultaneously co-amplifying a known quantity of a control sequence using the same primers. This provides an internal standard that may be used to calibrate the PCR reaction. A high density array may then include probes specific to the internal standard for quantification of the amplified nucleic acid.

One preferred internal standard is a synthetic AW106 cRNA. The AW106 cRNA is combined with RNA isolated from the sample according to standard techniques known to those of skilled in the art. The RNA is then reverse transcribed using a reverse transcriptase to provide copy DNA. The cDNA sequences are then amplified (e.g., by PCR) using labeled primers. The amplification products are separated, typically by electrophoresis, and the amount of radioactivity (proportional to the amount of amplified product) is determined. The amount of mRNA in the sample is then calculated by comparison with the signal produced by the known AW106 RNA standard. Detailed protocols for quantitative PCR are provided in PCR Protocols, A Guide to Methods and Applications, Innis et al., Academic Press, Inc. N.Y., (1990).

In a preferred embodiment, a sample mRNA is reverse transcribed with a reverse transcriptase and a primer consisting of oligo(dT) and a sequence encoding the phage T7 promoter to provide single stranded DNA template. The second DNA strand is polymerized using a DNA polymerase. After synthesis of double-stranded cDNA, T7 RNA polymerase is added and RNA is transcribed from the cDNA template. Successive rounds of transcription from each single cDNA template results in amplified RNA. Methods of in vitro polymerization are well known to those of skill in the art (see, e.g., Sambrook & Russell, (supra) and this particular method is described in detail by Van Gelder, et al., Proc. Natl. Acad. Sci. USA, 87: 1663-1667 (1990) who demonstrate that in vitro amplification according to this method preserves the relative frequencies of the various RNA transcripts). Moreover, Eberwine et al. Proc. Natl. Acad. Sci. USA, 89: 3010-3014 provide a protocol that uses two rounds of amplification via in vitro transcription to achieve greater than 106 fold amplification of the original starting material, thereby permitting expression monitoring even where biological samples are limited.

It will be appreciated by one of skill in the art that the direct transcription method described above provides an antisense (aRNA) pool. Where antisense RNA is used as the target nucleic acid, the oligonucleotide probes provided in the array are chosen to be complementary to subsequences of the antisense nucleic acids. Conversely, where the target nucleic acid pool is a pool of sense nucleic acids, the oligonucleotide probes are selected to be complementary to subsequences of the sense nucleic acids. Finally, where the nucleic acid pool is double stranded, the probes may be of either sense as the target nucleic acids include both sense and antisense strands.

### 6.2.2 Analyzing RNA

In certain embodiments, it is sufficient to determine the expression of one or only a few genes, as opposed to hundreds or thousands of genes. Although microarrays can be used in these embodiments, various other methods of detection of gene expression are available. This section describes a few exemplary methods for detecting and quantifying mRNA or polypeptide encoded thereby. Where the first step of the methods includes isolation of mRNA from cells, this step can be conducted as described above. Labeling of one or more nucleic acids can be performed as described below.

In one embodiment, mRNA obtained from a sample is reverse transcribed into a first cDNA strand and subjected to PCR, e.g., RT-PCR. House keeping genes, or other genes whose expression does not vary can be used as internal controls and controls across experiments. Following the PCR reaction, the amplified products can be separated by electrophoresis and detected. By using quantitative PCR, the level of amplified product will correlate with the level of RNA that was present in the sample. The amplified samples can also be separated on a agarose or polyacrylamide gel, transferred onto a filter, and the filter hybridized with a probe specific for the gene of interest. Numerous samples can be analyzed simultaneously by conducting parallel PCR amplification, e.g., by multiplex PCR.

A quantitative PCR technique that can be used is based on the use of TaqMan^{™} probes. Specific sequence detection occurs by amplification of target sequences in the PE Applied Biosystems 7700 Sequence Detection System in the presence of an oligonucleotide probe labeled at the 5' and 3' ends with a reporter and quencher fluorescent dye, respectively (FQ probe), which anneals between the two PCR primers. Only specific product will be detected when the probe is bound between the primers. As PCR amplification proceeds, the 5'-nuclease activity of Taq polymerase initially cleaves the reporter dye from the probe. The signal generated when the reporter dye is physically separated from the quencher dye is detected by measuring the signal with an attached CCD camera. One can also use an intercalating dye such as sybr green. Each signal generated equals one probe cleaved which corresponds to amplification of one target strand. PCR reactions may be set up using the PE Applied Biosystem TaqMan PCR Core Reagent Kit according to the instructions supplied. This technique is further described, e.g., in U.S. Patent 6,326,462. Alternatively, probes can be obtained from Applied Biosystems and Qiagen for use with Invitrogen's Platinum quantitative PCR kit and the Rotorgene 3000.

In another embodiment, mRNA levels is determined by dotblot analysis and related methods (see, e.g., G. A. Beltz et al., in Methods in Enzymology, Vol. 100, Part B, R. Wu, L. Grossmam, K. Moldave, Eds., Academic Press, New York, Chapter 19, pp. 266-308, 1985). In one embodiment, a specified amount of RNA extracted from cells is blotted (i.e., non-covalently bound) onto a filter, and the filter is hybridized with a probe of the gene of interest. Numerous RNA samples can be analyzed simultaneously, since a blot can comprise multiple spots of RNA. Hybridization is detected using a method that depends on the type of label of the probe. In another dotblot method, one or more probes of one or more genes which are up- or down-regulated in cognitive impairment are attached to a membrane, and the membrane is incubated with labeled nucleic acids obtained from and optionally derived from RNA of a cell or tissue of a subject. Such a dot blot is essentially an array comprising fewer probes than a microarray.

"Dot blot" hybridization gained wide-spread use, and many versions were developed (see, e.g., M. L. M. Anderson and B. D. Young, in Nucleic Acid Hybridization-A Practical Approach, B. D. Hames and S. J. Higgins, Eds., IRL Press, Washington D.C., Chapter 4, pp. 73-111, 1985).

Another format, the so-called "sandwich" hybridization, involves covalently attaching oligonucleotide probes to a solid support and using them to capture and detect multiple nucleic acid targets (see, e.g., M. Ranki et al., Gene, 21, pp. 77-85, 1983; A. M. Palva, T. M. Ranki, and H. E. Soderlund, in UK Patent Application GB 2156074A, Oct. 2, 1985; T. M. Ranki and H. E. Soderlund in U.S. Pat. No. 4,563,419, Jan. 7, 1986; A. D. B. Malcolm and J. A. Langdale, in PCT WO 86/03782, Jul. 3, 1986; Y. Stabinsky, in U.S. Pat. No. 4,751,177, Jan. 14, 1988; T. H. Adams et al., in PCT WO 90/01564, Feb. 22, 1990; R. B. Wallace et al. 6 Nucleic Acid Res. 11, p. 3543, 1979; and B. J. Connor et al., 80 Proc. Natl. Acad. Sci. USA pp. 278-282, 1983). Multiplex versions of these formats are called "reverse dot blots."

mRNA levels can also be determined by Northern blots. Specific amounts of RNA are separated by gel electrophoresis and transferred onto a filter which are then hybridized with a probe corresponding to the gene of interest. This method, although more burdensome when numerous samples and genes are to be analyzed provides the advantage of being very accurate.

A preferred method for high throughput analysis of gene expression is the serial analysis of gene expression ("SAGE") technique, first described in Velculescu et al. (1995) Science 270, 484-487. Among the advantages of SAGE is that it has the potential to provide detection of all genes expressed in a given cell type, provides quantitative information about the relative expression of such genes, permits ready comparison of gene expression of genes in two cells, and yields sequence information that can be used to identify the detected genes. Thus far, SAGE methodology has proved itself to reliably detect expression of regulated and nonregulated genes in a variety of cell types (Velculescu et al. (1997) Cell 88, 243-251; Zhang et al. (1997) Science 276, 1268-1272 and Velculescu et al. (1999) Nat. Genet. 23, 387-388).

Techniques for producing and probing nucleic acids are further described, for example, in Sambrook & Russell, (supra).

Alternatively, the level of expression of one or more genes which are up- or down-regulated in cognitive impairment is determined by in situ hybridization histochemistry. In one embodiment, a tissue sample is obtained from a subject, a thin section is prepared, and in situ hybridization is performed according to methods known in the art, to determine the level of expression of the genes of interest.

The above methods may be used to assess an increase expression of an endogenous gene that may be activated by introducing into the mammal a new transcriptional unit, or gene activation construct, that comprises an exogenous regulatory sequence, an exogenous exon, and a splice site, operably linked to the second exon of an endogenous gene, wherein the cell comprises the exogenous exon in addition to exons present in the endogenous gene (see, for example, U.S. Patent Nos.: 5,641.670; 5,773,746; 5,733,761; 5,968,502; 6,702,989 and 6,565,844).

In other methods, the level of expression of a gene is detected by measuring the level of protein encoded by the gene. This can be done, e.g., by immunoprecipitation, ELISA, or immunohistochemistry using an agent, e.g., an antibody, that specifically detects the protein encoded by the gene. Other techniques include Western blot analysis. Immunoassays are commonly used to quantitate the levels of proteins in cell samples, and many other immunoassay techniques are known in the art. The disclosure is not limited to a particular assay procedure, and therefore is intended to include both homogeneous and heterogeneous procedures. Exemplary immunoassays which can be conducted include fluorescence polarization immunoassay (FPIA), fluorescence immunoassay (FIA), enzyme immunoassay (EIA), nephelometric inhibition immunoassay (NIA), enzyme linked immunosorbent assay (ELISA), and radioimmunoassay (RIA). An indicator moiety, or label group, can be attached to the subject antibodies and is selected so as to meet the needs of various uses of the method which are often dictated by the availability of assay equipment and compatible immunoassay procedures. General techniques to be used in performing the various immunoassays noted above are known to those of ordinary skill in the art.

In the case of polypeptides which are secreted from cells, the level of expression of these polypeptides can be measured in biological fluids.

In some embodiments, mRNA levels are detected and/or measured by microarray analysis as described in detail in the following sections.

### 6.3 Introduction: Microarray

Generally, determining expression profiles with arrays involves the following steps:
(a) obtaining a mRNA sample from a subject and preparing labeled nucleic acids therefrom (the "target nucleic acids" or "targets"); (b) contacting the target nucleic acids with the array under conditions sufficient for target nucleic acids to bind with corresponding probes on the array, e.g. by hybridization or specific binding; (c) optionally removing unbound targets from the array; (d) detecting bound targets, and (e) analyzing the results. As used herein, "nucleic acid probes" or "probes" are nucleic acids attached to the array, whereas "target nucleic acids" are nucleic acids that are hybridized to the array. Each of these steps is described in more detail below.

### 6.3.1 Labeling the nucleic acid for the microarray analysis

Generally, the target molecules will be labeled to permit detection of hybridization of target molecules to a microarray. By "labeled" is meant that the probe comprises a member of a signal producing system and is thus detectable, either directly or through combined action with one or more additional members of a signal producing system. Examples of directly detectable labels include isotopic and fluorescent moieties incorporated into, usually covalently bonded to, a moiety of the probe, such as a nucleotide monomeric unit, e.g. dNMP of the primer, or a photoactive or chemically active derivative of a detectable label which can be bound to a functional moiety of the probe molecule.

Nucleic acids can be labeled after or during enrichment and/or amplification of RNAs. For example, labeled cDNA can be prepared from mRNA by oligo dT-primed or random-primed reverse transcription, both of which are well known in the art (see, e.g., Klug and Berger, 1987, Methods Enzymol. 152:316-325). Reverse transcription may be carried out in the presence of a dNTP conjugated to a detectable label, most preferably a fluorescently labeled dNTP. Alternatively, isolated mRNA can be converted to labeled antisense RNA synthesized by in vitro transcription of double-stranded cDNA in the presence of labeled dNTPs (Lockhart et al., Nature Biotech. 14:1675, 1996). In alternative embodiments, the cDNA or RNA probe can be synthesized in the absence of detectable label and may be labeled subsequently, e.g., by incorporating biotinylated dNTPs or rNTP, or some similar means (e.g., photo-cross-linking a psoralen derivative of biotin to RNAs), followed by addition of labeled streptavidin (e.g., phycoerythrin-conjugated streptavidin) or the equivalent.

In one embodiment, labeled cDNA is synthesized by incubating a mixture containing RNA and 0.5 mM dGTP, dATP and dCTP plus 0.1 mM dTTP plus fluorescent deoxyribonucleotides (e.g., 0.1 mM Rhodamine 110 UTP (Perken Elmer Cetus) or 0.1 mM Cy3 dUTP (Amersham)) with reverse transcriptase (e.g., SuperScript^{™}II, LTI Inc.) at 42°C for 60 min.

Fluorescent moieties or labels of interest include coumarin and its derivatives, e.g. 7-amino-4-methylcoumarin, aminocoumarin, bodipy dyes, such as Bodipy FL, cascade blue, fluorescein and its derivatives, e.g. fluorescein isothiocyanate, Oregon green, rhodamine dyes, e.g. Texas red, tetramethylrhodamine, eosins and erythrosins, cyanine dyes, e.g. Cy2, Cy3, Cy3.5, Cy5, Cy5.5, Cy7, FluorX, macrocyclic chelates of lanthanide ions, e.g. quantum dye^{™}, fluorescent energy transfer dyes, such as thiazole orange-ethidium heterodimer, TOTAB, dansyl, etc. Individual fluorescent compounds which have functionalities for linking to an element desirably detected in an apparatus or assay disclosed, or which can be modified to incorporate such functionalities include, e.g., dansyl chloride; fluoresceins such as 3,6-dihydroxy-9-phenylxanthydrol; rhodamineisothiocyanate; N-phenyl 1-amino-8-sulfonatonaphthalene; N-phenyl 2-amino-6-sulfonatonaphthalene; 4-acetamido-4-isothiocyanato-stilbene-2,2'-disulfonic acid; pyrene-3-sulfonic acid; 2-toluidinonaphthalene-6-sulfonate; N-phenyl-N-methyl-2-aminoaphthalene-6-sulfonate; ethidium bromide; stebrine; auromine-0,2-(9'-anthroyl)palmitate; dansyl phosphatidylethanolamine; N,N'-dioctadecyl oxacarbocyanine: N,N'-dihexyl oxacarbocyanine; merocyanine, 4-(3'-pyrenyl)stearate; d-3-aminodesoxy-equilenin; 12-(9'-anthroyl)stearate; 2-methylanthracene; 9-vinylanthracene; 2,2'(vinylene-p-phenylene)bisbenzoxazole; p-bis(2- -methyl-5-phenyl-oxazolyl))benzene; 6-dimethylamino-1,2-benzophenazin; retinol; bis(3'-aminopyridinium) 1,10-decandiyl diiodide; sulfonaphthylhydrazone of hellibrienin; chlorotetracycline; N-(7-dimethylamino-4-methyl-2-oxo-3-chromenyl)maleimide; N-(p-(2benzimidazolyl)-phenyl)maleimide; N-(4-fluoranthyl)maleimide; bis(homovanillic acid); resazarin; 4-chloro-7-nitro-2,1,3-benzooxadiazole; merocyanine 540; resorufin; rose bengal; and 2,4-diphenyl-3(2H)-furanone. (see, e.g., Kricka, 1992, Nonisotopic DNA Probe Techniques, Academic Press San Diego, Calif.). Many fluorescent tags are commercially available from SIGMA-Aldrich, Amersham Biosciences, Molecular Probes, Pfizer (formerly Pharmacia), BD Biosciences (formerly CLONTECH), ChemGenes Corp., Glen Research Corp., Invitrogen, Fluka Chemica-Biochemika Analytika (Fluka Chemie AG, Buchs, Switzerland), and Applied Biosystems (Foster City, Calif.) as well as other commercial sources known to one of skill.

Chemiluminescent labels include luciferin and 2,3-dihydrophthalazinediones, e.g., luminol.

Isotopic moieties or labels of interest include ³²P, ³³P, ³⁵S, ¹²⁵I, ²H, ¹⁴C, and the like (see Zhao et al., Gene 156:207, 1995; Pietu et al., Genome Res. 6:492, 1996).

Labels may also be members of a signal producing system that act in concert with one or more additional members of the same system to provide a detectable signal. Illustrative of such labels are members of a specific binding pair, such as ligands, e.g. biotin, fluorescein, digoxigenin, antigen, polyvalent cations, chelator groups and the like, where the members specifically bind to additional members of the signal producing system, where the additional members provide a detectable signal either directly or indirectly, e.g. antibody conjugated to a fluorescent moiety or an enzymatic moiety capable of converting a substrate to a chromogenic product, e.g. alkaline phosphatase conjugate antibody and the like.

Additional labels of interest include those that provide for signal only when the probe with which they are associated is specifically bound to a target molecule, where such labels include: "molecular beacons" as described in Tyagi & Kramer, Nature Biotechnology 14:303, 1996 and EP 0 070 685 B1. Other labels of interest include those described in U.S. Pat. No. 5,563,037; WO 97/17471 and WO 97/17076.

In some cases, hybridized target nucleic acids may be labeled following hybridization. For example, where biotin labeled dNTPs are used in, e.g., amplification or transcription, streptavidin linked reporter groups may be used to label hybridized complexes.

In other embodiments, the target nucleic acid is not labeled. In this case, hybridization can be determined, e.g., by plasmon resonance, as described, e.g., in Thiel et al., Anal. Chem. 69:4948, 1997.

In one embodiment, a plurality (e.g., 2, 3, 4, 5 or more) of sets of target nucleic acids are labeled and used in one hybridization reaction ("multiplex" analysis). For example, one set of nucleic acids may correspond to RNA from one cell or tissue sample and another set of nucleic acids may correspond to RNA from another cell or tissue sample. The plurality of sets of nucleic acids can be labeled with different labels, e.g., different fluorescent labels which have distinct emission spectra so that they can be distinguished. The sets can then be mixed and hybridized simultaneously to one microarray.

The use of a two-color fluorescence labeling and detection scheme to define alterations in gene expression has been described, e.g., in Shena et al., Science 270:467-470, 1995. An advantage of using cDNA labeled with two different fluorophores is that a direct and internally controlled comparison of the mRNA levels corresponding to each arrayed gene in two cell states can be made, and variations due to minor differences in experimental conditions (e.g, hybridization conditions) will not affect subsequent analyses.

Examples of distinguishable labels for use when hybridizing a plurality of target nucleic acids to one array are well known in the art and include: two or more different emission wavelength fluorescent dyes, like Cy3 and Cy5, combination of fluorescent proteins and dyes, like phicoerythrin and Cy5, two or more isotopes with different energy of emission, like ³²P and ³³P, gold or silver particles with different scattering spectra, labels which generate signals under different treatment conditions, like temperature, pH, treatment by additional chemical agents, etc., or generate signals at different time points after treatment. Using one or more enzymes for signal generation allows for the use of an even greater variety of distinguishable labels, based on different substrate specificity of enzymes (alkaline phosphatase/peroxidase).

Further, it is preferable in order to reduce experimental error to reverse the fluorescent labels in two-color differential hybridization experiments to reduce biases peculiar to individual genes or array spot locations. In other words, it is preferable to first measure gene expression with one labeling (e.g., labeling nucleic acid froma first cell with a first fluorochrome and nucleic acid from a second cell with a second fluorochrome) of the mRNA from the two cells being measured, and then to measure gene expression from the two cells with reversed labeling (e.g., labeling nucleic acid from the first cell with the second fluorochrome and nucleic acid from the second cell with the first fluorochrome). Multiple measurements over exposure levels and perturbation control parameter levels provide additional experimental error control.

The quality of labeled nucleic acids can be evaluated prior to hybridization to an array. For example, a sample of the labeled nucleic acids can be hybridized to probes derived from the 5', middle and 3' portions of genes known to be or suspected to be present in the nucleic acid sample. This will be indicative as to whether the labeled nucleic acids are full length nucleic acids or whether they are degraded. In one embodiment, the GeneChip® Test3 Array from Affymetrix (Santa Clara, CA) can be used for that purpose. This array contains probes representing a subset of characterized genes from several organisms including mammals. Thus, the quality of a labeled nucleic acid sample can be determined by hybridization of a fraction of the sample to an array, such as the GeneChip® Test3 Array from Affymetrix (Santa Clara, CA).

### 6.3.2 Microarray analysis

Preferred arrays, e.g., microarrays, for use according to the disclosure include one or more probes of genes which are candidate genes for their involvement in cognitive function. Exemplary arrays include one or more genes of interest to studying cognitive function such as those genes found on the GeneChip® Rat Expression Set 230 or GeneChip® Rat Neurobiology U34 Array, which contains over 1,200 sequences relevant to the study of neurobiology (including genes for kinases, cell surface). Additionally, one could use the GeneChip® HuSNP™ Array to survey the entire human genome by simultaneously tracking nearly 1,500 genetic variations, known as single nucleotide polymorphisms (SNPs), dispersed throughout the genome. SNPs are excellent markers for genomic searches because they are simple, abundant, widespread, and account for most of the genetic variability across human populations. Using high-throughput techniques, such as GeneChip® arrays, SNPs can be more easily tracked than traditional markers, such as microsatellite sequences.

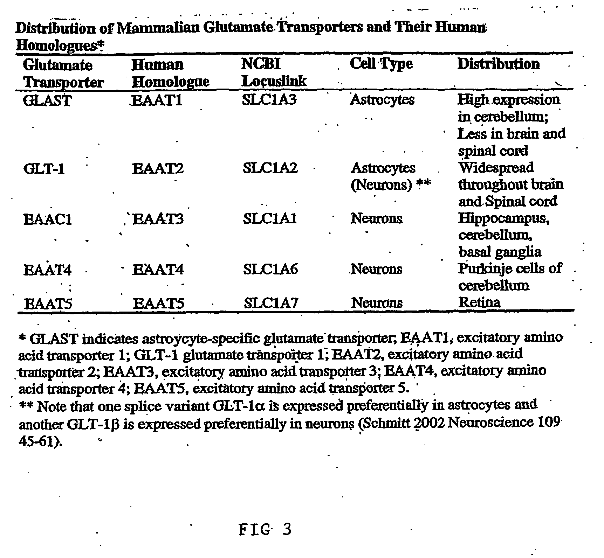

The array may comprise probes corresponding to at least 10, preferably at least 20, at least 50, at least 100 or at least 1000 genes. The array may comprise probes corresponding to about 10%, 20%, 50%, 70%, 90% or 95% of the genes listed in Figure 3 or other genes available on a microarray. The array may comprise probes corresponding to about 10%, 20%, 50%, 70%, 90% or 95% of the genes listed in Figure 3 or other gene whose expression is at least 2 fold, preferably at least 3 fold, more preferably at least 4 fold, 5 fold, 7 fold and most preferably at least about 10 fold higher in cells. One exemplary preferred array that can be used is the array used and described in the Examples.

There can be one or more than one probe corresponding to each gene on a microarray. For example, a microarray may contain from 2 to 20 probes corresponding to one gene and preferably about 5 to 10. The probes may correspond to the full length RNA sequence or complement thereof of genes characteristic of candidate disease genes., or they may correspond to a portion thereof, which portion is of sufficient length for permitting specific hybridization. Such probes may comprise from about 50 nucleotides to about 100, 200, 500, or 1000 nucleotides or more than 1000 nucleotides. As further described herein, microarrays may contain oligonucleotide probes, consisting of about 10 to 50 nucleotides, preferably about 15 to 30 nucleotides and even more preferably 20-25 nucleotides. The probes are preferably single stranded. The probe will have sufficient complementarity to its target to provide for the desired level of sequence specific hybridization (see below).

Typically, the arrays used in the present disclosure will have a site density of greater than 100 different probes per cm2. Preferably, the arrays will have a site density of greater than 500/cm2, more preferably greater than about 1000/cm2, and most preferably, greater than about 10,000/cm2. Preferably, the arrays will have more than 100 different probes on a single substrate, more preferably greater than about 1000 different probes still more preferably, greater than about 10,000 different probes and most preferably, greater than 100,000 different probes on a single substrate.

Microarrays can be prepared by methods known in the art, as described below, or they can be custom made by companies, e.g., Affymetrix (Santa Clara, CA).

Generally, two types of microarrays can be used. These two types are referred to as "synthesis" and "delivery." In the synthesis type, a microarray is prepared in a step-wise fashion by the in situ synthesis of nucleic acids from nucleotides. With each round of synthesis, nucleotides are added to growing chains until the desired length is achieved. In the delivery type of microarray, preprepared nucleic acids are deposited onto known locations using a variety of delivery technologies. Numerous articles describe the different microarray technologies, e.g., Shena et al., Tibtech 16: 301, 1998; Duggan et al., Nat. Genet. 21:10, 1999; Bowtell et al., Nat. Genet. 21: 25, 1999.

One novel synthesis technology is that developed by Affymetrix (Santa Clara, CA), which combines photolithography technology with DNA synthetic chemistry to enable high density oligonucleotide microarray manufacture. Such chips contain up to 400,000 groups of oligonucleotides in an area of about 1.6 cm2. Oligonucleotides are anchored at the 3' end thereby maximizing the availability of single-stranded nucleic acid for hybridization. Generally such chips, referred to as "GeneChips®" contain several oligonucleotides of a particular gene, e.g., between 15-20, such as 16 oligonucleotides. Since Affymetrix (Santa Clara, CA) sells custom made microarrays, microarrays containing genes which are up- or down-regulated with cognitive impairments can be ordered for purchase from Affymetrix (Santa Clara, CA).

Microarrays can also be prepared by mechanical microspotting, e.g., those commercialized at Synteni (Fremont, CA). According to these methods, small quantities of nucleic acids are printed onto solid surfaces. Microspotted arrays prepared at Synteni contain as many as 10,000 groups of cDNA in an area of about 3.6 cm².

A third group of microarray technologies consist in the "drop-on-demand" delivery approaches, the most advanced of which are the ink-jetting technologies, which utilize piezoelectric and other forms of propulsion to transfer nucleic acids from miniature nozzles to solid surfaces. Inkjet technologies is developed at several centers including Incyte Pharmaceuticals (Palo Alto, CA) and Protogene (Palo Alto, CA). This technology results in a density of 10,000 spots per cm². See also, Hughes et al., Nat. Biotechn. 19:342,2001.

Arrays preferably include control and reference nucleic acids. Control nucleic acids are nucleic acids which serve to indicate that the hybridization was effective. For example, all Affymetrix (Santa Clara, CA) expression arrays contain sets of probes for several prokaryotic genes, e.g., bioB, bioC and bioD from biotin synthesis of E. coli and cre from P1 bacteriophage. Hybridization to these arrays is conducted in the presence of a mixture of these genes or portions thereof, such as the mix provided by Affymetrix (Santa Clara, CA) to that effect (Part Number 900299), to thereby confirm that the hybridization was effective. Control nucleic acids included with the target nucleic acids can also be mRNA synthesized from cDNA clones by in vitro transcription. Other control genes that may be included in arrays are polyA controls, such as dap, lys, phe, thr, and trp (which are included on Affymetrix GeneChips®)

Reference nucleic acids allow the normalization of results from one experiment to another, and to compare multiple experiments on a quantitative level. Exemplary reference nucleic acids include housekeeping genes of known expression levels, e.g., glyceraldehyde-3-phosphate dehydrogenase (GAPDH), hexokinase and actin.

Mismatch controls may also be provided for the probes to the target genes, for expression level controls or for normalization controls. Mismatch controls are oligonucleotide probes or other nucleic acid probes identical to their corresponding test or control probes except for the presence of one or more mismatched bases.

Arrays may also contain probes that hybridize to more than one allele of a gene. For example the array can contain one probe that recognizes allele 1 and another probe that recognizes allele 2 of a particular gene.

Microarrays can be prepared as follows. In one embodiment, an array of oligonucleotides is synthesized on a solid support. Exemplary solid supports include glass, plastics, polymers, metals, metalloids, ceramics, organics, etc. Using chip masking technologies and photoprotective chemistry it is possible to generate ordered arrays of nucleic acid probes. These arrays, which are known, e.g., as "DNA chips," or as very large scale immobilized polymer arrays ("VLSIPSTM" arrays) can include millions of defined probe regions on a substrate having an area of about 1 cm² to several cm², thereby incorporating sets of from a few to millions of probes (see, e.g., U.S. Patent No. 5,631,734).

The construction of solid phase nucleic acid arrays to detect target nucleic acids is well described in the literature. See, Fodor et al., Science, 251: 767-777, 1991; Sheldon et al., Clinical Chemistry 39(4): 718-719, 1993; Kozal et al., Nature Medicine 2(7): 753-759, 1996 and Hubbell U.S. Pat. No. 5,571,639; Pinkel et al. PCT/US95/16155 (WO 96/17958); U.S. Pat. Nos. 5,677,195; 5,624,711; 5,599,695; 5,451,683; 5,424,186; 5,412,087; 5,384,261; 5,252,743 and 5,143,854; PCT Patent Publication Nos. 92/10092 and 93/09668; and PCT WO 97/10365. In brief, a combinatorial strategy allows for the synthesis of arrays containing a large number of probes using a minimal number of synthetic steps. For instance, it is possible to synthesize and attach all possible DNA 8 mer oligonucleotides (48, or 65,536 possible combinations) using only 32 chemical synthetic steps. In general, VLSIPSTM procedures provide a method of producing 4n different oligonucleotide probes on an array using only 4n synthetic steps (see, e.g., U.S. Pat. No. 5,631,734 5; 143,854 and PCT Patent Publication Nos. WO 90/15070; WO 95/11995 and WO 92/10092).

Light-directed combinatorial synthesis of oligonucleotide arrays on a glass surface can be performed with automated phosphoramidite chemistry and chip masking techniques similar to photoresist technologies in the computer chip industry. Typically, a glass surface is derivatized with a silane reagent containing a functional group, e.g., a hydroxyl or amine group blocked by a photolabile protecting group. Photolysis through a photolithogaphic mask is used selectively to expose functional groups which are then ready to react with incoming 5'-photoprotected nucleoside phosphoramidites. The phosphoramidites react only with those sites which are illuminated (and thus exposed by removal of the photolabile blocking group). Thus, the phosphoramidites only add to those areas selectively exposed from the preceding step. These steps are repeated until the desired array of sequences have been synthesized on the solid surface.

Algorithms for design of masks to reduce the number of synthesis cycles are described by Hubbel et al., U.S. Pat. No. 5,571,639 and U.S. Pat. No. 5,593,839. A computer system may be used to select nucleic acid probes on the substrate and design the layout of the array as described in U.S. Pat. No. 5,571,639.

Another method for synthesizing high density arrays is described in U.S. Patent No. 6,083,697. This method utilizes a novel chemical amplification process using a catalyst system which is initiated by radiation to assist in the synthesis the polymer sequences. Such methods include the use of photosensitive compounds which act as catalysts to chemically alter the synthesis intermediates in a manner to promote formation of polymer sequences. Such photosensitive compounds include what are generally referred to as radiation-activated catalysts (RACs), and more specifically photo activated catalysts (PACs). The RACs can by themselves chemically alter the synthesis intermediate or they can activate an autocatalytic compound which chemically alters the synthesis intermediate in a manner to allow the synthesis intermediate to chemically combine with a later added synthesis intermediate or other compound.

Arrays can also be synthesized in a combinatorial fashion by delivering monomers to cells of a support by mechanically constrained flowpaths. See Winkler et al., EP 624,059. Arrays can also be synthesized by spotting monomers reagents on to a support using an ink jet printer. See id. and Pease et al., EP 728,520.

cDNA probes can be prepared according to methods known in the art and further described herein, e.g., reverse-transcription PCR (RT-PCR) of RNA using sequence specific primers. Oligonucleotide probes can be synthesized chemically. Sequences of the genes or cDNA from which probes are made can be obtained, e.g., from GenBank, other public databases or publications.

Nucleic acid probes can be natural nucleic acids, chemically modified nucleic acids, e.g., composed of nucleotide analogs, as long as they have activated hydroxyl groups compatible with the linking chemistry. The protective groups can, themselves, be photolabile. Alternatively, the protective groups can be labile under certain chemical conditions, e.g., acid. In this example, the surface of the solid support can contain a composition that generates acids upon exposure to light. Thus, exposure of a region of the substrate to light generates acids in that region that remove the protective groups in the exposed region. Also, the synthesis method can use 3'- protected 5'-0-phosphoramidite-activated deoxynucleoside. In this case, the oligonucleotide is synthesized in the 5' to 3' direction, which results in a free 5' end.

Oligonucleotides of an array can be synthesized using a 96 well automated multiplex oligonucleotide synthesizer (A.M.O.S.) that is capable of making thousands of oligonucleotides (Lashkari et al., PNAS 93: 7912, 1995).

It will be appreciated that oligonucleotide design is influenced by the intended application. For example, it may be desirable to have similar melting temperatures for all of the probes. Accordingly, the length of the probes are adjusted so that the melting temperatures for all of the probes on the array are closely similar (it will be appreciated that different lengths for different probes may be needed to achieve a particular T[m] where different probes have different GC contents). Although melting temperature is a primary consideration in probe design, other factors are optionally used to further adjust probe construction, such as selecting against primer self-complementarity and the like.

Arrays, e.g., microarrrays, may conveniently be stored following fabrication or purchase for use at a later time. Under appropriate conditions, the subject arrays are capable of being stored for at least about 6 months and may be stored for up to one year or longer. Arrays are generally stored at temperatures between about -20°C to room temperature, where the arrays are preferably sealed in a plastic container, e.g. bag, and shielded from light.

### 6.3.3 Hybridizing the target nucleic acid to the microarray

The next step is to contact the target nucleic acids with the array under conditions sufficient for binding between the target nucleic acids and the probes of the array. In a preferred embodiment, the target nucleic acids will be contacted with the array under conditions sufficient for hybridization to occur between the target nucleic acids and probes on the microarray, where the hybridization conditions will be selected in order to provide for the desired level of hybridization specificity.

Contact of the array and target nucleic acids involves contacting the array with an aqueous medium comprising the target nucleic acids. Contact may be achieved in a variety of different ways depending on specific configuration of the array. For example, where the array simply comprises the pattern of size separated probes on the surface of a "plate-like" rigid substrate, contact may be accomplished by simply placing the array in a container comprising the target nucleic acid solution, such as a polyethylene bag, and the like. In other embodiments where the array is entrapped in a separation media bounded by two rigid plates, the opportunity exists to deliver the target nucleic acids via electrophoretic means. Alternatively, where the array is incorporated into a biochip device having fluid entry and exit ports, the target nucleic acid solution can be introduced into the chamber in which the pattern of target molecules is presented through the entry port, where fluid introduction could be performed manually or with an automated device. In multiwell embodiments, the target nucleic acid solution will be introduced in the reaction chamber comprising the array, either manually, e.g. with a pipette, or with an automated fluid handling device.

Contact of the target nucleic acid solution and the probes will be maintained for a sufficient period of time for binding between the target and the probe to occur. Although dependent on the nature of the probe and target, contact will generally be maintained for a period of time ranging from about 10 min to 24 hrs, usually from about 30 min to 12 hrs and more usually from about L hr to 6 hrs.

When using commercially available microarrays, adequate hybridization conditions are provided by the manufacturer. When using non-commercial microarrays, adequate hybridization conditions can be determined based on the following hybridization guidelines, as well as on the hybridization conditions described in the numerous published articles on the use of microarrays.

Nucleic acid hybridization and wash conditions are optimally chosen so that the probe "specifically binds" or "specifically hybridizes" to a specific array site, i.e., the probe hybridizes, duplexes or binds to a sequence array site with a complementary nucleic acid sequence but does not hybridize to a site with a non-complementary nucleic acid sequence. As used herein, one polynucleotide sequence is considered complementary to another when, if the shorter of the polynucleotides is less than or equal to 25 bases, there are no mismatches using standard base-pairing rules or, if the shorter of the polynucleotides is longer than 25 bases, there is no more than a 5% mismatch. Preferably, the polynucleotides are perfectly complementary (no mismatches). It can easily be demonstrated that specific hybridization conditions result in specific hybridization by carrying out a hybridization assay including negative controls.

Hybridization is carried out in conditions permitting essentially specific hybridization.

The length of the probe and GC content will determine the Tm of the hybrid, and thus the hybridization conditions necessary for obtaining specific hybridization of the probe to the template nucleic acid. These factors are well known to a person of skill in the art, and can also be tested in assays. An extensive guide to the hybridization of nucleic acids is found in Tijssen (1993), "Laboratory Techniques in biochemistry and molecular biology-hybridization with nucleic acid probes." Generally, stringent conditions are selected to be about 5°C lower than the thermal melting point (Tm) for the specific sequence at a defined ionic strength and pH. The Tm is the temperature (under defined ionic strength and pH) at which 50% of the target sequence hybridizes to a perfectly matched probe. Highly stringent conditions are selected to be equal to the Tm point for a particular probe. Sometimes the term "Td" is used to define the temperature at which at least half of the probe dissociates from a perfectly matched target nucleic acid. In any case, a variety of estimation techniques for estimating the Tm or Td are available, and generally described in Tijssen, supra. Typically, G-C base pairs in a duplex are estimated to contribute about 3°C to the Tm, while A-T base pairs are estimated to contribute about 2°C, up to a theoretical maximum of about 80-100°C. However, more sophisticated models of Tm and Td are available and appropriate in which G-C stacking interactions, solvent effects, the desired assay temperature and the like are taken into account. For example, probes can be designed to have a dissociation temperature (Td) of approximately 60°C, using the formula: Td = (((((3 x #GC) + (2 x #AT)) x 37) - 562)/#bp) - 5; where #GC, #AT, and #bp are the number of guanine-cytosine base pairs, the number of adenine-thymine base pairs, and the number of total base pairs, respectively, involved in the annealing of the probe to the template DNA.

The stability difference between a perfectly matched duplex and a mismatched duplex, particularly if the mismatch is only a single base, can be quite small, corresponding to a difference in Tm between the two of as little as 0.5 degrees (See Tibanyenda, N. et al., Eur. J. Biochem. 139:19, 1984 and Ebel, S. et al., Biochem. 31:12083, 1992). More importantly, it is understood that as the length of the homology region increases, the effect of a single base mismatch on overall duplex stability decreases.

Theory and practice of nucleic acid hybridization is described, e.g., in S. Agrawal (ed.) Methods in Molecular Biology, volume 20; and Tijssen (1993) "Laboratory Techniques in biochemistry and molecular biology-hybridization with nucleic acid probes", e.g., part I chapter 2 "Overview of principles of hybridization and the strategy of nucleic acid probe assays", Elsevier, New York provide a basic guide to nucleic acid hybridization.

Certain microarrays are of "active" nature; i.e., they provide independent electronic control over all aspects of the hybridization reaction (or any other affinity reaction) occurring at each specific microlocation. These devices provide a new mechanism for affecting hybridization reactions which is called electronic stringency control (ESC). Such active devices can electronically produce "different stringency conditions" at each microlocation. Thus, all hybridizations can be carried out optimally in the same bulk solution. These arrays are described in Sosnowski et al., U.S. Patent No. 6,051,380.

In a preferred embodiment, background signal is reduced by the use of a detergent (e.g, C-TAB) or a blocking reagent (e.g., sperm DNA, cot-1 DNA, etc.) during the hybridization to reduce non-specific binding. In a particularly preferred (embodiment, the hybridization is performed in the presence of about 0.5 mg/ml DNA (e.g., herring sperm DNA). The use of blocking agents in hybridization is well known to those of skill in the art (see, e.g., Chapter 8 in Laboratory Techniques in Biochemistry and Molecular Biology, Vol. 24: Hybridization With Nucleic Acid Probes, P. Tijssen, ed. Elsevier, N.Y., (1993)).

The method may or may not further comprise a non-bound label removal step prior to the detection step, depending on the particular label employed on the target nucleic acid. For example, in certain assay formats (e.g., "homogenous assay formats") a detectable signal is only generated upon specific binding of target to probe. As such, in these assay formats, the hybridization pattern may be detected without a non-bound label removal step. In other embodiments, the label employed will generate a signal whether or not the target is specifically bound to its probe. In such embodiments, the non-bound labeled target is removed from the support surface. One means of removing the non-bound labeled target is to perform the well known technique of washing, where a variety of wash solutions and protocols for their use in removing non-bound label are known to those of skill in the art and may be used. Alternatively, non-bound labeled target can be removed by electrophoretic means.

Where all of the target sequences are detected using the same label, different arrays will be employed for each physiological source (where different could include using the same array at different times). The above methods can be varied to provide for multiplex analysis, by employing different and distinguishable labels for the different target populations (representing each of the different physiological sources being assayed). According to this multiplex method, the same array is used at the same time for each of the different target populations.

In another embodiment, hybridization is monitored in real time using a charge-coupled device (CCD) imaging camera (Guschin et al., Anal. Biochem. 250:203, 1997). Synthesis of arrays on optical fibre bundles allows easy and sensitive reading (Healy et al., Anal. Biochem. 251:270, 1997). In another embodiment, real time hybridization detection is carried out on microarrays without washing using evanescent wave effect that excites only fluorophores that are bound to the surface (see, e.g., Stimpson et al., PNAS 92:6379, 1995).

### 6.3.4 Detecting hybridized nucleic acids and analyzing the results from the microarray

The above steps result in the production of hybridization patterns of target nucleic acid on the array surface. These patterns may be visualized or detected in a variety of ways, with the particular manner of detection being chosen based on the particular label of the target nucleic acid. Representative detection means include scintillation counting, autoradiography, fluorescence measurement, colorimetric measurement, light emission measurement, light scattering, and the like.

One method of detection includes an array scanner that is commercially available from Affymetrix (Santa Clara, CA), e.g., the 417TM Arrayer, the 418TM Array Scanner, or the Agilent GeneArrayTM Scanner. This scanner is controlled from the system computer with a WindowsR interface and easy-to-use software tools. The output is a 16-bit.tif file that can be directly imported into or directly read by a variety of software applications. Preferred scanning devices are described in, e.g., U.S. Pat. Nos. 5,143,854 and 5,424,186.

When fluorescently labeled probes are used, the fluorescence emissions at each site of a transcript array can be detected by scanning confocal laser microscopy. In one embodiment, a separate scan, using the appropriate excitation line, is carried out for each of the two fluorophores used. Alternatively, a laser can be used that allows simultaneous specimen illumination at wavelengths specific to the two fluorophores and emissions from the two fluorophores can be analyzed simultaneously (see Shalon et al., Genome Research 6:639-645, 1996). In a preferred embodiment, the arrays are scanned with a laser fluorescent scanner with a computer controlled X-Y stage and a microscope objective. Sequential excitation of the two fluorophores can be achieved with a multi-line, mixed gas laser and the emitted light is split by wavelength and detected with two photomultiplier tubes. In one embodiment in which fluorescent target nucleic acids are used, the arrays may be scanned using lasers to excite fluorescently labeled targets that have hybridized to regions of probe arrays, which can then be imaged using charged coupled devices ("CCDs") for a wide field scanning of the array. Fluorescence laser scanning devices are described, e.g., in Schena et al., supra. Alternatively, the fiber-optic bundle described by Ferguson et al., Nature Biotech. 14:1681-1684, 1996, may be used to monitor mRNA abundance levels.

Following the data gathering operation, the data will typically be reported to a data analysis operation. To facilitate the sample analysis operation, the data obtained by the reader from the device will typically be analyzed using a digital computer. Typically, the computer will be appropriately programmed for receipt and storage of the data from the device, as well as for analysis and reporting of the data gathered, e.g., subtraction of the background, deconvolution of multi-color images, flagging or removing artifacts, verifying that controls have performed properly, normalizing the signals, interpreting fluorescence data to determine the amount of hybridized target, normalization of background and single base mismatch hybridizations, and the like. In a preferred embodiment, a system comprises a search function that allows one to search for specific patterns, e.g., patterns relating to differential gene expression, e.g., between the expression profile of a sample from a patient with cognitive impairments and the expression profile of a counterpart normal subject. A system preferably allows one to search for patterns of gene expression between more than two samples.

A desirable system for analyzing data is a general and flexible system for the visualization, manipulation, and analysis of gene expression data. Such a system preferably includes a graphical user interface for browsing and navigating through the expression data, allowing a user to selectively view and highlight the genes of interest. The system also preferably includes sort and search functions and is preferably available for general users with PC, Mac or Unix workstations. Also preferably included in the system are clustering algorithms that are qualitatively more efficient than existing ones. The accuracy of such algorithms is preferably hierarchically adjustable so that the level of detail of clustering can be systematically refined as desired.

Various algorithms are available for analyzing the gene expression profile data, e.g., the type of comparisons to perform. In certain embodiments, it is desirable to group genes that are co-regulated. This allows the comparison of large numbers of profiles. A preferred embodiment for identifying such groups of genes involves clustering algorithms (for reviews of clustering algorithms, see, e.g., Fukunaga, 1990, Statistical Pattern Recognition, 2nd Ed., Academic Press, San Diego; Everitt, 1974, Cluster Analysis, London: Heinemann Educ. Books; Hartigan, 1975, Clustering Algorithms, New York: Wiley; Sneath and Sokal, 1973, Numerical Taxonomy, Freeman; Anderberg, 1973, Cluster Analysis for Applications, Academic Press: New York).

Clustering analysis is useful in helping to reduce complex patterns of thousands of time curves into a smaller set of representative clusters. Some systems allow the clustering and viewing of genes based on sequences. Other systems allow clustering based on other characteristics of the genes, e.g., their level of expression (see, e.g., U.S. Patent No. 6,203,987). Other systems permit clustering of time curves (see, e.g. U.S. Patent No. 6,263,287). Cluster analysis can be performed using the hclust routine (see, e.g., "hclust"routine from the software package S-Plus, MathSoft, Inc., Cambridge, Mass.).

In some specific embodiments, genes are grouped according to the degree of co-variation of their transcription, presumably co-regulation, as described in U.S. Patent No. 6,203,987. Groups of genes that have co-varying transcripts are termed "genesets." Cluster analysis or other statistical classification methods can be used to analyze the co-variation of transcription of genes in response to a variety of perturbations, e.g. caused by a disease or a drug. In one specific embodiment, clustering algorithms are applied to expression profiles to construct a "similarity tree" or "clustering tree" which relates genes by the amount of co-regulation exhibited. Genesets are defined on the branches of a clustering tree by cutting across the clustering tree at different levels in the branching hierarchy.

In some embodiments, a gene expression profile is converted to a projected gene expression profile. The projected gene expression profile is a collection of geneset expression values. The conversion is achieved, in some embodiments, by averaging the level of expression of the genes within each geneset. In some other embodiments, other linear projection processes may be used. The projection operation expresses the profile on a smaller and biologically more meaningful set of coordinates, reducing the effects of measurement errors by averaging them over each cellular constituent sets and aiding biological interpretation of the profile.

Values that can be compared include gross expression levels; averages of expression levels, e.g., from different experiments, different samples from the same subject or samples from different subjects; and ratios of expression levels.

### 6.3. 5 Data analysis methods for the microarray

Comparison of the expression levels of one or more genes which are up-regulated in response to the inhibition of cognitive impairment with reference to expression levels in the absence of inhibition of cognitive impairment, e.g., expression levels characteristic of a disease or in normal subject, is preferably conducted using computer systems. In one embodiment, one or more expression levels are obtained from two samples and these two sets of expression levels are introduced into a computer system for comparison. In a preferred embodiment, one set of one or more expression levels is entered into a computer system for comparison with values that are already present in the computer system, or in computer-readable form that is then entered into the computer system.

There is provided a computer readable form of the gene expression profile data of the invention, or of values corresponding to the level of expression of at least one gene which is up-regulated in response to inhibition of cognitive impairment in a subject. The values can be mRNA expression levels obtained from experiments, e.g., microarray analysis. The values can also be mRNA levels normalized relative to a reference gene whose expression is constant in numerous cells under numerous conditions, e.g., GAPDH. In other embodiments, the values in the computer are ratios of, or differences between, normalized or non-normalized mRNA levels in different samples.

The computer readable medium may comprise values of at least 2, at least 3, at least 5, 10, 20, 50, 100, 200, 500 or more genes. In a preferred embodiment, the computer readable medium comprises at least one expression profile.

Gene expression data can be in the form of a table, such as an Excel table. The data can be alone, or it can be part of a larger database, e.g., comprising other expression profiles, e.g., publicly available database. The computer readable form can be in a computer. In another embodiment, the invention provides a computer displaying the gene expression profile data.

Methods are provided methods in which the level of expression of a single gene can be compared in two or more cells or tissue samples. In some embodiments, the level of expression of a plurality of genes is compared. For example, the level of expression of at least 2, at least 3, at least 5, 10, 20, 50, 100, 200, 500 or more genes. In an embodiment, expression profiles are compared.

A method is provided for determining the similarity between the level of expression of one or more genes which are up-regulated in response to inhibition of cognitive impairment. The method preferably comprises obtaining the level of expression of one or more genes which are up-regulated in response to inhibition of cognitive impairment in a first sample and entering these values into a computer comprising (i) a database including records comprising values corresponding to levels of expression of one or more genes in a control untreated sample, and (ii) processor instructions, e.g., a user interface, capable of receiving a selection of one or more values for comparison purposes with data that is stored in the computer. The computer may further comprise a means for converting the comparison data into a diagram or chart or other type of output.

A system is provided that comprises a means for receiving gene expression data for one or a plurality of genes; a means for comparing the gene expression data from each of said one or plurality of genes to a common reference frame; and a means for presenting the results of the comparison. This system may further comprise a means for clustering the data.

A computer program is provided for analyzing gene expression data comprising (i) a computer code that receives as input gene expression data for a plurality of genes and (ii) a computer code that compares said gene expression data from each of said plurality of genes to a common reference frame.

A machine-readable or computer-readable medium is provided including program instructions for performing the following steps: (i) comparing a plurality of values corresponding to expression levels of one or more genes which are up--regulated in response to inhibition of NMD in a query cell with a database including records comprising reference expression of one or more reference cells and an annotation of the type of cell; and (ii) indicating to which cell the query cell is most similar based on similarities of expression levels.

The relative levels of expression, e.g., abundance of an mRNA, in two biological samples can be scored as a perturbation (relative abundance difference) or as not perturbed (i.e., the relative abundance is the same). For example, a perturbation can be a difference in expression levels between the two sources of RNA of at least a factor of about 25% (RNA from one source is 25% more abundant in one source than the other source), more usually about 50%, even more often by a factor of about 2 (twice as abundant), 3 (three times as abundant) or 5 (five times as abundant). Perturbations can be used by a computer for calculating and expressing comparisons.

Preferably, in addition to identifying a perturbation as positive or negative, it is advantageous to determine the magnitude of the perturbation. This can be carried out, as noted above, by calculating the ratio of the emission of the two fluorophores used for differential labeling, or by analogous methods that will be readily apparent to those of skill in the art.

The computer readable medium may further comprise a pointer to a descriptor of the level of expression or expression profile, e.g., from which source it was obtained, e.g., from which patient it was obtained. A descriptor can reflect the stage of disease, the therapy that the patient is undergoing or any other descriptions of the source of expression levels.

In operation, the means for receiving gene expression data, the means for comparing the gene expression data, the means for presenting, the means for normalizing, and the means for clustering within the context of the systems of the present invention can involve a programmed computer with the respective functionalities described herein, implemented in hardware or hardware and software; a logic circuit or other component of a programmed computer that performs the operations specifically identified herein, dictated by a computer program; or a computer memory encoded with executable instructions representing a computer program that can cause a computer to function in the particular fashion described herein.

Those skilled in the art will understand that the systems and methods of the present disclosure may be applied to a variety of systems, including IBM-compatible personal computers running MS-DOS or Microsoft Windows. Additionally the personal computer would have all of the hardware and software components normally associated with such a system such that the user would have capable memory, network connectivity, printing capability and programming capability with various computer languages. With the proper computer system the user could first load expression profile data into the computer system, U.S. Patent No. 6,203,987. Geneset profile definitions are loaded into the memory from the storage media or from a remote computer, preferably from a dynamic geneset database system, through the network. Next the user causes execution of projection software which performs the steps of converting expression profile to projected expression profiles. The projected expression profiles are then displayed.

In yet another exemplary implementation, a user first leads a projected profile into the memory. The user then causes the loading of a reference profile into the memory. Next, the user causes the execution of comparison software which performs the steps of objectively comparing the profiles.

### 7. Screening for compounds that promote or preserve cognitive function

Guided by the present disclosure, agents that modulate the expression of genes associated with cognitive function can be identified using in vitro and in vivo screening methods of the invention.

Methods are provided for identification of agents useful for promoting or preserving cognitive function in mammals, e.g., rats and humans. In one aspect, the screening methods involve conducting assays to identify agents that modulate the expression of a gene encoding a glutamate transporter protein, e.g., an EAAT1, EAAT2, EAAT3, EAAT4 or EAAT5, or the activity of a glutamate transporter protein encoded by such a gene. For simplicity, reference below to "EAAT" is intended to refer to each of EAAT1, EAAT2, EAAT3, EAAT4 and EAAT5 individually, to the group comprising all of the genes/proteins, and to all subcombinations (e.g., EAAT1 and EAAT2). Alternatively, the screening methods involve conducting assays to identify agents that modulate the expression of aspartate aminotransferase.

A number of different screening protocols can be utilized to identify agents that modulate the level of expression of EAAT and/or aspartate aminotransferase in mammalian cells (e.g., rat cells, non-human primate cells or human cells). In general terms, the screening methods involve screening a plurality of agents ("test agents") to identify an agent that changes the activity or level of EAAT by, for example without limitation, binding to an EAAT polypeptide, preventing an inhibitor from binding to an EAAT polypeptide, or increasing expression of an EAAT gene. Moreover, the screening methods involve screening a plurality of agents to identify an agent that changes the activity or level of aspartate aminotransferase by, for example without limitation, binding to an aspartate aminotransferase polypeptide, preventing an inhibitor from binding to an aspartate aminotransferasepolypeptide, or increasing expression of an aspartate aminotransferase gene.

"Test agents" include compounds of a variety of general types including, but not limited to, small organic molecules, known pharmaceuticals, polypeptides; carbohydrates such as oligosaccharides and polysaccharides; polynucleotides; lipids or phospholipids; fatty acids; steroids; or amino acid analogs. Test agents can be obtained from libraries, such as natural product libraries and combinatorial libraries. A number of different types of libraries are commercially available and methods for preparing libraries have been described, including for example, PCT publications WO 93/06121, WO 95/12608, WO 95/35503, WO 94/08051 and WO 95/30642. In addition, methods of automating assays are known that permit screening of several thousands of compounds in a short period.

Certain screening methods involve screening for a compound that increases the expression or activity of an EAAT and/or an aspartate aminotransferase protein in a cell. Such methods can involve conducting cell-based assays in which test compounds are contacted with one or more cells expressing an EAAT gene or protein and then detecting a change in EAAT expression (e.g., levels of EAAT RNA) or activity. Another method can involve conducting cell-based assays in which test compounds are contacted with one or more cells expressing an aspartate aminotransferase gene or protein and then detecting a change in aspartate aminotransferase expression (e.g., levels of aspartate aminotransferase RNA) or activity. Thus, in an embodiment the method comprises contacting a cell with a test agent and determining whether the level of expression of the gene is changed in the presence of the test agent, where a change (e.g., increase) in expression is an indication that the test agent is useful for promoting or preserving cognitive function. Cells can be contacted *in vitro, in vivo* or *ex vivo.* Typically expression is increased by at least about 10%, at least about 20%, at least about 50%, at least about 75%, or at least about 100% compared to expression in the absence of the test compound.

A method is provided of screening for an agent to determine its usefulness for reduction of cognitive impairment by providing a cell expressing a glutamate transporter or aspartate aminotransferase gene expressed by mammalian neural cells, contacting the cell with a test agent; and determining whether the activity or level of expression of the glutamate transporter (e.g., one or more of EAAT1, EAAT2, EAAT3, EAAT4 and EAAT5) and/or aspartate aminotransferase (AT) is increased in the presence of the test agent, where such an increase is an indication that the test agent is useful in promoting or preserving cognitive function. Expression can be assessed by art known methods including detecting changes in the rate or abundance of EAAT or AT mRNA. Glutamate transporter protein activity can be assessed by art-known methods, including measuring the uptake of ³H-glutamate uptake into cells (Lin et al., Nature 410: 84-88, 2001). Aspartate aminotransferase protein activity may be assess by art-known methods, including in a coupled reaction with malate dehydrogenase in the presence of NADH (Karmen, J Clin Invest 34:131, 1955; Amador and Wacker, Clin Chem 8:343, 1962).

Usually this determination comprises comparing the activity or expression in the test cell compared to a similar cell or cells (i.e., control cells) that have not been contacted with the test compound. In a related embodiment, the test compound is administered to a multicellular organism (e.g., animal). The EAAT or aspartate aminotransferase component may be wholly endogenous to the cell or multicellular organism or may be a recombinant cell or transgenic organism comprising one or more recombinantly expressed EAAT and/or aspartate aminotransferase proteins. Expression of recombinant EAAT and/or aspartate aminotransferase proteins can be accomplished using published gene and protein sequences and routine methods (see, e.g., Ausubel et al., Current Protocols In Molecular Biology, Greene Publishing and Wiley-Interscience, New York (supplemented through 2002).

The assays can be carried out using any cell type that expresses an EAAT and/or aspartate aminotransferase gene including, in various embodiments, a cultured cell (e.g., a cell in a primary culture or an established cell line) and a cell in vivo. Exemplary cells include neurons, glia cells, mixed neuronal cultures or cells in which EEAT and/or aspartate aminotransferase gene expression is induced by recombinant expression. Such cells (e.g., primary cultures) can be obtained from fetal hippocampus. Many other suitable cells or cell lines will be known to the practitioner.

The effect of an agent on EAAT and/or aspartate aminotransferase gene expression in a cell or in vitro system can be compared to a baseline value, which is typically the level of expression by the cell or in vitro system in the absence of the test agent. Expression levels can also be determined for cells that do not express EAAT and/or aspartate aminotransferase as a negative control. Such cells generally are otherwise substantially genetically the same as the test cells.

Other cell-based assays are reporter assays conducted with cells that do not necessarily express an EAAT and/or aspartate aminotransferase. Certain of these assays are conducted with a heterologous nucleic acid construct that includes an EAAT or aspartate aminotransferase gene promoter that is operably linked to a reporter gene that encodes a detectable product. EAAT gene promoters are located, in most cases, within a region about 300 to 1000 bp upstream (or 5') of the transcription start sites and are described in, for exampe, Su et al., PNAS 100:1955-1960, 2003. Aspartate aminotransferase gene promoters are located, in most cases, within a region about 300 to 1000 bp upstream (or 5') of the transcription start sites and are described in, for exampe Obaru et al., J Mol Biol. 200:13-22, 1988. Certain EAAT and aspartate aminotransferase gene promoters are described in GenBank (http://www.ncbi.nlm.nih.gov/) and the scientific literature. A number of different reporter genes can be utilized. Exemplary reporters include green fluorescent protein, J-glucuronidase, chloramphenicol acetyl transferase, luciferase, J-galactosidase, alkaline phosphatase, and the like. In these assays, cells harboring the reporter construct are contacted with a test compound. A test compound that either activates the promoter by binding to it or triggers a cascade that produces a molecule that activates the promoter causes expression of the detectable reporter. A variety of different types of cells can be utilized in the reporter assays (e.g., eukaryotic cells such as yeast, COS, CHO, HepG2, and HeLa cell lines).

Identification of agents that increase activity of the EAAT or aspartate aminotransferase protein can also include screening for compounds capable of binding to an EAAT or aspartate aminotransferase protein, as at least some of the compounds so identified are likely EAAT or aspartate aminotransferase modulators. Lead compounds identified during these screens can serve as the basis for the synthesis of more active analogs. Thus, in one aspect, the disclosure provides a method of screening for an agent to determine its usefulness in reduction of cognitive impairment by (a) contacting a polypeptide encoded by an EAAT or aspartate aminotransferase gene, or a cell expressing such a polypeptide with a test compound, and (b) determining whether the polypeptide binds to the test compound. Such binding is an indication that the test agent is useful in reduction of cognitive impairment. The binding assays usually involve contacting an EAAT or aspartate aminotransferase polypeptide with one or more test compounds and allowing sufficient time for the protein and test compounds to form a binding complex. Determining the ability of the test compound to directly bind to an EAAT or aspartate aminotransferase polypeptide can be accomplished, for example, by coupling the compound to a radioisotope or enzymatic label such that binding of the compound to the EAAT or aspartate aminotransferase polypeptide can be determined by detecting the labeled EAAT or aspartate aminotransferase polypeptide in a complex. Any binding complexes formed can be detected using any of a number of established analytical techniques. Protein binding assays include, but are not limited to, methods that measure co-precipitation, co-migration on non-denaturing SDS-polyacrylamide gels, and co-migration on Western blots (see, e.g., E.C. Hulme, 1992, "Receptor-Ligand Interactions" in A Practical Approach/The Practical Approach Series (Series Eds D. Rickwood and BD Hames) IRL Press at Oxford University Press). The EAAT (or aspartate aminotransferase) polypeptide utilized in such assays can be purified or recombinant. As noted above, recombinant expression and purification of EAAT (or aspartate aminotransferase) proteins can be accomplished using routine methods.

The EAAT or aspartate aminotransferase proteins can, in vivo, interact with one or more cellular and extracellular molecules (such as, without limitation, peptides, proteins, hormones, cofactors and nucleic acids) herein referred to as "binding partners." Methods are known for identify its natural in vivo binding partners of EAATs, e.g., two and three-hybrid assays (see, e.g., U.S. Pat. No. 5,283,317; Zervos et al, 1993, Cell 72:223-232; Madura et al, 1993, J. Biol. Chem. 268:12046-12054; Bartel et al, 1993, Biotechniques 14:920-924; Iwabuchi et al, 1993 Oncogene 8:1693-1696; Brent WO94/10300). Such EAAT or aspartate aminotransferase protein binding partners may be involved in the propagation of signals by the EAAT o r aspartate aminotransferase protein or downstream elements of an EAAT or aspartate aminotransferase protein-mediated signaling pathway, or, alternatively, may be found to be inhibitors of the EAAT or aspartate aminotransferase protein. Art-known assays can be devised through the use of the disclosure to identify compounds that modulate (e.g.; affect either positively or negatively) interactions between an EAAT or aspartate aminotransferase protein and its binding partners. Typically, the assay for compounds that interfere with the interaction between the EAAT or aspartate aminotransferase protein and its binding partner involves preparing a reaction mixture containing the EAAT or aspartate aminotransferase protein and its binding partner under conditions and for a time sufficient to allow the two products to interact and bind, thus forming a complex. In order to test an agent for inhibitory activity, the reaction mixture is prepared in the presence and absence of the test compound. Methods are provided for direct detection of interactions between the EAAT or aspartate aminotransferase protein and its natural binding partner and/or a test compound in a homogeneous or heterogeneous assay system without further sample manipulation. For example, the technique of fluorescence energy transfer may be utilized (see, e.g., Lakowicz et al, U.S. Pat. No. 5,631,169; Stavrianopoulos et al, U.S. Pat. No. 4,868,103).

In one aspect, agents identified by assay(s) described above can be administered to experimental animals to measure their cognition promoting and preserving activities (see, e.g., Example, *infra*).

A method is provided of screening compounds for utility in promoting cognitive function of a mammal by administering a test compound to a mammal, determining the level of expression of one or more EAAT or AT gene(s) in neural tissue of the mammal following administration of said test compound, comparing the level of expression of the gene(s) to a reference level of expression in neural tissue of a mammal to which the test compound was not administered and determining whether the level of expression of the gene differs from the corresponding reference level, where a difference indicates that the test compound is a candidate therapeutic agent for promoting cognitive function. The method may also include a further step of comparing the level of expression of a gene to reference level of expression in neural tissue of a mammal to whom ceftriaxone or valproic acid was administered. In one embodiment, the mammal is a rat, such as an aged rat

### 8. Therapeutic methods and compositions

Also disclosed is the administration of a pharmaceutical or sterile composition, in conjunction with a pharmaceutically acceptable carrier, for any of the therapeutic effects discussed above. Such pharmaceutical compositions may contain a molecule, such as a small molecule, that beneficially modulates expression of a gene associated with preservation or promotion of cognitive function during aging.

The inventors have unexpectly discovered that reduction of L-glutamate levels in the extracellular space surrounding neurons and glia cells in the brain, including the synaptic cleft and extrasynaptic space, is correlated with preservation or promotion of cognitive function during ageing. A method is provided for preserving or promoting cognitive function (e.g., to treat cognitive impairment associated with aging) in a mammal by increasing expression of glutamate transport proteins by brain cells. A method is for provided for reducing cognitive impairment associated with aging in a mammal by increasing the activity of glutamate transport proteins expressed in brain cells.

In one aspect, expression or activity of a glutamate transporter protein is increased by administration of a small molecule to the mammal. Exemplary small molecules include cephalosporin and analogs or derivatives thereof, valproic acid and analogs or derivatives thereof, MS-153 and analogs and derivatives thereof; and agonists of metabotropic glutamate receptors (mGluR's; see Aronica et al. *supra*). The small molecule may increase the expression or activity of the transporter protein directly (e.g., by interacting with a promoter of a transporter protein-encoding gene, or by interacting with the protein product itself) or indirectly (e.g., increasing expression or activity of a protein that stimulates expression or activity of a transporter protein or decreasing expression or activity of a protein that inhibits expression or activity of a transporter protein); and PACAP ("pituitary adenyl cyclase activator polypeptide").

Other exemplary compounds that can increase expression or activity of glutamate transporter proteins include lidocaine (Do et al., Anesth Analg. 2002 95:1263-8 "The effects of lidocaine on the activity of glutamate transporter EAAT3: the role of protein kinase C and phosphatidylinositol 3-kinase") and kinase inhibitors (e.g., Conradt, J Neurochem. 199768:1244-51 "Inhibition of the high-affinity brain glutamate transporter GLAST-1 via direct phosphorylation").

For illustration and not limitation exemplary therapeutic compounds are described in more detail in the following section.

### 8.1 Exemplary herapeutic compositions

Examples of small molecules, that beneficially modulates expression of a gene associated with promoting or preserving cognitive function during aging (e.g., an EAAT gene) include coumpound related to cephalosporin of the formula **I**: 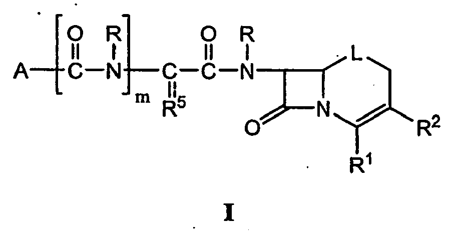 wherein, individually for each occurrence:
L is O or S;
R is H, C₁₋₁₀ alkyl, C₁₋₁₀ alkoxy, aryl, aralkyl, -OCH₂CO₂H;
R¹ is -(CH₂)ₙ-C(O)X
   wherein
   X is OH, NR₂, SH, O-alkali metal, or -OC(CH₃)OC(O)OCH(CH₃)₂; and n is an integer from 0 to 6 inclusive;
R² is H, C₁₋₁₀ alkyl, C₂₋₈ alkenyl, or -(CH₂)ₐ-W-R³
   wherein
   R³ is H, C₁₋₁₀ alkyl, -C(O)C₁₋₁₀ alkyl, -C(O)NR₂, aryl, aralkyl, or A;
   W is O, S, or NR⁴; and
   a is an integer from 1 to 6 inclusive;
      wherein
      R⁴ is H, C₁₋₁₀ alkyl, -C(O)C₁₋₁₀ alkyl, aryl, aralkyl, or R³ and R⁴ taken together may form an unsubstituted or substituted heteroalkyl or heteroaryl ring;
the line 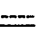 indicates either a single or double bond;
R⁵ is R¹, H, SO₃H, aryl, C₁₋₁₀ alkyl, aralkyl; or R⁵ is selected from the group consisting of =CHCH₂CO₂H and =NR when the 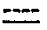 line is a double bond;
m is 0 or 1; and
A is aryl or heteroaryl of formula **Ia:** 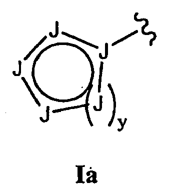 wherein, independently for each occurrence:
J is O, S, NR⁶, or CR⁶; and
y is 1 or 2;
   wherein R⁶ is an electron pair, H, C₁₋₁₀ alkyl, C₁₋₁₀ alkoxy, aryl, or -NR₂;
or A is heterocycloalkyl of formula **Ib** or **Ic**: 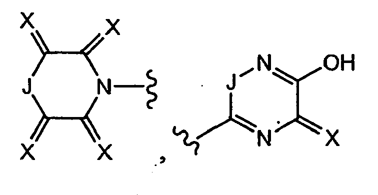 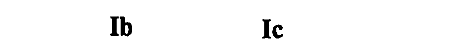 wherein, independently for each occurrence:
J is O, S, or NR; and
X is O or H₂.

Particular compounds of the class described by formula I includes "ceftriaxone" which refers to the broad spectrum cephalosporin antibotic, (6R,7R)-7-[2-(2-Amino-4-thiazolyl)glyoxylamido]-8-oxo-3-[[(1,2,5,6-tetrahydro-2-methyl-5,6-dioxo- as-triazin-3-yl)thio]methyl]-5-thia-1-azabicyclo[4.2.0]oct-2-ene-2-carboxyalic acid,7²-(Z)-(O-methyloxime), disodium salt, sesquaterhydrate. Ceftriaxone is available commercially from Roche under the trade name Rocephin™. Methods for making the compounds of formula may be found in, for example, U.S. Patent Nos.: 5,574,155; 5,739,346; 5,856,502; 5,869,649; 5,945,414; 5,945,532 and 6,090,801. Derivatives of ceftriaxone include any of the third-generation cephalosporins that are capable of killing aerobic gram-negative rods. Examples of third-generation cephalosporins are cefsulodin, cefotaxime, ceftizoxime, ceftriaxone, cefoperazone, moxalactam, and ceftazidime.

Other examples of small molecules that beneficially modulate expression of a gene associated with promoting or preserving cognitive function during aging (e.g., an EAAT gene) include compounds related to valproic acid of the formula **II**: 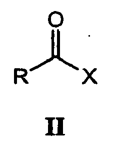 wherein, independently for each occurrence:
X is -OH, C₁₋₁₀ alkoxy, -O-alkali metal, -N(R¹)₂, -SH, or -S-C₁₋₁₀ alkyl;
R is a straight chain or branched C₁₋₃₀ alkyl; and
R¹ is H, C₁₋₁₀ alky, C₂₋₁₀ alkenyl, C₂₋₁₀ alkynyl, aryl, or aralkyl;
provided that R may be unsubstituted or substituted by one or more -OH, C₁₋₁₀ alkoxy, -N(R¹)₂, -SH, -S-C₁₋₁₀ alkyl, or aryl.

Particular compounds of the class described be formula II include "valproic acid" which refers to 2-propylpentanoate the anticonvulsant drug that may be related to increased brain concentrations of γ-aminobutyric acid (GABA). Other names and descriptions of valproic acid are also envisioned herein, such as Depakote™, Valproate™, Valrelease™ and sodium valproate. Methods for making the compounds of formula may be found in, for example, U.S. Patent Nos.: 4,558,070; 4,595,695; 4,654,370; 4,895,873; 4,913,906; 5,017,613; 5,019,398; 5,049,586, 5,162,573; 5,440,023; 5,856,569; 6,131,106 and 6,610,326.

Other examples of small molecules that beneficially modulate expression of a gene associated with promoting or preserving cognitive function during aging (e.g., an EAAT gene) include compounds related to (R)-(-)-5-methyl-1-nicotinoyl-2-pyrazoline of the formula **III:** 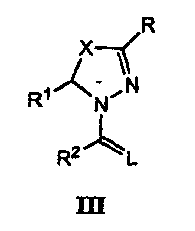 wherein, independently for each occurrence:
R is H, C₁-C₁₀ alkyl, C₂-C₁₀ alkenyl, C₂-C₁₀ alkynyl, aryl, or aralkyl;
R¹ is H, C₁-C₁₀ alkyl, C₂-C₁₀ alkenyl, C₂-C₁₀ alkynyl, aryl, or aralkyl;
R² is a heterocyclic or heteroaryl ring comprising from 1-4 heteroatoms selected from the following: N, O, or S;
L is O, S, or NR; and
X is CR₂, O, or S.

A particular compound of the formula III includes (R)-(-)-5-methyl-1-nicotinoyl-2-pyrazoline (MS-153).

Also included in the methods are pharmaceutically acceptable addition salts and complexes of the compounds of formula I, II and III. In cases wherein the compounds may have one or more chiral centers, unless specified, the present invention comprises each unique racemic compound, as well as each unique nonracemic compound.

In cases in which the compounds have unsaturated carbon-carbon double bonds, both the cis (Z) and trans (E) isomers are within the scope of this invention. In cases wherein inhibitors may exist in tautomeric forms, such as keto-enol tautomers, such as 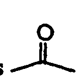 and 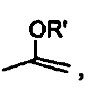 each tautomeric form is contemplated as being included within this invention, whether existing in equilibrium or locked in one form by appropriate substitution with R'. The meaning of any substituent at any one occurrence is independent of its meaning, or any other substituent's meaning, at any other occurrence.

Also included in the methods are prodrugs of the compounds of formula I, II and III.

The pharmaceutical compositions may be administered by any number of routes including, but not limited to, oral, intravenous, intramuscular, intra-arterial, intramedullary, intrathecal, intraventricular, transdermal, subcutaneous, intraperitoneal, intranasal, enteral, topical, sublingual, or rectal means.

The compositions may be administered alone or in combination with at least one other agent, such as a stabilizing compound, which may be administered in any sterile, biocompatible pharmaceutical carrier including, but not limited to, saline, buffered saline, dextrose, and water. The compositions may be administered to a patient alone, or in combination with other agents, drugs, or hormones.

Certain compounds may exist in particular geometric or stereoisomeric forms. In addition, polymers may also be optically active. All such compounds are contemplated; including cis- and trans-isomers, *R-* and *S*-enantiomers, diastereomers, (D)-isomers, (L)-isomers, the racemic mixtures thereof, and other mixtures thereof, as falling within the scope of the invention. Additional asymmetric carbon atoms may be present in a substituent such as an alkyl group. All such isomers, as well as mixtures thereof, are intended to be included .

If, for instance, a particular enantiomer of a compound is desired, it may be prepared by asymmetric synthesis, or by derivation with a chiral auxiliary, where the resulting diastereomeric mixture is separated and the auxiliary group cleaved to provide the pure desired enantiomers. Alternatively, where the molecule contains a basic functional group, such as amino, or an acidic functional group, such as carboxyl, diastereomeric salts are formed with an appropriate optically-active acid or base, followed by resolution of the diastereomers thus formed by fractional crystallization or chromatographic means well known in the art, and subsequent recovery of the pure enantiomers.

For purposes of this invention, the chemical elements are identified in accordance with the Periodic Table of the Elements, CAS version, Handbook of Chemistry and Physics, 67th Ed., 1986-87, inside cover. Also for purposes of this invention, the term "hydrocarbon" is contemplated to include all permissible compounds having at least one hydrogen and one carbon atom. In a broad aspect, the permissible hydrocarbons include acyclic and cyclic, branched and unbranched, carbocyclic and heterocyclic, aromatic and nonaromatic organic compounds that may be substituted or unsubstituted.

### 8.2 Therapeutic compositions

Contemplated equivalents of the compositions described herein include compositions which otherwise correspond thereto, and which have the same general properties thereof, wherein one or more simple variations of substituents or components are made which do not adversely affect the characteristics of the compositions of interest.

In addition to the active ingredients, e.g., one or more of the therapeutic agents, pharmaceutical compositions of the present invention may contain suitable pharmaceutically-acceptable carriers comprising excipients and auxiliaries which facilitate processing of the active compounds into preparations which can be used pharmaceutically. Further details on techniques for formulation and administration may be found in the latest edition of Remington's Pharmaceutical Sciences (Maack Publishing Co., Easton, Pa.).

Pharmaceutical compositions for oral administration can be formulated using pharmaceutically acceptable carriers well known in the art in dosages suitable for oral administration. Such carriers enable the pharmaceutical compositions to be formulated as tablets, pills, dragees, capsules, liquids, gels, syrups, slurries, suspensions, and the like, for ingestion by the patient.

Pharmaceutical preparations for oral use can be obtained through combining active compounds with solid excipient and processing the resultant mixture of granules (optionally, after grinding) to obtain tablets or dragee cores. Suitable auxiliaries can be added, if desired. Suitable excipients include carbohydrate or protein fillers, such as sugars, including lactose, sucrose, mannitol, and sorbitol; starch from corn, wheat, rice, potato, or other plants; cellulose, such as methyl cellulose, hydroxypropylmethyl-cellulose, or sodium carboxymethylcellulose; gums, including arabic and tragacanth; and proteins, such as gelatin and collagen. If desired, disintegrating or solubilizing agents may be added, such as the cross-linked polyvinyl pyrrolidone, agar, and alginic acid or a salt thereof, such as sodium alginate.

Dragee cores may be used in conjunction with suitable coatings, such as concentrated sugar solutions, which may also contain gum arabic, talc, polyvinylpyrrolidone, carbopol gel, polyethylene glycol, and/or titanium dioxide, lacquer solutions, and suitable organic solvents or solvent mixtures. Dyestuffs or pigments may be added to the tablets or dragee coatings for product identification or to characterize the quantity of active compound, i.e., dosage.

Pharmaceutical preparations which can be used orally include push-fit capsules made of gelatin, as well as soft, sealed capsules made of gelatin and a coating, such as glycerol or sorbitol. Push-fit capsules can contain active ingredients mixed with fillers or binders, such as lactose or starches, lubricants, such as talc or magnesium stearate, and, optionally, stabilizers. In soft capsules, the active compounds may be dissolved or suspended in suitable liquids, such as fatty oils, liquid, or liquid polyethylene glycol with or without stabilizers.

Pharmaceutical formulations suitable for parenteral administration may be formulated in aqueous solutions, preferably in physiologically compatible buffers such as Hanks' solution, Ringer's solution, or physiologically buffered saline. Aqueous injection suspensions may contain substances which increase the viscosity of the suspension, such as sodium carboxymethyl cellulose, sorbitol, or dextran. Additionally, suspensions of the active compounds may be prepared as appropriate oily injection suspensions. Suitable lipophilic solvents or vehicles include fatty oils, such as sesame oil, or synthetic fatty acid esters, such as ethyl oleate, triglycerides, or liposomes. Non-lipid polycationic amino polymers may also be used for delivery. Optionally, the suspension may also contain suitable stabilizers or agents to increase the solubility of the compounds and allow for the preparation of highly concentrated solutions.

For topical or nasal administration, penetrants appropriate to the particular barrier to be permeated are used in the formulation. Such penetrants are generally known in the art.

The pharmaceutical compositions of the present invention may be manufactured in a manner that is known in the art, e.g., by means of conventional mixing, dissolving, granulating, dragee-making, levigating, emulsifying, encapsulating, entrapping, or lyophilizing processes.

The pharmaceutical composition may be provided as a salt and can be formed with many acids, including but not limited to, hydrochloric, sulfuric, acetic, lactic, tartaric, malic, and succinic acids. Salts tend to be more soluble in aqueous or other protonic solvents than are the corresponding free base forms. In other cases, the preferred preparation may be a lyophilized powder which may contain any or all of the following: 1 mM to 50 mM histidine, 0. 1% to 2% sucrose, and 2% to 7% mannitol, at a pH range of 4.5 to 5.5, that is combined with buffer prior to use.

After pharmaceutical compositions have been prepared, they can be placed in an appropriate container and labeled for treatment of an indicated condition. For administration of ceftriaxone, for example, such labeling would include amount, frequency, and method of administration.

Pharmaceutical compositions include compositions wherein the active ingredients are contained in an effective amount to achieve the intended purpose. The determination of an effective dose is well within the capability of those skilled in the art.

For any compound, the therapeutically effective dose can be estimated initially either in cell culture assays, e.g., according to the method of Aronica et al., supra, or in animal models such as mice, rats, rabbits, dogs, or pigs. An animal model may also be used to determine the appropriate concentration range and route of administration. A particularly preferred animal model uses behaviorally characterized rats as described herein. Such information can then be used to determine useful doses and routes for administration in humans.

A therapeutically effective dose refers to that amount of active ingredient, for example ceftriaxone, ceftriaxone analogs, ceftriaxone derivatives, valproic acid, valproic acid analogs, valproic acid derivative, MS-153, MS-153 analogs or MS-153 derivatives, which ameliorate the symptoms or condition. Therapeutic efficacy and toxicity may be determined by standard pharmaceutical procedures in cell cultures or with experimental animals, such as by calculating the ED₅₀ (the dose therapeutically effective in 50% of the population) or LD₅₀ (the dose lethal to 50% of the population) statistics. The dose ratio of therapeutic effects to toxic effects is the therapeutic index, which can be expressed as the LD₅₀ /ED₅₀ ratio. Pharmaceutical compositions which exhibit large therapeutic indices are preferred. The data obtained from cell culture assays and animal studies are used to formulate a range of dosage for human use. The dosage contained in such compositions is preferably within a range of circulating concentrations that includes the ED₅₀ with little or no toxicity. The dosage varies within this range depending upon the dosage form employed, the sensitivity of the patient, and the route of administration.

The exact dosage will be determined by the practitioner, in light of factors related to the subject requiring treatment. Dosage and administration are adjusted to provide sufficient levels of the active moiety or to maintain the desired effect. Factors which may be taken into account include the degree of cogntive impairment, the general health of the subject, the age, weight, and gender of the subject, time and frequency of administration, drug combination(s), reaction sensitivities, and response to therapy. Long-acting pharmaceutical compositions may be administered every 3 to 4 days, every week, or biweekly depending on the half-life and clearance rate of the particular formulation.

Normal dosage amounts may vary from about 0.1 µg to 100,000 µg, up to a total dose of about 1 gram, depending upon the route of administration. Guidance as to particular dosages and methods of delivery is provided in the literature and generally available to practitioners in the art.

To be exert a therapeutic effect on central nervous system targets, such as EAAT1, 2, 3, 4 or 5, the compounds used in the methods of the present invention should readily penetrate the blood-brain barrier when peripherally administered. Compounds which cannot penetrate the blood-brain barrier, however, can still be effectively administered directly into the central nervous system, e.g., by an intraventricular route.

The term "pharmaceutically-acceptable salts" is art-recognized and refers to the relatively non-toxic, inorganic and organic acid addition salts of compounds, including, for example, those contained in compositions of the present disclosure.

The term "pharmaceutically acceptable carrier" is art-recognized and refers to a pharmaceutically-acceptable material, composition or vehicle, such as a liquid or solid filler, diluent, excipient, solvent or encapsulating material, involved in carrying or transporting any subject composition or component thereof from one organ, or portion of the body, to another organ, or portion of the body. Each carrier must be "acceptable" in the sense of being compatible with the subject composition and its components and not injurious to the patient. Some examples of materials which may serve as pharmaceutically acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

The terms "systemic administration," "administered systemically," "peripheral administration" and "administered peripherally" are art-recognized and refer to the administration of a subject composition, therapeutic or other material other than directly into the central nervous system, such that it enters the patient's system and, thus, is subject to metabolism and other like processes, for example, subcutaneous administration.

The terms "parenteral administration" and "administered parenterally" are art-recognized and refer to modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intra-articular, subcapsular, subarachnoid, intraspinal, and intrasternal injection and infusion.

### 9. Exemplification

The disclosure having been generally described, may be more readily understood by reference to the following examples, which are included merely for purposes of illustration of certain aspects and embodiments of the present disclosure

### 9.1 Characterizing the young, aged-impaired (AI) and aged-unimpaired (AU) animals 9.1.1 Morris Water Maze (MWM) and Radial Arm Maze (RAM) Subjects

We performed behavioral tests on 9 young (4-6 mo) and 18 aged (25-27 months) pathogen-free male Long-Evans rats with the MWM and used the same animals for microarray analysis. An additional 10 aged rats were tested in the MWM, followed by training and testing in the RAM to assess test-retest reliability for individual differences in cognitive function across the two tasks.

### 9.1.2 Morris Water Maze Apparatus

The MWM apparatus consists of a large, circular pool (diameter 1.83 m; height, 0.58 m) filled with water (27°C) that has been made opaque through the addition of non-toxic pigment or some other substance. In the typical "hidden platform" version of the task, rats are trained to find a camouflaged white escape platform (height, 34.5 cm) that is positioned in the center of one quadrant of the maze just 1.0 cm below the water surface. This platform could be retracted to the bottom of the tank or raised to its normal position from outside the maze during behavioral testing. The location of this platform remained constant from trial to trial. Because there were no local cues that marked the position of the platform, the rat's ability to locate it efficiently from any starting position at the perimeter of the pool depended on using information surrounding the maze. The maze was surrounded by black curtains with white patterns affixed to provide a configuration of spatial cues. A second platform (height 37.5 cm), with its surface painted black was elevated 2 cm above the water surface during cue training, the version of the task used to control for factors unrelated to cognition. The behavior of a rat in the pool was recorded by a camera suspended 2.5 m above the center of the pool, connected to a video tracking system (HVS Image Advanced Tracker VP200) and a PC computer running HVS software developed by Richard Baker of HVS Image, Hampton, UK.

### 9.1.3 Morris Water Maze Procedure

We optimized the MWM protocol for sensitivity to the effects of aging on cognition and for measures of reliable individual differences within the aged population of out-bred Long-Evans rats (Gallagher M, Burwell R, Burchinal M. Behav. Neurosci. 107:618-626; 1993).

Rats received three trials per day for 8 consecutive days, using a 60 sec intertrial interval. On each training trial, the rat was released in the maze from one of four equally spaced starting positions around the perimeter of the pool. The starting position varied from trial to trial, thus preventing the use of a response strategy (e.g. always turning left from the start location to locate the escape platform). If a rat did not locate the escape platform within 90 sec on any trial, the experimenter guided the rat to the platform, where it remained for 30 sec. Every sixth trial consisted of a probe trial to assess the development of spatial bias in the maze. During these trials, the rat swam with the platform retracted to the bottom of the pool for 30 sec, at which time the platform was raised to its normal position for completion of an escape trial. At the completion of the protocol using the hidden platform, rats were assessed for cue learning using the visible platform. The location of this platform varied from trial to trial in a single session of 6 training trials.

We used the proximity of the animal's position with respect to the goal for analysis of training trial and probe trial performance. The proximity measure was obtained by sampling the position of the animal in the maze (10X/sec) to provide a record of distance from the escape platform in 1 sec averages. For both probe trials and training trials, a correction procedure was implemented so that trial performance was relatively unbiased by differences in distance to the goal from the various start locations at the perimeter of the pool. In making this correction the average swimming speed was calculated for each trial (pathlength/latency). Then the amount of time required to swim to the goal at that speed from the start location used on the trial was removed from the record prior to computing trial performance, i.e. cumulative distance on training trials and average distance from the goal on probe trials. Thus, scores obtained using the proximity measure are designed to reflect search error, representing deviations from an optimal search, i.e. direct path to the goal and search in the immediate vicinity of that location during probe trials.

### 9.1.4 Morris Water Maze Analysis

Computer records of video-tracking were compiled to provide data on each rat's performance in the maze. Measures on training trials and probe trials were analyzed by Analysis of Variance.

### 9.1.5 Morris Water Maze Data Results

The performance during training with the hidden, camouflaged platform differed between the groups of young and aged rats [F(1,23)= 12.69, p<.002]. No difference between the groups occurred for the cue training trials with a visible platform. Latencies to escape during cue training averaged 9.36 seconds for young and 10.60 seconds for the aged rats.

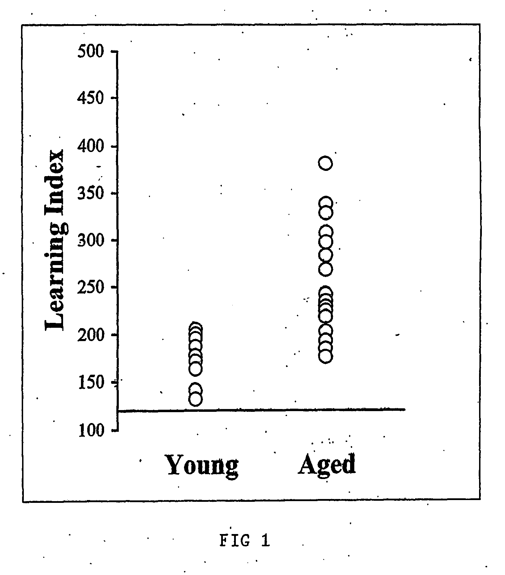

The average proximity measure on interpolated probe trials was used to calculate a spatial learning index for each individual subject as described in detail in Gallagher M, Burwell R, Burchinal M. Behav. Neurosci. 107:618-626; 1993. When a rat rapidly learned to search for the platform close to its position, it's spatial learning index is low. Overall, aged rats differed from young [F(1,23) =15.18, p<.001]. Aged rats were classified as either unimpaired or impaired relative to the learning index profile of the young study population. Aged rats that fall within the normative range of young rats (index scores <241) were designated aged unimpaired (Figure 1). The remaining aged subjects that have index scores outside the range of young performance were designated aged impaired.

### 9.1.6 Radial Arm Maze Apparatus

Each arm (7 x 75 cm) of the elevated eight arm radial maze projected from each facet of an octagonal center platform (30 cm diameter, 51.5 cm height). Clear side walls on the arms were 10 cm high and were angled at 65° to form a trough. A food well (4 cm diameter, 2 cm deep) was located at the distal end of each arm. Blocks constructed of Plexiglas (30 cm H x 12 cm W) could be positioned to block entry to any arm. Numerous extra maze cues were provided in the room surrounding the apparatus and lighting was provided by overhead fixtures.

### 9.1.7 Radial Arm Maze Procedures

Rats were first habituated to the maze for an 8 min session on four consecutive days. In each of these sessions food rewards were scattered on the RAM, initially on the center platform and arms and then progressively confined to the arms. After this habituation phase, a standard training protocol was used in which a food pellet was located at the end of each arm. Rats received one trial each day for 18 days; each daily trial terminated when all eight food pellets had been obtained or when either 16 choices were made or 15 min had elapsed. An error consisted of returning to an arm (all four paws on the arm) from which food had already been obtained. After completion of this phase, the memory demand of the task was increased by imposing a delay during the trial. At the beginning of each trial three arms were blocked. The identity and configuration of the blocked arms was varied across trials. Rats were allowed to obtain food on the five arms to which access was permitted at the beginning of the trial. The rat was then removed from the maze for 60 s, during which time the barriers on the maze were removed, thus allowing access to all eight arms. Rats were then placed back onto the center platform and allowed to obtain the remaining food rewards.

### 9.1.8 Radial Arm Maze Analysis

A memory error occurred during test trials using a 60 second delay when a rat returned to one of the five arm that was already visited prior to the delay. Each rat's performance was averaged across four consecutive test trials. Parametric statistics (unpaired t-tests) were used to compare performance between young and aged groups. Correlational analysis (Pearson's r) was used to examine the relationship between performance of aged rats (N=10) in the Morris Water maze (learning index scores) and radial-arm maze (memory errors).

### 9.1.9 Radial Arm Maze Results

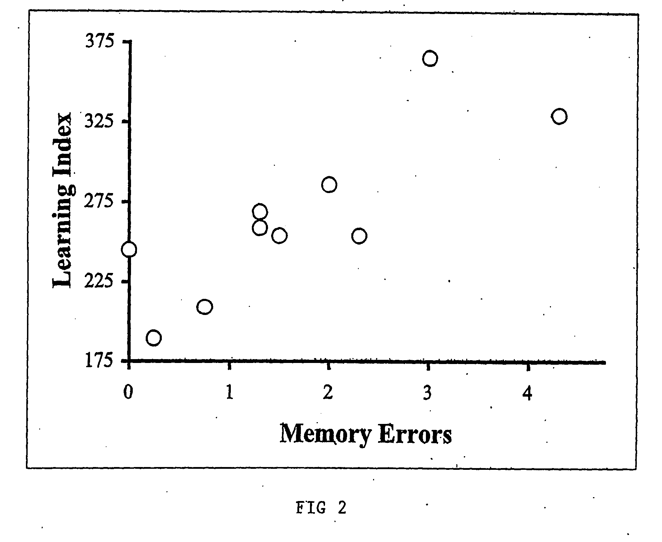

The performance of young adult rats in the delay version of the RAM varies as a function of the delay interval, ranging from 60 seconds to eight hours (Chappell et al. Neuropharmacology 37: 481-488, 1998). Aged rats previously characterized in the MWM, committed more memory errors after a 60 second delay relative to young rats (p < .025). On average young rats committed 0.17 errors, whereas aged rats committed an average of 1.52 errors. The ten aged rats, however, exhibited a wide range of performance on the RAM. A significant relationship was found between the initial MWM characterization and memory performance in the RAM (r value = .82, data shown in Figure 2).

### 9.2 Gene expression analysis of the young, aged-impaired (AI) and aged-unimpaired (AU) animals

### 9.2.1 Preparation of RNA from behaviorally characterized animals

Twenty-seven behaviorally characterized rats (data shown in Figure 1) were killed with an overdose of sodium pentobarbital (100 mg/kg). The hippocampus was dissected bilaterally and frozen (-80°C). One hippocampus from each animal was weighed and homogenized in the appropriate volume of phenol-guanadine isothiocyanate (Trizol reagent; 1ml per 100mg of tissue with a minimum volume of 1 ml). Each sample was extracted with chloroform (200µl per ml of Trizol) and precipitated with isopropanol. RNA pellets were air dried and resuspended in DEPC treated water. All samples were stored at -80°C. A portion of the RNA was further purified using Qiagen's RNeasy mini RNA extraction kit according to manufacturer's instructions and subsequently stored at -80°C. Samples were quantified by absorbance at 260 nm and purity determined by ratio of absorbance at 260nm and 280nm. Sample integrity and concentration was confirmed by agarose gel electrophoresis. Photographs of agarose gels were scanned, the pixels were inverted and quantified using NIH-image. Concentrations were then adjusted if needed.

For analysis on gene microarrays, samples from three rats of the same phenotype, either Y, AI or AU, were pooled to yield independent microarray analysis for a sample size of three GeneChips®/phenotype. With respect to behavioral characterization by spatial learning index in the MWM, tissue was pooled as set forth in Table I

**Table I**

| **Young** | **Aged Unimpaired** | **Aged Impaired** |
|---|---|---|
| Y1: 143.1, 171.4, 194.6 | AU1: 181.5, 202.9, 229.5 | AI1: 268.3, 283.2, 381 |
| Y2:139.5, 169.4, 186.6 | AU2: 183.9, 218.4, 229.3 | AI2: 285.5, 303.4, 337.7 |
| Y3: 169, 173.9, 196.5 | AU3: 193.3, 228.2, 230.6 | AI3: 282.2, 307.2, 329.3 |

### 9.2.2 Reverse transcription and hybridization to microarray

Labeled cRNA probes for hybridization were prepared using the Affymetrix Enzo Bioarray high yield RNA transcript labeling system. RNAs were reverse transcribed into cDNA and converted to biotin labeled cRNA. Internal standards provided with each labeling system were added to test RNA prior to reverse transcription. cRNAs were then tested on control chips to ensure that reverse transcription and labeling were optimal before performing hybridization onto experimental GeneChips®. cRNAs were applied to U34A Affymetrix GeneChip® arrays. These arrays included specific sequences for 7000 expressed rat genes and 1000 EST clusters, and included all genes represented on a recently developed, smaller, neuroscience gene microarray. A GeneChip Fluidics Station automated introduction of the labeled cRNAs on to the gene arrays and hybridization as conducted in a GeneChip hybridization oven. A GeneArray scanner was used to detect and quantify hybridization signals for each oligomer set based on confocal laser scanning.

### 9.2.3 Data analysis of microarray

A Genechip Analysis Suite and Affymetrix MAS 4.0 and the more recently developed MAS 5.0 algorithms were used in our analysis of data. Both algorithms had a default threshold based on known negative genes and average signal intensities per chip. Normalization and scaling methods based on predefined oligomer sets defined by Affymetrix were applied to permit comparisons between Genechips. Both algorithms generated values for levels of expression per set of perfectly matched (PM) oligomers corresponding to each expressed gene, relative to a set of mismatched (MM) oligomers designed with variant bases calculated to inhibit hybridization of a perfectly matched cRNA. The empirical MAS 4.0 algorithm used raw data to generate an average difference call which provides a measure of the hybridization signal intensity for PM oligomers relative to control MM oligomers for each gene. An absolute call of present (P), marginal (M), or absent (A) was based on the number of PM oligomers which were positive relative to MM oligomers. The statistical MAS 5.0 algorithms relied on essentially the same type of comparison but applied statistical methods to generate a p value to enhance the probability that a call of present reflected a level of expression higher than background. In addition, statistical criteria were applied to the signal algorithm in calculating hybridization signal intensity which were intended to minimize the impact of outliers within PM and MM oligomers in each probe set. The basic difference in data representing expression levels yielded by the two algorithms was that MAS 5.0 was designed to eliminate negative expression levels which would lead to an overestimate of the number of genes with altered expression levels between two comparison groups. Overall the signal obtained per oligomer set with MAS 5.0 was less than that with MAS 4.0.

The power of our model lies, in part, in the ability to compare across the three groups Y, AU and AI to identify those genes which either change between young and aged hippocampus and thus generally relate to the aging process and those which discriminate AU and AI rats. The genes identified through this process relate specifically to aging-cognitive impairment or preservation of cognitive function.

In generating the data disclosed herein we conducted a series of analytic steps to identify 3 sets of genes informative for the model of age and cognitive impairment. Set 1 comprised genes of interest that differ from young as a function of age alone. Set 2 comprised of genes of interest that differ in the impaired aged rats relative to both young and aged unimpaired. Set 3 (referred to as Aged Unimpaired genes) consisted of genes that differ in the aged unimpaired relative to both young and aged impaired and may, therefore, related to age-induced preservation of cognitive function. Each set was generated from the full microarray dataset following the MAS 5.0 analysis and then used similar algorithms for an effect size analysis.

Herein we described the steps for generating Set 3 (Aged Unimpaired genes), in which glutamate transporters were included. In the first step the values for all probe sets on the chips representing gene expression in the young rats (N=3) and chips for the aged impaired (N=3) were examined for a detection criterion. All probe pair sets that did not meet the detection criterion using the absolute call from the MAS 5.0 analysis were eliminated from further consideration. A simple effects analysis was then conducted to determine that the values on the chips for the two groups (young and aged impaired) did not differ by an effect size of greater than 1.0. All probe sets that met both detection criterion and the criterion for pooling in the comparison group (young and aged impaired not differing by an effect size of 1.0 or greater). A simple effects analysis was then conducted on those pooled probe sets for young and aged impaired along with the corresponding probe sets from the aged unimpaired chips. The simple effects analysis yielded 384 probe sets, representing "genes of interest" for age-induced preservation of cognitive function, with an effect size of 1.25 or greater. Power analysis for inferential statistical tests of significance indicated that the sample sizes of the microarray experiment (3 aged unimpaired chips and 6 chips in the pooled comparison group) would detect a difference at p<.05 with 80% power for genes with an effect size of 2.5 or greater. In follow-up analysis with sample sizes of 9 aged unimpaired, and 9 subjects in each of the comparison groups (young and aged impaired) that are now underway, statistical power at 80% could be expected for effect sizes of 1.25 and greater.

### 9.2.4 Results from the microarray

Different transcripts for glutamate transporters were detected in the human and rodent nervous system (Figure 3). Two of these transcripts were not expressed in the hippocampal formation (EAAT 4 and EAAT5). The three remaining transcripts had differing patterns of cellular localization in neurons and glia, and were all expressed in the hippocampus. In the effect size analysis for Aged Unimpaired genes (Set 3) in the microarray dataset GLT-1 had an effect size of 5.87 (the largest value in the effect size analysis for aged unimpaired genes) and GLAST, a second glutamate transporter, had an effect size of 1.93 (Figure 4). In both cases the glutamate transporter mRNA was increased in the unimpaired aged rats relative to the pooled comparison of young and aged impaired. As expected from the power analysis for the microarray experimental design, GLT1 mRNA was significantly increased (p<.0002) relative to the comparison young and aged impaired. In a similar analysis, GLAST was also significantly increased (p=.0484; Figure 4).

An additional indication that glutamate is regulated differently in aged rats with preserved cognitive function was provided by a difference in mRNA for aspartate aminotransferase in Aged Unimpaired relative to Young and Aged Impaired. The probe set for aspartate aminotransferase, a major mechanism for inactivation of extracellular glutamate, showed a significant increase in the AU rats relative to the comparison Y + AI, which did not differ from each other. The effect size for that mRNA was 2.58.

A probe set on the microarray was an identified gene for pituitary adenyl cyclase activator polypeptide (PACAP; GenBank Accession No.: AI227715; EST224410), that regulates glutamate transport and metabolism (Figiel and Engele, J. Neurosci. 15: 3596-3605, 2000). PACAP mRNA was significantly elevated in aged unimpaired relative to comparison young and aged impaired rats (Effect size 3.29, t=4.13, p<.005; Figure 4).

Because PACAP receptors are of a type that exhibit desensitization, a strong increase in β-arrestin 2 mRNA may participate in effects on glutamate tranporters. Beta-arrestin 2 has a dual role in receptor endocytosis and in mediating signaling cascades through the same receptors (Wei et al., PNAS, 100;10782-7,2003; Ahn et al., PNAS, 100;1740-4,2003; e.g. GenBank Accession No.: XM_345084). The probe sets for β-arrestin 2 in the aged unimpaired rats showed significant elevation relative to comparison young and aged impaired (Effect size = 2.78, t=2.59, p<.05).

### 9.2.5 In situ hybridization histochemistry analysis

A follow-up analysis was conducted using an independent set of animals (N of 3 or 4 per group) using sections of hippocampus processed for in situ hybridization histochemistry.

### 9.2. 6 In situ hybridization probe preparation

We used reverse-transcriptase polymerase chain reaction (RT-PCR) to generate specific probes corresponding to glutamate transporters identified as up-regulated in hippocampus AU rats by the microarray analyses. A sample RNA prepared from hippocampus of aged rats ( pool of animals with learning index scores within (AU) or outside (AI) the scores of young animals was reverse transcribed using oligodT and reverse transcriptase. This cDNA was then used as a template with sense and antisense oligomer probes (Table II) in a first round PCR reactions to amplify double stranded cDNAs corresponding to the particular glutamate transporters described below. Standard PCR conditions were employed (initial denaturation at 94°C for 4 min, initial annealing at 72°C for 40 sec, 35 cycles of 94°C, 30 sec denaturation, 55°C, 30 sec annealing and 72°C 30 sec extension, a final extension at 72°C for 4 min and cooling to 4°C). Aliquots of PCR products were electrophoresed on ethidium bromide-agarose gels to verify that probes of appropriate size were generated. A second round PCR reaction was used with each of the amplified cDNAs and extended oligomers (Table II) comprising a sense oligomer corresponding to an SP6 promoter sequence plus the original sense oligomer used to derive each glutamate transporter PCR product, and an antisense oligomer comprising the T7 promoter sequence and the original antisense oligomer for each glutamate transporter. This generated specific glutamate transporter cDNAs with SP6 and T7 promoters on sense and antisense strands, respectively. PCR products were collected by ethanol precipitation and the sequences verified by nucleotide sequencing using an automated DNA sequencer and SP6 primers.

Sequence-verified PCR products corresponding to each glutamate transporter with SP6 and T7 promoter sites were used in DNA directed in vitro transcription of sense and antisense RNA from each second round glutamate transporter PCR product. In vitro transcription reactions were performed with each PCR product, SP6 or T7 polymerase (each enzyme used in separate reactions for each probe), unlabelled nucleotide triphosphates and high specific activity ³⁵S labeled uridine triphosphate. These reactions yielded three radiolabeled antisense glutamate transporter probes which specifically recognize each of the different glutamate transporter mRNAs in tissue sections by base pairing and corresponding sense probes which comprised the same sequence as glutamate transporter mRNAs, cannot base pair with the mRNAs and served as a negative control. Antisense probes were incubated with frozen sections of hippocampus from Y, AU an AI rats representing sections throughout the entire hippocampus. Sense probes were hybridized with sections from fewer selected regions of hippocampus but sufficient to verify uniformly negative hybridization signals and validate specificity of hybridization signals obtained with antisense probes. After hybridization and washing, sections were apposed to X-ray film and standard quantitative autoradiography (Bizon 2001) was used to quantify signals.

Oligomers used to derive templates for preparation of glutamate transporter sense and antisense RNA probes for in situ hybridization histochemistry were described in Table II.

**Table II**

| | **GLT-1** | **EAAC1** | **GLAST** |
|---|---|---|---|
| PCR product | 405bp | 409bp | 441bp |
| Sense | 5'-GAGCATTGGTG CAGCCAGTA-3' | 5’-GTCTGAGAACA AGACAAAGG-3’ | 5'-GGTAGAAGCCT GCTTTAAAC-3' |
| Antisense | 5'-CCAAGGTTCTTCC TCAACAC-3' | 5'-TGAGAGCTGTCA GGAGAGC-3' | 5'-GGCATGAATGAG GAGGCCGAC-3' |
| Extended SP6 Sense | 5'-tatttaggtgacactatagGAGCA TTGGTGCAGCCAGTA-3' | 5’-tatttaggtgacactatagGTCT GAGAACAAGACAAAG G-3’ | 5'-tatttaggtgacactatag GGTA GAAGCCTGCTTTAAAC-3' |
| Extended T7 Sense | 5'taatacgactcactataggggCCAA GGTTCTTCCTCAAC-3' | 5'taatacgactcactataggggTGA GAGCTGTCAGGAGAG C-3' | 5'-taatacgactcactataggggGG CATGAATGAGGAGGCCGA C-3' |

### 9.2.7 Preparation of tissue for in situ hybridization histochemistry

Eleven behaviorally characterized rats (n=4 young and n=7 old) were killed with an overdose of sodium pentobarbital (100 mg/kg) and intracardial perfusion with 4% paraformaldehyde in 0.1 M phosphate buffer, pH 7.4 (PPB). All perfusions were performed between 0700 h and 1000 h. Following fixation, brains were removed from the cranium and both right and left hippocampi were immediately dissected from the surrounding tissue. Dissected hippocampi were postfixed in PPB for 24 h, cryoprotected in PPB containing 20% sucrose for 24 h, frozen on powdered dry ice in an "extended" orientation, and stored at -80°C until further processing.

The hippocampus for each animal was sectioned (25 mm) coronal to the longitudinal axis using a freezing microtome, and collected into cold PPB. Free-floating sections of tissue were washed in 0.75% glycine in 0.1 M phosphate buffer, pH, 7.2 (PB) and 0.1M PB alone to remove excess fixative. Sections were treated for 30 min at 37°C with proteinase K (1 mg/ml in 0.1 M Tris buffer containing 0.05% SDS), acetylated in 0.25% acetic anhydride in 0.1 M triethanolamine, pH 8.0, and rinsed twice in 2X saline sodium citrate buffer (SSC; 1XSSC = 0.15 M sodium chloride and 0.015 M sodium citrate, pH 7.0). Tissue was then hybridized for 42-44 h at 60°C in solution containing 50% formamide, 1 X Denhardt's solution, 10% dextran sulfate, 4XSSC, 0.25 mg/ml yeast tRNA, 0.3 mg/ml herring sperm DNA, 100 mm dithiothreitol (DTT), and the appropriate ³⁵S-labeled cRNA at a final concentration of 1X107 CPM/ml. Following hybridization, sections were washed at 30 min intervals, twice in 4XSSC, once in 50% formamide/2XSSC at 60°C and then treated with ribonuclease A (20 mg/ml in 10 mM Tris saline buffer containing 1 mM ethylene-diaminetetracetic acid) for 30 min at 37°C. Tissue sections were washed further in descending concentrations of SSC buffer containing 100 mM DTT to a final wash of 0.1XSSC and mounted onto gelatin-coated slides for film autoradiography. Air-dried sections of the hippocampal sections were exposed with ¹⁴C-standards (American Radiolabeled Chemicals, Inc., St. Louis, MO) to β-max hyperfilm (Amersham Pharmacia Biotech, Piscataway, New Jersey) for 24-72 hours. The exposure time for rostral coronal sections was 110-130 hours. Films were developed using GBX developer and fixed with Kodak rapid fixer.

In situ hybridization labeling was quantified by densitometric analysis of film autoradiograms using the MCID imaging system (Imaging Research, St. Catherine's, Ontario, Canada). Film densities were linearized and calibrated relative to the ¹⁴C-labeled standards that were exposed to each sheet of film with tissue sections. Values for hybridization signal intensity (mCi/ gram protein) were calculated for each rat as the average of multiple measures from 6-8 tissue sections. Mean hybridization signal intensities for each rat within a group were averaged to obtain a group mean ± standard error. Statistical comparisons were made using a one-way ANOVA. For all statistical tests, a 95% confidence level (p < 0.05) was considered significant.

### 9.2.8 Results of in situ hybridization histochemistry

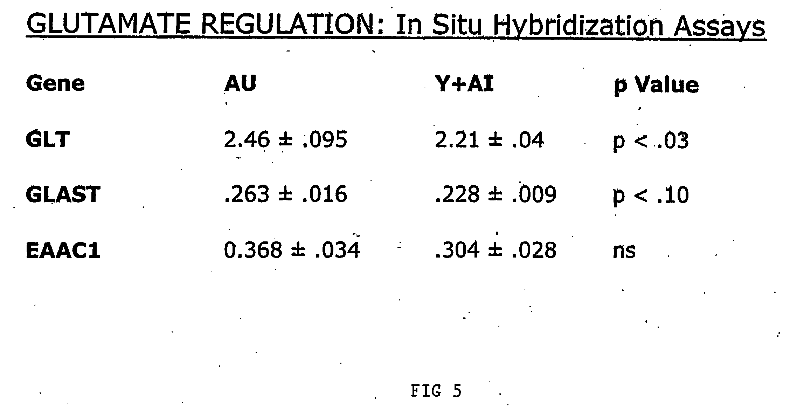

The abundance of GLT1 was significantly greater in aged unimpaired relative to young and aged impaired (p<.03), whereas a trend for greater GLAST mRNA was also found (p=.089; Figure 5). The learning index scores representing cognitive status in the set of rats used for in situ hybridization were similar to the animals in the microarray study; aged unimpaired (182, 223, 240) young (177, 219, 200, 227) and aged impaired (290, 285, 297, 298).

In the microarray dataset and the in situ dataset, values for a third glutamate transporter mRNA (EAACl) were numerically higher in the unimpaired aged group than in the comparison young and impaired aged rats, but those differences were not statistically significant. The mRNA for one probe set in the microarray (AF038571) showed that EAAC1 was significantly elevated in the aged unimpaired relative to aged impaired. The mRNA in a second probe set for EAAC1 showed a similar trend (aged unimpaired greater than aged impaired, p=.09).

### 9.3 Preserved cognitive function using ceftriaxone

### 9.3.1 Effect of ceftriaxone treatment on GLT1 mRNA in young rats

Young rats were given daily injection of ceftriaxone at 200 mg/kg intramuscularly (N=2) or vehicle (N=3) for one week. After sacrifice, the hippocampus was dissected and frozen.

### 9.3.2 Real Time reverse transcription-PCR method for quantifying GLT-1 mRNA

### 9.3.2.1 Preparation of RNA for real time RT-PCR

RNA was extracted from the right hippocampus of ceftriaxone or saline treated animals (3 young animals per condition) using Trizol reagent and purified over Qiagen RNeasy columns in a manner identical to the procedure used for microarray experiments. After the concentration and integrity was determined (in the same manner as for microarray RNA) samples were diluted to a concentration of 50ng per microliter. 100 ng of this sample was reverse transcribed in a total volume of 10 µl using Applied Biosysten's Taqman reverse transcription reagents with the following conditions: 1x reverse transcription buffer, 0.5mM of each dNTP, 5.5mM MgCl2, 1.25 Units/µL of Multiscribe reverse transcriptase, 0.4Units/µL RNase inhibitor, and 2.5µM oligo dT primer. Samples were incubated at room temperature for 10 minutes followed by 45 minutes at 48°C and then 5 minutes at 95°C. Samples were diluted 1:20 and 1:100 for use in a real time PCR reaction. A Standard RNA was generated by combining the extracted and purified hippocampal RNA from 2 separate animals and reverse transcribing this RNA in conditions identical to that for the experimental samples above except that 500ng of RNA is used in a 50µl reaction. Standard cDNA was diluted to 1:20, 1:100, 1:500 and 1:2500 by serial dilution. In addition 200ng of standard RNA was placed in a 20µl reaction with the same conditions as above except that the reverse transcriptase is omitted. This sample was referred to as the no RT sample and is included to indicate background activity of the RNA sample itself. This sample was serially diluted 1:20 and 1:100.

### 9.3.2.2 Real Time PCR

PCR reactions were performed in triplicate on each cDNA sample at two different concentrations for GLT1a, GLT1 and GAPDH using the 1:20 and 1:100 dilution of the cDNA. The final concentration of cDNA in the PCR reaction was 100pg/µl and g/µl, and was based on extrapolation from the concentration of input RNA. The standard was used at all four dilutions to generate extrapolated final concentrations of 100pg/µl, 20pg/µl, 4pg/µl and 0.8pg/µl. A PCR reaction mix using Invitrogen's Platinum quantitative PCR kit was assembled for all samples using the same primer and probe set which was then divided into separate tubes for each cDNA at each concentration. The cDNA was then added to the mixture which was then distributed to each of three real time PCR tubes. All reactions were run in a RotorGene 3000 (Corbett Research) with the following conditions: 2 minutes at 50°C, 5 minutes at 95°C and then 45 cycles of 25 seconds at 95°C and 60 seconds at 60°C. Data was acquired on the Joe channel for GAPDH and FAM/SYBR channel for GLT1 and GLT1a probe/primer sets. Spike suppression and dynamic tube normalization were used for all runs. GLT1 or GLT1a and GAPDH real time PCR was occasionally performed during separate runs.

For GAPDH the optimal reaction conditions were 0.6Units Platinum Taq DNA polymerase, 20mM Tris-HCl (pH 8.4), 50 mM KCl, 200µM dGTP, 200µM dATP, 200µM dCTP, 400µM dUTP, 0.4Units UDG, 4.5mM MgC12, 200nM Forward primer, 200nM Reverse primer, 50nM probe labeled with Vic on the 5' end and TAMRA quencher on the 3' end. Primers and probe were obtained from Applied Biosystems(Cat # 4308313); the sequences were unknown, but the amplicon length was 177bps.

For GLT1 the optimal reaction conditions were 0.6Units Platinum Taq DNA polymerase, 20mM Tris-HCl (pH 8.4), 50 mM KCl, 200µM dGTP, 200µM dATP, 200µM dCTP, 400µM dUTP, 0.4Units UDG, 6.0mM MgCl2, 50nM Forward primer, 200nM Reverse primer, 50nM probe. The amplicon length was 65 bps. The forward primer was 5' GAG CTG GAC ACC ATT GAC TC 3' and reverse primer was 5' GAC TGC GTC TTG GTC ATT TC 3'. The probe was 5'CAA CAC CGA ATG CAC GAA GAC ATC 3' labeled with a 5' 6-fam and 3' tamra.

For GLT 1 a the optimal reaction conditions were 0.6Units Platinum Taq DNA polymerase, 20mM Tris-HCl (pH 8.4), 50 mM KCl, 200µM dGTP, 200µM dATP, 200µM dCTP, 400µM dUTP, 0.4Units UDG, 6.0mM MgCl2, 200nM Forward primer, 200nM Reverse primer, 100nM probe. Amplicon length was 76 bps. The forward primer was 5' ATG AGT GCA AGG TAA CTC TGG 3' and the reverse primer was 5' TCA CGT TTC CAA GGT TCT TC 3'. The probe was 5' CCA ATG GAA AGT CAG CTG ACT GCA 3' labeled with 5' 6-fam and 3' BHQ1 (Black hole quencher 1).

### 9.3.2.3 Data analysis of Real Time PCR

Comparative amounts of GAPDH and GLT1 or GLT1a were determined using the DDCt method (Livak and Schmittgen, Methods 25:402-408 2001). The threshold for all reactions was set at 0.05 fluorescent units and the Ct for each sample determined by the Rotorgene software. Average Ct values for each GLT1 cDNA at each concentration were determined and subtracted from the average values for GAPDH. Samples were excluded if the GAPDH value is more than 15% above or below the mean GAPDH value. GLT1 mRNA increased an average of 1.34 fold in the drug treated animals and GLT1a mRNA increased an average of 1.27 in the drug treated animals. These values were consistent with the full change with GLT1 observed in AU rats in the microarray, which was an increase of 1.31.

### 9.4 Effect of ceftriaxone treatment on the performance of aged rats in the RAM

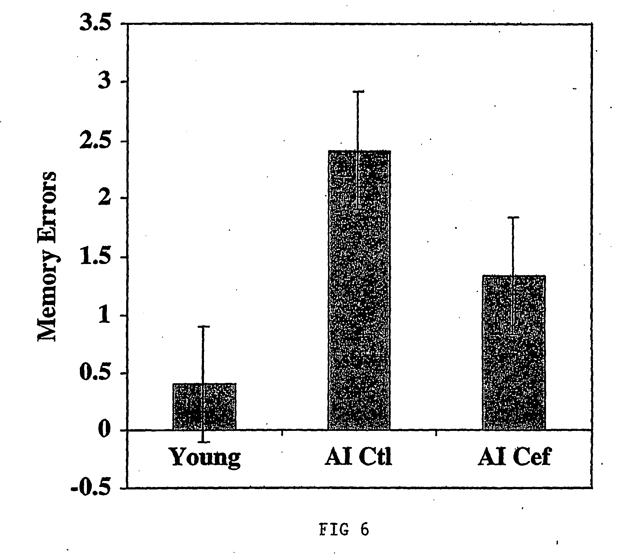

After characterization for cognitive status in the MWM, impaired aged rats were assigned to one of two treatment conditions (control vehicle or ceftriaxone at 200 mg/kg) that were equated with respect to their MWM learning index scores. Initially rats in both treatment conditions were trained on the RAM task (habituation, no-delay version, and then delay of 60 seconds). Then daily injection of vehicle or drug occurred in the morning (8:00-9:00 AM). Rats received daily testing on the radial-arm maze with the delay extended to 3 hr. Critical tests at the 3 hour delay occurred on Days 8-10 (after 7 days of injection). Memory errors for aged rats receiving vehicle were significantly elevated relative to a young group tested concurrently. Compared to aged rats receiving vehicle, aged rats receiving drug administration had fewer errors (Figure 6). This initial pilot assessment using 3 drug-treated rats and 4 aged rats treated with vehicle is currently being replicated with additional behaviorally characterized rats to increase sample sizes to 7-8 rats per group.

### 9.4.1 Effect of ceftriaxone treatment on GLT1 MRNA in aged rats

At completion of RAM testing aged rats under vehicle and drug treatment are sacrificed. Dissected hippocampus is frozen and stored (-80°C) for analysis of GLT1 mRNA, which is conducted at the completion of the replication of behavioral testing with additional aged rats. If drug treatment increases GLT1 mRNA, as expected based on the effect of drug treatment at the same dose in young rats, a microarray analysis is conducted to compare gene expression profiles in aged impaired rats treated with ceftriaxone relative to, control aged impaired subject.

### 9.4.2 Effect of ceftriaxone treatment on test-retest in the MWM

Test-retest reliability is obtained when aged rats are characterized in the standard MWM protocol and then tested after weeks or months in a new spatial environment using the MWM. The preclinical efficacy of a compound to improve function in aged rats with cognitive impairment can be assessed in a re-test using the MWM. Effectiveness of a treatment across the two tasks (RAM and MWM) strengthens evidence for a drug action on cognitive function independent of other components that differ across the two tasks (e.g. motivational basis for performance).

### 9.5 Improved and preserved cognitive function with other compounds

This work includes administration of the test compound or vehicle to young rats in order to establish a treatment regimen that improves or preserves cognitive function. Test compounds include valproic acid, compounds that modulate metabotropic glutamate receptor (mGluR) activity and compounds that modulate pituitary adenyl cyclase activator polypeptide (PACAP) expression. Aged impaired rats characterized on the MWM are assigned to drug or vehicle treatment and tested in the RAM. Analysis of glutamate transporter expression and other gene profiling, such as mGluR expression and activity, PACAP expression or β-arrestin 2 expression are then conducted at the completion of behavioral testing

Preferred embodiments are set out below, and are referred to as embodiment E1 to embodiment E53.
E1. A method of identifying a gene associated with a desired behavior in a mammal, comprising the steps of:
   (a) Providing a test population of mammals having the desired behavior;
   (b) Providing a control population of mammals lacking the desired behavior;
   (c) Isolating and pooling expressed RNA from neural tissue of the test population;
   (d) Isolating and pooling expressed RNA from neural tissue of the control population;
   (e) Determining the level of expression of a plurality of genes in each of the RNA pools created in steps (c) and (d); and,
   (f) Selecting a gene from the plurality of genes, the expression of which differs between the test population and the control population of mammals, wherein the selected gene is a candidate gene associated with said desired behavior.
E2. A method of identifying a gene associated with cognitive function in a mammal, comprising the steps of:
   (a) Providing a test population of mammals having a desired cognitive function;
   (b) Providing a control population of mammals impaired in such cognitive function;
   (c) Isolating and pooling expressed RNA from neural tissue of the test population;
   (d) Isolating and pooling expressed RNA from neural tissue of the control population;
   (e) Determining the level of expression of a plurality of genes in each of the RNA pools created in steps (c) and (d); and,
   (f) Selecting a gene from the plurality of genes, the expression of which differs between the test population and the control population of mammals, wherein the selected gene is a candidate gene associated with cognitive function.
E3. The method of E1 or E2 wherein the level of expression of said plurality of genes is detected by a method selected from the group consisting of microarray analysis, in situ hybridization histochemistry, quantitative PCR, SAGE analysis, Northern blot analysis, and dot blot analysis.
E4. The method of E1 or E2 wherein said plurality of genes comprises a gene involved in glutamate transport.
E5. The method of E4 wherein said gene involved in glutamate transport is selected from the group consisting of: EAAT1, EAAT2, EAAT3, EAAT4, and EAAT5.
E6. The method of E1 or E2 wherein said plurality of genes comprises a gene other than a glutamate transporter selected from the group consisting of: EAAT1, EAAT2, EAAT3, EAAT4, and EAAT5.
E7. A method of screening compounds for utility in promoting cognitive function, comprising the steps of:
   (a) Administering a test compound to a mammal;
   (b) Determining the level of expression of a gene in neural tissue of said mammal following administration of said test compound;
   (c) Comparing said level of expression of said gene to a reference level of expression thereof in neural tissue of a mammal to whom said test compound was not administered; and,
   (d) Determining whether the level of expression of said gene differs from the corresponding reference level of expression thereof, wherein said difference indicates that the test compound is a candidate therapeutic agent for promoting cognitive function.
E8. The method of E7 comprising the further step of comparing said level of expression of said gene to a reference level of expression thereof in neural tissue of a mammal to whom ceftriaxone was administered.
E9. The method of E7 wherein the level of expression of said gene is detected by a method selected from the group consisting of: microarray analysis, in situ hybridization histochemistry, quantitative PCR, SAGE analysis, Northern blot analysis, and dot blot analysis.
E10. The method of E7 wherein said gene is a glutamate transporter.
E11. The method of E10 wherein said gene is selected from the group consisting of:
   EAAT1, EAAT2, EAAT3, EAAT4, and EAAT5.
E12. The method of E1, E2 or E7 wherein said neural tissue is hippocampal tissue.
E13. A method of screening compounds for utility in promoting cognitive function, comprising the steps of:
   (a) Administering a test compound to a mammal;
   (b) Determining the level of expression of a glutamate transporter gene in neural tissue of said mammal following administration of said test compound;
   (c) Comparing said level of expression of said gene to a reference level of expression thereof in neural tissue of a mammal to whom said test compound was not administered; and,
   (d) Determining whether the level of expression of said gene differs from the corresponding reference level of expression thereof, wherein said difference indicates that the test compound is a candidate therapeutic agent for promoting cognitive function.
E14. A method of screening compounds for utility in promoting cognitive function in a mammal, comprising the steps of:
   (a) Contacting a test compound with a cell expressing a gene listed in Figure 4; and
   (b) Determining whether the level of expression of said gene is changed by contact of said cell with said test compound, said change if present being indicative of the ability of said compound to promote cognitive function in a mammal in need thereof.
E 15. The method of E 14 wherein said cell is derived from neural tissue.
E16. The method of E15 wherein said cell is an immortalized cell.
E17. The method of E16 wherein said cell is a neuronal cell line, a glial cell line, or an astrocyte cell line.
E18. The method of E14 wherein said gene is a glutamate transporter.
E19. The method of E1, E2, E7, E13 or E14 wherein the level of expression of said gene is increased.
E20. The method of E1, E2, E7, E13 or E14- wherein the level of expression of said gene is decreased.
E21. The method ofE7, E13 or E14 wherein said test compound is a small molecule.
E22. The method of E21 wherein said test compound is: 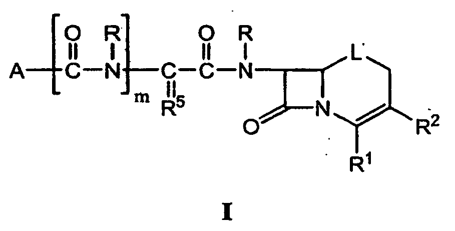 wherein, individually for each occurrence:
   L is 0 or S;
   R is H, C₁₋₁₀ alkyl, C₁₋₁₀ alkoxy, aryl, aralkyl, -OCH₂CO₂H;
   R¹ is -(CH₂)ₙ-C(O)X
      wherein
      X is OH, NR₂, SH, O-alkali metal, or -OC(CH₃)OC(O)OCH(CH₃)₂; and
      n is an integer from 0 to 6 inclusive;
   R² is H, C₁₋₁₀ alkyl, C₂₋₈ alkenyl, or -(CH₂)ₐ-W-R³
      wherein
      R³ is H, C₁₋₁₀ alkyl, -C(O)C₁₋₁₀ alkyl, -C(O)NR₂, aryl, aralkyl, or A;
      W is O, S, or NR⁴; and
      a is an integer from 1 to 6 inclusive;
         wherein
         R⁴ is H, C₁₋₁₀ alkyl, -C(O)C₁₋₁₀ alkyl, aryl, aralkyl, or R³ and R⁴ taken together may form an unsubstituted or substituted heteroalkyl or heteroaryl ring;
   the ---- line indicates either a single or double bond;
   R⁵ is R¹, H, SO₃H, aryl, C₁₋₁₀ alkyl, aralkyl; or R⁵ is selected from the group consisting of =CHCH₂CO₂H and =NR when the ---- line is a double bond;
   m is 0 or 1; and
   A is aryl or heteroaryl of formula **Ia:** 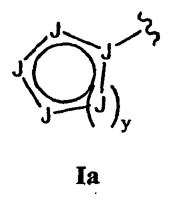 wherein, independently for each occurrence:
   J is O, S, NR⁶, or CR⁶; and
   y is 1 or 2;
      wherein R⁶ is an electron pair, H, C₁₋₁₀ alkyl, C₁₋₁₀ alkoxy, aryl, or -NR₂;
   or A is heterocycloalkyl of formula **Ib** or **Ic:** 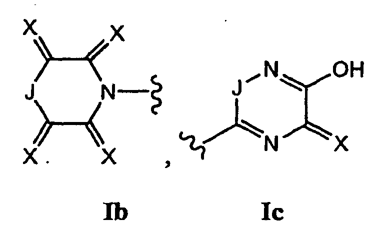 wherein, independently for each occurrence:
   J is O, S, or NR; and
   X is O or H₂.
E23. The method of E21 wherein said test compound is: 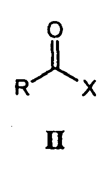 wherein, independently for each occurrence:
   X is -OH, C₁₋₁₀ alkoxy, -O-alkali metal, -N(R¹)₂, -SH, or -S-C₁₋₁₀ alkyl;
   R is a straight chain or branched C₁₋₃₀ alkyl; and
   R¹ is H, C₁₋₁₀ alky, C₂₋₁₀ alkenyl, C₂₋₁₀ alkynyl, aryl, or aralkyl;
   provided that R may be unsubstituted or substituted by one or more -OH, C₁₋₁₀ alkoxy, -N(R¹)₂, -SH, -S-C₁₋₁₀ alkyl, or aryl.
E24. A library comprising a plurality of cDNA sequences coding for genes that are differentially expressed in neural tissue upon preservation of cognitive function in a mammal.
E25. A library comprising a plurality of cDNA sequences coding for genes that are differentially expressed in neural tissue upon treatment of a mammal with ceftriaxone.
E26. A library comprising a plurality of cDNA sequences coding for genes that are differentially expressed in neural tissue upon treatment of a mammal with valproic acid.
E27. The library of E24, E25 or E26 wherein said plurality of cDNA sequences comprises a sequence derived from a glutamate transporter gene.
E28. The library of E27 wherein said glutamate transporter gene is selected from the group consisting of: EAAT1, EAAT2, EAAT3, EAAT4, and EAAT5.
E29. The library of E27 wherein said plurality of cDNA sequences comprises at least 20% of all sequences present therein.
E30. The library of E27 wherein said plurality of cDNA sequences comprises at least 50% of all sequences present therein.
E31. The library of E27 wherein said plurality of cDNA sequences comprises at least 80% of all sequences present therein.
E32. A microarray chip comprising a solid support having attached thereto, at individually addressed locations, cDNA sequences corresponding to members of the library of E24, E25 or E26.
E33. Use in therapy of a composition capable of stimulating neural tissue expression of a gene listed in Figure 4.
E34. The use of E33 wherein said gene is a glutamate transporter.
E35. The use of E34 wherein said glutamate transporter is EAAT1, EAAT2, EAAT3, EAAT4, or EAAT5.
E36. The use of E33 wherein said compound is a small molecule.
E37. A use in therapy of a therapeutically effective amount of: 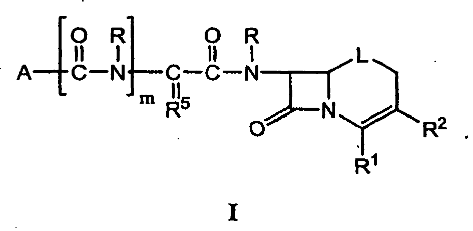 wherein, individually for each occurrence:
   L is O or S;
   R is H, C₁₋₁₀ alkyl, C₁₋₁₀ alkoxy, aryl, aralkyl, -OCH₂CO₂H;
   R¹ is -(CH₂)ₙ-C(O)X
      wherein
      X is OH, NR₂, SH, O-alkali metal, or -OC(CH₃)OC(O)OCH(CH₃)₂; and
      n is an integer from 0 to 6 inclusive;
   R² is H, C₁₋₁₀ alkyl, C₂₋₈ alkenyl, or -(CH₂)ₐ-W-R³
      wherein
      R³ is H, C₁₋₁₀ alkyl, -C(O)C₁₋₁₀ alkyl, -C(O)NR₂, aryl, aralkyl, or A;
      W is O, S, or NR⁴; and
      a is an integer from 1 to 6 inclusive;
         wherein
         R⁴ is H, C₁₋₁₀ alkyl, -C(O)C₁₋₁₀ alkyl, aryl, aralkyl, or R³ and R⁴ taken together may form an unsubstituted or substituted heteroalkyl or heteroaryl ring;
   the ---- line indicates either a single or double bond;
   R⁵ is R¹, H, SO₃H, aryl, C₁₋₁₀ alkyl, aralkyl; or R⁵ is selected from the group consisting of =CHCH₂CO₂H and =NR when the ---- line is a double bond;
   m is 0 or 1; and
   A is aryl or heteroaryl of formula Ia: 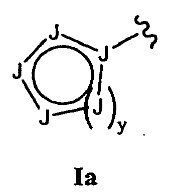 wherein, independently for each occurrence:
   J is O, S, NR⁶, or CR⁶; and
   y is 1 or 2;
      wherein R⁶ is an electron pair, H, C₁₋₁₀ alkyl, C₁₋₁₀ alkoxy, aryl, or -NR₂;
   or A is heterocycloalkyl of formula Ib or Ic: 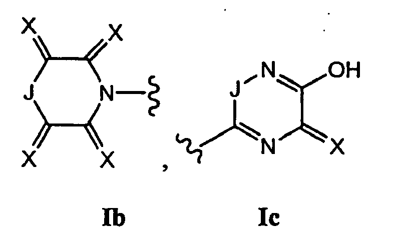 wherein, independently for each occurrence:
   J is O, S, or NR; and
   X is O or H₂
E38. A use in therapy of a therapeutically effective amount of: 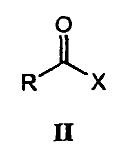 wherein, independently for each occurrence:
   X is -OH, C₁₋₁₀ alkoxy, -O-alkali metal, -N(R¹)₂, -SH, or -S-C₁₋₁₀ alkyl;
   R is a straight chain or branched C₁₋₃₀ alkyl; and
   R¹ is H, C₁₋₁₀ alky, C₂₋₁₀ alkenyl, C₂₋₁₀ alkynyl, aryl, or aralkyl;
   provided that R may be unsubstituted or substituted by one or more -OH, C₁₋₁₀ alkoxy, -N(R¹)₂, -SH, -S-C₁₋₁₀ alkyl, or aryl.
E39. A use in therapy of a composition, other than ceftriaxone or valproic acid, identified according to the method of E7, E13 or E14.
E40. A method for the manufacture of a medicament for preserving cognitive function in a mammal, wherein said medicament stimulates neural tissue expression of a glutamate transporter gene in said mammal.
E41. A method for the manufacture of a medicament for treating impaired cognitive function in a mammal, wherein said medicament stimulates neural tissue expression of a glutamate transporter gene in said mammal.
E42. A method for the manufacture of a medicament for preserving cognitive function in a mammal, wherein said medicament comprisings composition of 33.
E43. A method for the manufacture of a medicament for preserving cognitive function in a mammal in need thereof, comprising the composition of E37.
E44. A method for the manufacture of a medicament for preserving cognitive function in a mammal in need thereof, comprising the composition of E38.
E45. A method for the manufacture of a medicament for preserving cognitive function in a mammal in need thereof, comprising the composition of E39 to said mammal.
E46. The method of E43, wherein said mammal is free of symptoms of an infectious disease for which antibiotic treatment is indicated.
E47 . A method for the manufacture of a medicament for promoting cognitive function in a mammal in need thereof, comprising composition of E33, wherein said cognitive function selected from the group consisting of: spatial memory acquisition, long-term spatial memory and spatial memory retrieval.
E48. A method for the manufacture of a medicament for preserving cognitive function in an aged mammal, comprising ceftriaxone or an analog or derivative thereof.
E49. A method for the manufacture of a medicament for treating impaired cognitive function in a mammal, comprising ceftriaxone or an analog or derivative thereof.
E50. The method of E41 or E49 wherein said impaired cognitive function is a condition selected from the group consisting of: mild cognitive impairment, age related cognitive decline, memory loss, senility, and dementia.
E51. The method of E41 or E49 wherein said impaired cognitive function is Alzheimer's Disease.
E52. The method of E41 or E49 wherein said mammal is human.
E53. The method of E40, E42, E43, E44, E45, E46, E47 or E48 wherein said mammal is human.

## Claims

1. A compound for use in treating cognitive impairment during aging in a subject in need thereof, wherein the compound is valproic acid or a pharmaceutically acceptable salt thereof.

2. A compound according to claim 1, wherein the cognitive impairment is mild cognitive impairment.

3. Use of a compound for the manufacture of a medicament for treating cognitive impairment during aging in a subject in need thereof, wherein the compound is valproic acid or a pharmaceutically acceptable salt thereof.

4. Use according to claim 3, wherein the cognitive impairment is mild cognitive impairment.

## Patentansprüche

1. Chemische Verbindung zur Verwendung bei der Behandlung einer kognitiven Behinderung während des Alterns bei einer diese benötigenden Person, wobei die chemische Verbindung Valproinsäure oder ein pharmazeutisch akzeptierbares Salz davon ist.

2. Chemische Verbindung nach Anspruch 1, wobei die kognitive Behinderung eine milde kognitive Behinderung ist.

3. Verwendung einer chemischen Verbindung zur Herstellung eines Medikaments zur Behandlung einer kognitiven Behinderung während des Alterns bei einer diese benötigenden Person, wobei die chemische Verbindung Valproinsäure oder ein pharmazeutisch akzeptierbares Salz davon ist.

4. Verwendung nach Anspruch 3, wobei die kognitive Behinderung eine milde kognitive Behinderung ist.

## Revendications

1. Composé destiné à être utilisé dans le traitement d'une détérioration cognitive pendant le vieillissement chez un sujet qui en a besoin, où le composé est l'acide valproïque ou un sel pharmaceutiquement acceptable de celui-ci.

2. Composé selon la revendication 1 où la détérioration cognitive est une détérioration cognitive faible.

3. Utilisation d'un composé pour la fabrication d'un médicament pour traiter une détérioration cognitive pendant le vieillissement chez un sujet qui en a besoin, où le composé est l'acide valproïque ou un sel pharmaceutiquement acceptable de celui-ci.

4. Utilisation selon la revendication 3 où la détérioration cognitive est une détérioration cognitive faible.


### SEQUENCE LISTING

<110> THE JOHNS HOPKINS UNIVERSITY
<120> TARGET FOR THERAPY OF COGNITIVE IMPAIRMENT
<130> P27593EP-D1-PCT
<150> EP03808425.7
   <151> 2003-11-24
<150> 60/428,229
   <151> 2002-11-22
<160> 18
<170> PatentIn Ver. 3.3
<210> 1
   <211> 20
   <212> DNA
   <213> Artificial Sequence
<220>
   <223> Description of Artificial sequence: Synthetic oligonucleotide
<400> 1
   gagcattggt gcagccagta 20
<210> 2
   <211> 20
   <212> DNA
   <213> Artificial sequence
<220>
   <223> Description of Artificial sequence: synthetic oligonucleotide
<400> 2
   gtctgagaac aagacaaagg 20
<210> 3
   <211> 20
   <212> DNA
   <213> Artificial Sequence
<220>
   <223> Description of Artificial sequence: synthetic oligonucleotide
<400> 3
   ggtagaagcc tgctttaaac 20
<210> 4
   <211> 20
   <212> DNA
   <213> Artificial Sequence
<220>
   <223> Description of Artificial Sequence: synthetic oligonucleotide
<400> 4
   ccaaggttct tcctcaacac 20
<210> 5
   <211> 19
   <212> DNA
   <213> Artificial Sequence
<220>
   <223> Description of Artificial sequence: synthetic oligonucleotide
<400> 5
   tgagagctgt caggagagc 19
<210> 6
   <211> 21
   <212> DNA
   <213> Artificial Sequence
<220>
   <223> Description of Artificial Sequence: synthetic oligonucleotide
<400> 6
   ggcatgaatg aggaggccga c 21
<210> 7
   <211> 39
   <212> DNA
   <213> Artificial Sequence
<220>
   <223> Description of Artificial sequence: synthetic oligonucleotide
<400> 7
   tatttaggtg acactatagg agcattggtg cagccagta 39
<210> 8
   <211> 39
   <212> DNA
   <213> Artificial Sequence
<220>
   <223> Description of Artificial Sequence: synthetic oligonucleotide
<400> 8
   tatttaggtg acactatagg tctgagaaca agacaaagg 39
<210> 9
   <211> 39
   <212> DNA
   <213> Artificial Sequence
<220>
   <223> Description of Artificial sequence: synthetic oligonucleotide
<400> 9
   tatttaggtg acactatagg gtagaagcct gctttaaac 39
<210> 10
   <211> 39
   <212> DNA
   <213> Artificial Sequence
<220>
   <223> Description of Artificial sequence: synthetic oligonucleotide
<400> 10
   taatacgact cactataggg gccaaggttc ttcctcaac 39
<210> 11
   <211> 40
   <212> DNA
   <213> Artificial Sequence
<220>
   <223> Description of Artificial sequence: synthetic oligonucleotide
<400> 11
   taatacgact cactataggg gtgagagctg tcaggagagc 40
<210> 12
   <211> 42
   <212> DNA
   <213> Artificial sequence
<220>
   <223> Description of Artificial sequence: synthetic oligonucleotide
<400> 12
   taatacgact cactataggg gggcatgaat gaggaggccg ac 42
<210> 13
   <211> 20
   <212> DNA
   <213> Artificial sequence
<220>
   <223> Description of Artificial sequence: synthetic oligonucleotide
<400> 13
   gagctggaca ccattgactc 20
<210> 14
   <211> 20
   <212> DNA
   <213> Artificial Sequence
<220>
   <223> Description of Artificial sequence: synthetic oligonucleotide
<400> 14
   gactgcgtct tggtcatttc 20
<210> 15
   <211> 24
   <212> DNA
   <213> Artificial sequence
<220>
   <223> Description of Artificial sequence: synthetic oligonucleotide
<400> 15
   caacaccgaa tgcacgaaga catc 24
<210> 16
   <211> 21
   <212> DNA
   <213> Artificial sequence
<220>
   <223> Description of Artificial sequence: synthetic oligonucleotide
<400> 16
   atgagtgcaa ggtaactctg g 21
<210> 17
   <211> 20
   <212> DNA
   <213> Artificial sequence
<220>
   <223> Description of Artificial sequence: synthetic oligonucleotide
<400> 17
   tcacgtttcc aaggttcttc 20
<210> 18
   <211> 24
   <212> DNA
   <213> Artificial sequence
<220>
   <223> Description of Artificial sequence: synthetic oligonucleotide
<400> 18
   ccaatggaaa gtcagctgac tgca 24